(19) Europäisches Patentamt
European Patent Office
Office européen des brevets

(11) EP 2 017 259 A2

(12) **EUROPEAN PATENT APPLICATION**

(43) Date of publication:
**21.01.2009 Bulletin 2009/04**

(21) Application number: **08168440.9**

(22) Date of filing: **11.12.2006**

(51) Int Cl.:
*C07C 227/32* (2006.01) *C07C 229/08* (2006.01)
*C07C 253/30* (2006.01) *C07C 255/19* (2006.01)
*C12P 13/00* (2006.01) *C12P 41/00* (2006.01)

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK RS**

(30) Priority: **21.12.2005 US 752839 P**

(62) Document number(s) of the earlier application(s) in accordance with Art. 76 EPC:
**06831738.7 / 1 973 867**

(71) Applicant: **Pfizer Products Inc.**
**Groton, CT 06340-5146 (US)**

(72) Inventors:
- **Evans, Margaret Claire**
  **Kalamazoo, MI 49007 (US)**
- **Franklin, Lloyd Charles**
  **Ann Arbor, MI 48105 (US)**
- **Murtagh, Lorraine Michelle**
  **Sandwich, Kent CT13 9NJ (GB)**
- **Nanninga, Thomas Norman**
  **Kalamazoo, MI 49009 (US)**
- **Pearlman, Bruce Allen**
  **West Field, NJ 07090 (US)**
- **Saenz, James Edward**
  **Groton, CT 06340 (US)**
- **Willis, Niamh Josephine**
  **Sittingbourne, Kent ME9 8AG (GB)**

(74) Representative: **Ruddock, Keith Stephen**
**Pfizer Limited**
**European Patent Department**
**Ramsgate Road**
**Sandwich**
**Kent CT13 9NJ (GB)**

Remarks:
This application was filed on 06-11-2008 as a divisional application to the application mentioned under INID code 62.

# (54) Preparation of gamma-amino acids having affinity for the alpha-2-delta protein

(57) Disclosed are materials and methods for preparing optically active γ-amino acids of Formula 1,

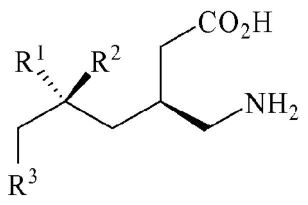

**1**

which bind to the alpha-2-delta (α2δ) subunit of a calcium channel.

Figure 1
Measured PXRD Pattern for (3*S*,5*R*)-3-aminomethyl-5-methyl-octanoic acid Form A

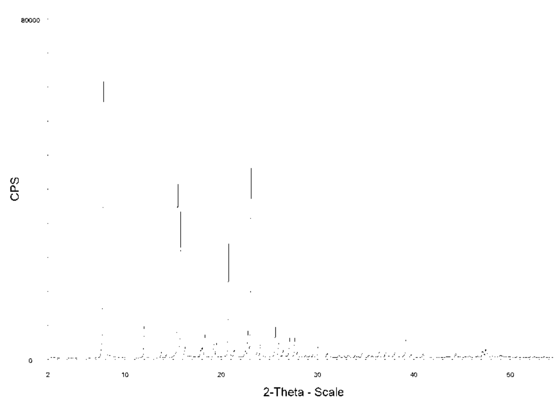


EP 2 017 259 A2

## Description

This invention relates to materials and methods for preparing optically-active γ-amino acids that bind to the alpha-2-delta (α2δ) subunit of a calcium channel. These compounds, including their pharmaceutically acceptable salts, solvates and hydrates, are useful for treating vasomotor symptoms (hot flashes and night sweats), restless legs syndrome, fibromyalgia, epilepsy, pain, and a variety of neurodegenerative, psychiatric and sleep disorders.

WO-A-2000/076958 and U.S. Patent No. 6,642,398 describe γ-amino acids of the formula:

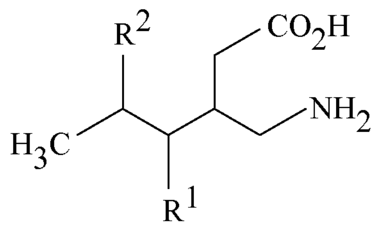

or a pharmaceutically acceptable salt thereof wherein:

$R^1$ is hydrogen, straight or branched alkyl of from 1 to 6 carbon atoms or phenyl;
$R^2$ is straight or branched alkyl of from 1 to 8 carbon atoms, straight or branched alkenyl of from 2 to 8 carbon atoms, cycloalkyl of from 3 to 7 carbon atoms, alkoxy of from 1 to 6 carbon atoms, alkylcycloalkyl, alkylalkoxy, alkyl OH, alkylphenyl, alkylphenoxy, phenyl or substituted phenyl; and
$R^1$ is straight or branched alkyl of from 1 to 6 carbon atoms or phenyl when $R^2$ is methyl.

These compounds, along with their pharmaceutically acceptable salts, solvates, and hydrates, bind to the α28 subunit of a calcium channel and may be used to treat a number of disorders, medical conditions, and diseases, including, among others, epilepsy; pain (e.g., acute and chronic pain, neuropathic pain, and psychogenic pain); neurodegenerative disorders (e.g., acute brain injury arising from stroke, head trauma, and asphyxia); psychiatric disorders (e.g., anxiety and depression); and sleep disorders (e.g., insomnia, drug-associated sleeplessness, hypersomnia, narcolepsy, sleep apnea, and parasomnias). WO-A-2004/054566 describes the use of these compounds in a method of treating a disorder selected from obsessive compulsive disorder (OCD), phobias, post traumatic stress disorder (PTSD), restless legs syndrome, premenstrual dysphoric disorder, hot flashes, and fibromyalgia.

Many of the γ-amino acids described in WO-A-2000/076958 are optically active. Some of the compounds, below, possess two or more stereogenic (chiral) centers, which make their preparation challenging. Although WO-A-2000/076958 describes useful methods for preparing optically-active γ-amino acids, some of the methods may be problematic for pilot- or full-scale production because of efficiency or cost concerns. Thus, improved methods for preparing optically-active γ-amino acids would be desirable.

The present invention provides improved methods for preparing compounds of Formula 1,

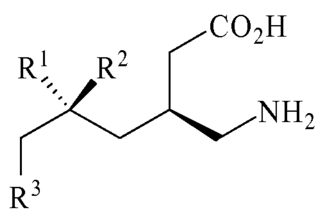

**1**

or a pharmaceutically acceptable salt, solvate or hydrate thereof, wherein:

$R^1$ and $R^2$ are each independently selected from hydrogen and $C_{1-3}$ alkyl, provided that when $R^1$ is hydrogen, $R^2$ is not hydrogen;
$R^3$ is selected from $C_{1-6}$ alkyl, $C_{2-6}$ alkenyl, $C_{3-6}$ cycloalkyl, $C_{3-6}$ cycloalkyl-$C_{1-6}$ alkyl, $C_{1-6}$ alkoxy, aryl, and aryl-$C_{1-3}$ alkyl, wherein each aryl moiety is optionally substituted with from one to three substituents independently selected from $C_{1-3}$ alkyl, $C_{1-3}$ alkoxy, amino, $C_{1-3}$ alkylamino, and halogeno; and

wherein each of the aforementioned alkyl, alkenyl, cycloalkyl, and alkoxy moieties are optionally substituted with from one to three fluorine atoms.

The processes provided by the present invention may be more cost-effective or efficient than known processes and require lower volumes of solvents.

One aspect of the invention provides, as Embodiment A, a process for preparing a compound of Formula 10, or a salt thereof, and a compound of Formula 11, or a salt thereof:

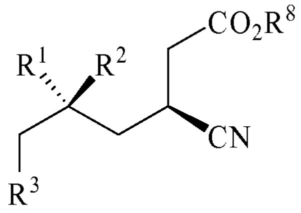

**10**

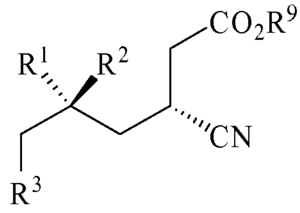

**11**

comprising

(a) contacting a compound of Formula 7,

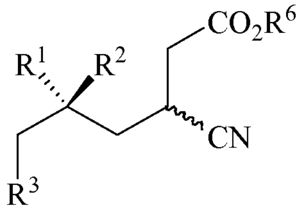

**7**

with an enzyme, wherein the enzyme diastereoselectively hydrolyzes the compound of Formula 7 to the compound of Formula 10 or a salt thereof, or to a compound of Formula 11 or a salt thereof;
(b) isolating the compound of Formula 10, a diastereomer thereof, or a salt thereof; and
(c) optionally hydrolyzing the compound of Formula 10 or 11 to give the free carboxylic acid; wherein

$R^1$ and $R^2$ are each independently selected from hydrogen and $C_{1-3}$ alkyl, provided that $R^1$ and $R^2$ are not both hydrogen;
$R^3$ is selected from $C_{1-6}$ alkyl, $C_{2-6}$ alkenyl, $C_{3-6}$ cycloalkyl, $C_{3-6}$ cycloalkyl-$C_{1-6}$ alkyl, $C_{1-6}$ alkoxy, aryl, and aryl-$C_{1-3}$ alkyl, wherein each aryl moiety is optionally substituted with from one to three substituents independently selected from $C_{1-3}$ alkyl, $C_{1-3}$ alkoxy, amino, $C_{1-3}$ alkylamino, and halogeno; and
wherein each of the aforementioned alkyl, alkenyl, cycloalkyl, and alkoxy moieties are optionally substituted with from one to three fluorine atoms;
$R^6$ in Formula 7 is selected from $C_{1-6}$ alkyl, $C_{2-6}$ alkenyl, $C_{2-6}$ alkynyl, $C_{3-7}$ cycloalkyl, $C_{3-7}$ cycloalkenyl, halo-$C_{1-6}$ alkyl, halo-$C_{2-6}$ alkenyl, halo-$C_{2-6}$ alkynyl, aryl-$C_{1-6}$ alkyl, aryl-$C_{2-6}$ alkenyl, and aryl-$C_{2-6}$ alkynyl; and
$R^8$ and $R^9$ in Formula 10 and 11 are each independently selected from hydrogen, $C_{1-6}$ alkyl, $C_{2-6}$ alkenyl, $C_{2-6}$ alkynyl, $C_{3-7}$ cycloalkyl, $C_{3-7}$ cycloalkenyl, halo-$C_{1-6}$ alkyl, halo-$C_{2-6}$ alkenyl, halo-$C_{2-6}$ alkynyl, aryl-$C_{1-6}$ alkyl, aryl-$C_{2-6}$ alkenyl, and aryl-$C_{2-6}$ alkynyl, provided that $R^8$ and $R^9$ are not both hydrogen; and
wherein each of the aforementioned aryl moieties may be optionally substituted with from one to three substituents independently selected from $C_{1-3}$ alkyl, $C_{1-3}$ alkoxy, amino, $C_{1-3}$ alkylamino, and halogeno.

As Embodiment A1, the invention provides a process for preparing a compound of Formula 10 or a salt thereof:

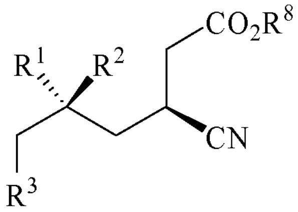

**10**

wherein $R^1$ and $R^2$ are each independently selected from hydrogen and $C_{1-3}$ alkyl, provided that when $R^1$ is hydrogen, $R^2$ is not hydrogen;
$R^3$ is selected from $C_{1-6}$ alkyl, $C_{2-6}$ alkenyl, $C_{3-6}$ cycloalkyl, $C_{3-6}$ cycloalkyl-$C_{1-6}$ alkyl, $C_{1-6}$ alkoxy, aryl, and aryl-$C_{1-3}$ alkyl, wherein each aryl moiety is optionally substituted with from one to three substituents independently selected from $C_{1-3}$ alkyl, $C_{1-3}$ alkoxy, amino, $C_{1-3}$ alkylamino, and halogeno, and wherein each of the aforementioned alkyl, alkenyl, cycloalkyl, and alkoxy moieties are optionally substituted with from one to three fluorine atoms; and
$R^8$ is selected from $C_{1-6}$ alkyl, $C_{2-6}$ alkenyl, $C_{2-6}$ alkynyl, $C_{3-7}$ cycloalkyl, $C_{3-7}$ cycloalkenyl, halo-$C_{1-6}$ alkyl, halo-$C_{2-6}$ alkenyl, halo-$C_{2-6}$ alkynyl, aryl-$C_{1-6}$ alkyl, aryl-$C_{2-6}$ alkenyl, and aryl-$C_{2-6}$ alkynyl, and wherein each of the aforementioned aryl moieties may be optionally substituted with from one to three substituents independently selected from $C_{1-3}$ alkyl, $C_{1-3}$ alkoxy, amino, $C_{1-3}$ alkylamino, and halogeno:

and wherein said process comprises:

(a) contacting a compound of Formula 7 with an enzyme, wherein the enzyme diastereoselectively hydrolyzes the compound of Formula 7 to the compound of Formula 11a;

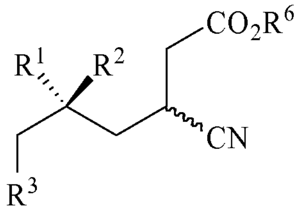

**7**

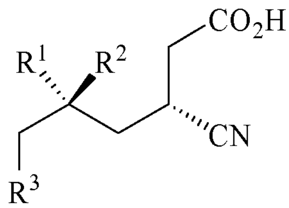

**11a**

wherein $R^1$, $R^2$ and $R^3$ are as defined for a compound of Formula10; and
$R^6$ in Formula 7 is selected from $C_{1-6}$ alkyl, $C_{2-6}$ alkenyl, $C_{2-6}$ alkynyl, $C_{3-7}$ cycloalkyl, $C_{3-7}$ cycloalkenyl, halo-$C_{1-6}$ alkyl, halo-$C_{2-6}$ alkenyl, halo-$C_{2-6}$ alkynyl, aryl-$C_{1-6}$ alkyl, aryl-$C_{2-6}$ alkenyl, and aryl-$C_{2-6}$ alkynyl, and wherein each of the aforementioned aryl moieties may be optionally substituted with from one to three substituents independently selected from $C_{1-3}$ alkyl, $C_{1-3}$ alkoxy, amino, $C_{1-3}$ alkylamino, and halogeno:
and

(b) isolating the compound of Formula 10.

As Embodiment A2, the invention provides a process as defined in Embodiment A, wherein $R^9$ is hydrogen.

As Embodiment A3, the invention provides a process as defined in Embodiment A, A1 or A2, wherein $R^6$ and $R^8$ are independently selected from $C_{1-6}$ alkyl; preferably methyl, ethyl, *n*-propyl and *i*-propyl; most preferably methyl and ethyl.

As Embodiment A4, the invention provides a process as defined in Embodiment A, A1, A2 or A3, wherein $R^1$ and $R^2$ are each independently hydrogen or methyl, provided that $R^1$ and $R^2$ are not both hydrogen, and $R^3$ is $C_{1-6}$ alkyl; preferably $R^1$ is hydrogen, $R^2$ is methyl, and $R^3$ is methyl, ethyl, *n*-propyl or *i*-propyl; most preferably $R^1$ is hydrogen, $R^2$ is methyl, and $R^3$ is ethyl.

As Embodiment A5, the invention provides a process as defined in Embodiment A, A1, A2, A3 or A4, wherein the enzyme in step (a) is a lipase; preferably the enzyme is a lipase from the microorganism *Burkholderia cepacia* or the microorganism Thermomyces *lanuginosus*.

As Embodiment A6, the invention provides a process as defined in Embodiment A1, A2, A3, A4, or A5, wherein the process further comprises the step:

(c) optionally converting the compound of Formula 10 to a salt thereof; preferably to an alkali metal salt thereof; most preferably to the sodium salt thereof.

A further aspect of the invention provides, as Embodiment A7, a process for preparing a compound of Formula 10a, or a salt thereof:

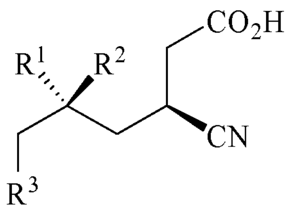

**10a**

wherein $R^1$, $R^2$, and $R^3$ are as defined in Embodiments A or A3. The process comprises the steps of:

(a) contacting a compound of Formula 7,

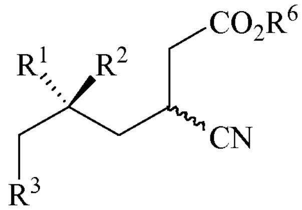

**7**

with an enzyme to yield the compound of Formula 10, or a salt thereof, and a compound of Formula 11, or a salt thereof,

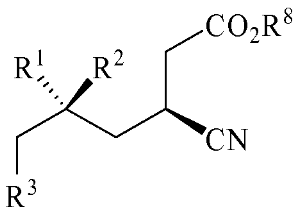

**10**

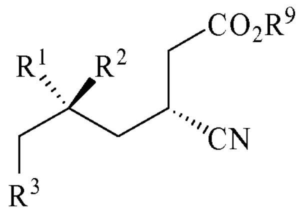

**11**

wherein the enzyme diastereoselectively hydrolyzes the compound of Formula 7 to the compound of Formula 10 or a salt thereof, or to a compound of Formula 11 or a salt thereof;
(b) isolating the compound of Formula 10, or a salt thereof; and
(c) optionally hydrolyzing the compound of Formula 10, to give the compound of Formula 10a, wherein

$R^1$, $R^2$, and $R^3$ in Formula 7, Formula 10, and Formula 11 are as defined for Formula 1, above;
$R^6$ in Formula 7 is selected from $C_{1-6}$ alkyl, $C_{2-6}$ alkenyl, $C_{2-6}$ alkynyl, $C_{3-7}$ cycloalkyl, $C_{3-7}$ cycloalkenyl, halo-$C_{1-6}$ alkyl, halo-$C_{2-6}$ alkenyl, halo-$C_{2-6}$ alkynyl, aryl-$C_{1-6}$ alkyl, aryl-$C_{2-6}$ alkenyl, and aryl-$C_{2-6}$ alkynyl; and
$R^8$ and $R^9$ in Formula 10 and 11 are each independently selected from hydrogen, $C_{1-6}$ alkyl, $C_{2-6}$ alkenyl, $C_{2-6}$ alkynyl, $C_{3-7}$ cycloalkyl, $C_{3-7}$ cycloalkenyl, halo-$C_{1-6}$ alkyl, halo-$C_{2-6}$ alkenyl, halo-$C_{2-6}$ alkynyl, aryl-$C_{1-6}$ alkyl, aryl-$C_{2-6}$ alkenyl, and aryl-$C_{2-6}$ alkynyl;

wherein each of the aforementioned aryl moieties may be optionally substituted with from one to three substituents independently selected from $C_{1-3}$ alkyl, $C_{1-3}$ alkoxy, amino, $C_{1-3}$ alkylamino, and halogeno.

The invention further provides, as Embodiment B, a compound of Formula 7, as defined above in Embodiment A, A1 or A4; preferably $R^6$ is $C_{1-6}$ alkyl; more preferably $R^6$ is methyl, ethyl, *n*-propyl or *i*-propyl.

In Embodiment B1, the invention provides a compound of Formula 7 selected from:

(2'*R*)-2-cyano-2-(2'-methyl-butyl)-succinic acid diethyl ester;
(2'*R*)-2-cyano-2-(2'-methyl-pentyl)-succinic acid diethyl ester;
(2'*R*)-2-cyano-2-(2'-methyl-hexyl)-succinic acid diethyl ester;
(2'*R*)-2-cyano-2-(2',4'-dimethyl-pentyl)-succinic acid diethyl ester;
(5*R*)-3-cyano-5-methyl-heptanoic acid ethyl ester;
(5*R*)-3-cyano-5-methyl-octanoic acid ethyl ester;
(5*R*)-3-cyano-5-methyl-nonanoic acid ethyl ester;
(5*R*)-3-cyano-5,7-dimethyl-octanoic acid ethyl ester;
(5*R*)-3-cyano-5-methyl-heptanoic acid;
(5*R*)-3-cyano-5-methyl-octanoic acid;
(5*R*)-3-cyano-5-methyl-nonanoic acid;
(5*R*)-3-cyano-5,7-dimethyl-octanoic acid;
(3*S*,5*R*)-3-cyano-5-methyl-heptanoic acid;
(3*S*,5*R*)-3-cyano-5-methyl-octanoic acid;
(3*S*,5*R*)-3-cyano-5-methyl-nonanoic acid;
(3*S*,5*R*)-3-cyano-5,7-dimethyl-octanoic acid;
(3*S*,*5R*)-3-cyano-5-methyl-heptanoic acid ethyl ester;
(3*S*,5*R*)-3-cyano-5-methyl-octanoic acid ethyl ester;
(3*S*,5*R*)-3-cyano-5-methyl-octanoic acid methyl ester;
(3*S*,5*R*)-3-cyano-5-methyl-nonanoic acid ethyl ester;
(3*S*,5*R*)-3-cyano-5,7-dimethyl-octanoic acid ethyl ester;
(3*R*,5*R*)-3-cyano-5-methyl-heptanoic acid;
(3*R*,5*R*)-3-cyano-5-methyl-octanoic acid;
(3*R*,5*R*)-3-cyano-5-methyl-nonanoic acid;
(3*R*,5*R*)-3-cyano-5,7-dimethyl-octanoic acid;
(3*R*,5*R*)-3-cyano-5-methyl-heptanoic acid ethyl ester;
(3*R*,5*R*)-3-cyano-5-methyl-octanoic acid ethyl ester;
(3*R*,5*R*)-3-cyano-5-methyl-nonanoic acid ethyl ester;
(3R,5R)-3-cyano-5,7-dimethyl-octanoic acid ethyl ester; and

diastereomers and opposite enantiomers of the aforementioned compounds, and salts of the aforementioned compounds, their diastereomers and opposite enantiomers.

As Embodiment B2, the invention provides a compound of Formula 10 selected from:

(3S,5R)-3-cyano-5-methyl-heptanoic acid;
(3S,5R)-3-cyano-5-methyl-octanoic acid;
(3S,5R)-3-cyano-5-methyl-nonanoic acid;
(3S,5R)-3-cyano-5,7-dimethyl-octanoic acid;
(3*S*,5*R*)-3-cyano-5-methyl-heptanoic acid ethyl ester;
(3*S*,5*R*)-3-cyano-5-methyl-octanoic acid ethyl ester;
(3*S*,5*R*)-3-cyano-5-methyl-octanoic acid methyl ester;
(3*S*,5*R*)-3-cyano-5-methyl-nonanoic acid ethyl ester;
(3*S*,5*R*)-3-cyano-5,7-dimethyl-octanoic acid ethyl ester;

and the salts and esters thereof.

As Embodiment B3, the invention provides the compound (3*S*,5*R*)-3-cyano-5-methyl-octanoic acid or a salt or ester thereof (compounds of Formula 10b):

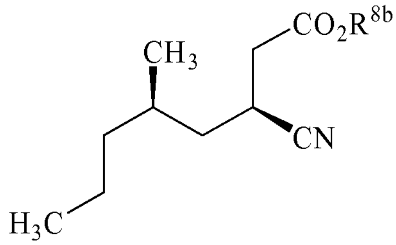

**10b**

wherein $R^{8b}$ is selected from hydrogen, $C_{1-6}$ alkyl, $C_{2-6}$ alkenyl, $C_{2-6}$ alkynyl, $C_{3-7}$ cycloalkyl, $C_{3-7}$ cycloalkenyl, halo-$C_{1-6}$ alkyl, halo-$C_{2-6}$ alkenyl, halo-$C_{2-6}$ alkynyl, aryl-$C_{1-6}$ alkyl, aryl-$C_{2-6}$ alkenyl, and aryl-$C_{2-6}$ alkynyl and wherein each of the aforementioned aryl moieties may be optionally substituted with from one to three substituents independently selected from $C_{1-3}$ alkyl, $C_{1-3}$ alkoxy, amino, $C_{1-3}$ alkylamino, and halogeno; and salts thereof. Preferably the ester thereof is a compound of Formula 10b wherein $R^{8b}$ is $C_{1-6}$ alkyl; more preferably $R^{8b}$ is methyl or ethyl. Preferably the salt thereof is an alkali metal salt of (3*S*,5*R*)-3-cyano-5-methyl-octanoic acid; more preferably the sodium salt thereof.

The invention further provides, as Embodiment C, a process for preparing a compound of Formula 7, or a salt thereof:

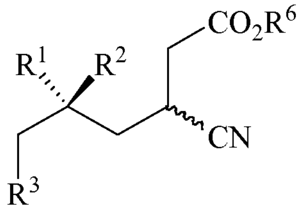

**7**

wherein $R^1$, $R^2$, $R^3$ are as defined in Embodiment B; and
$R^6$ is $C_{1-6}$ alkyl:
and wherein said process comprises

(a) reacting a compound of Formula 19 with an orthoester compound of Formula 20 in the presence of a base

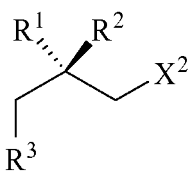

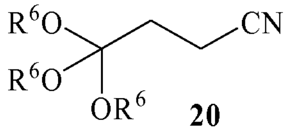

**20**

**19**

wherein $R^1$, $R^2$, $R^3$ and $R^6$ are as defined for a compound of Formula 7; and
$X^2$ is halogeno:
and

(b) hydrolysis of the resulting orthoester intermediate product to provide the carboxylic ester of Formula 7.

The invention further provides, as Embodiment D, a process for the preparation of a compound of Formula 1, as defined above, a diastereomer thereof, or pharmaceutically acceptable complex, salt, solvate or hydrate thereof, comprising steps (a) to (c) of the process as defined in Embodiment A, A6 or A7 and further comprising the steps:

(d) reducing the cyano moiety of a compound of Formula 10, or a salt thereof:

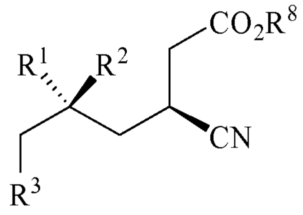

**10**

wherein $R^1$, $R^2$, and $R^3$ in Formula 10 are as defined for a compound of Formula 1 and $R^8$ is as defined in Embodiment A;
and

(e) optionally further converting the compound of Formula 1 or a salt thereof into a pharmaceutically acceptable salt, solvate or hydrate thereof.

As Embodiment D1, the invention provides a process for the preparation of a compound of Formula 1, as defined above, or a pharmaceutically acceptable salt, solvate or hydrate thereof, comprising steps (a) to (c) of the process as defined in Embodiment A6, and further comprising the steps:

(d) reducing the cyano moiety of a salt of the compound of Formula 10 to give a salt of the compound of Formula 1; and
(e) optionally further converting the resulting salt of the compound of Formula 1, or to a pharmaceutically acceptable salt, solvate or hydrate thereof.

As Embodiment D2, the invention provides a process as defined in Embodiment D1, wherein in step (c) the compound of Formula 10 is converted to an alkali metal salt; most preferably the sodium salt.
As Embodiment D3, the invention provides a process as defined in Embodiment D, wherein in step (e), the resulting salt is converted to the free acid of Formula 1.
The invention further relates to a process for preparing a compound of Formula 1, as defined above, including a diastereomer thereof, or a pharmaceutically acceptable salt, solvate or hydrate thereof, comprising steps (a) to (c) of the process as defined in Embodiment A, and further comprising the steps:

(d) reducing a cyano moiety of a compound of Formula 8,

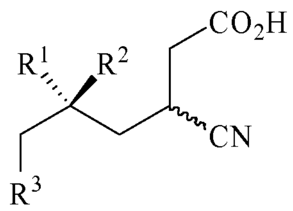

**8**

or a salt thereof to give a compound of Formula 9,

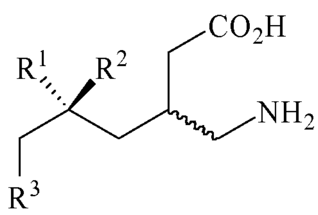

**9**

or a salt thereof, wherein $R^1$, $R^2$, and $R^3$ in Formula 8 and Formula 9 are as defined for Formula 1;
(b) optionally treating a salt of the compound of Formula 9 with an acid;
(c) resolving the compound of Formula 9 or a salt thereof; and
(d) optionally converting the compound of Formula 1 or a salt thereof into a pharmaceutically acceptable complex, salt, solvate or hydrate thereof.

An additional aspect of the invention provides a compound of Formula 19,

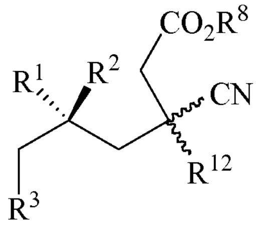

**19**

including salts thereof, wherein $R^1$, $R^2$, and $R^3$ are as defined for Formula 1, above;
$R^8$ is selected from hydrogen, $C_{1-6}$ alkyl, $C_{2-6}$ alkenyl, $C_{2-6}$ alkynyl, $C_{3-7}$ cycloalkyl, $C_{3-7}$ cycloalkenyl, halo-$C_{1-6}$ alkyl, halo-$C_{2-6}$ alkenyl, halo-$C_{2-6}$ alkynyl, aryl-$C_{1-6}$ alkyl, aryl-$C_{2-6}$ alkenyl, and aryl-$C_{2-6}$ alkynyl;
$R^{12}$ is hydrogen or -C(O)OR$^7$; and
$R^7$ is selected from $C_{1-6}$ alkyl, $C_{2-6}$ alkenyl, $C_{2-6}$ alkynyl, $C_{3-7}$ cycloalkyl, $C_{3-7}$ cycloalkenyl, halo-$C_{1-6}$ alkyl, halo-$C_{2-6}$ alkenyl, halo-$C_{2-6}$ alkynyl, aryl-$C_{1-6}$ alkyl, aryl-$C_{2-6}$ alkenyl, and aryl-$C_{2-6}$ alkynyl; wherein each of the aforementioned aryl moieties is optionally substituted with from one to three substituents independently selected from $C_{1-3}$ alkyl, $C_{1-3}$ alkoxy, amino, $C_{1-3}$ alkylamino, and halogeno; and

wherein each of the aforementioned alkyl, alkenyl, cycloalkyl, and alkoxy moieties are optionally substituted with from one to three fluorine atoms.

A further aspect of the invention provides compounds of Formula 7, Formula 8, Formula 10, Formula 11, and Formula 10a, above, selected from:

(2'*R*)-2-cyano-2-(2'-methyl-butyl)-succinic acid diethyl ester;
(2'*R*)-2-cyano-2-(2'-methyl-pentyl)-succinic acid diethyl ester;
(2'*R*)-2-cyano-2-(2'-methyl-hexyl)-succinic acid diethyl ester;
(2'*R*)-2-cyano-2-(2',4'-dimethyl-pentyl)-succinic acid diethyl ester;
(5*R*)-3-cyano-5-methyl-heptanoic acid ethyl ester;
(5*R*)-3-cyano-5-methyl-octanoic acid ethyl ester;
(5*R*)-3-cyano-5-methyl-nonanoic acid ethyl ester;
(5*R*)-3-cyano-5,7-dimethyl-octanoic acid ethyl ester;
(5*R*)-3-cyano-5-methyl-heptanoic acid;
(5*R*)-3-cyano-5-methyl-octanoic acid;
(5*R*)-3-cyano-5-methyl-nonanoic acid;
(5*R*)-3-cyano-5,7-dimethyl-octanoic acid;
(3*S*,5*R*)-3-cyano-5-methyl-heptanoic acid;
(3*S*,5*R*)-3-cyano-5-methyl-octanoic acid;
(3*S*,5*R*)-3-cyano-5-methyl-nonanoic acid;
(3*S*,5*R*)-3-cyano-5,7-dimethyl-octanoic acid;
(3*S*,5*R*)-3-cyano-5-methyl-heptanoic acid ethyl ester;
(3*S*,5*R*)-3-cyano-5-methyl-octanoic acid ethyl ester;
(3*S*,5*R*)-3-cyano-5-methyl-nonanoic acid ethyl ester;
(3*S*,5*R*)-3-cyano-5,7-dimethyl-octanoic acid ethyl ester;
(3*R*,5*R*)-3-cyano-5-methyl-heptanoic acid;
(3*R*,5*R*)-3-cyano-5-methyl-octanoic acid;
(3*R*,5*R*)-3-cyano-5-methyl-nonanoic acid;
(3*R*,5*R*)-3-cyano-5,7-dimethyl-octanoic acid;
(3*R*,5*R*)-3-cyano-5-methyl-heptanoic acid ethyl ester;

(3*R*,5*R*)-3-cyano-5-methyl-octanoic acid ethyl ester;
(3*R*,5*R*)-3-cyano-5-methyl-nonanoic acid ethyl ester;
(3*R*,5*R*)-3-cyano-5,7-dimethyl-octanoic acid ethyl ester;

and the salts thereof.

The invention further provides, as Embodiment E, essentially pure, crystalline (3S,5R)-3-aminomethyl-5-methyl-octanoic acid Form A, which is characterized by a powder X-ray diffraction pattern (PXRD) obtained by irradiation with CuK$\alpha$ radiation which includes peaks at 7.7, 15.8, 20.8 and 23.1 degrees of two theta-angle $\pm$ 0.2 degree.

As Embodiment E1, the invention provides essentially pure, crystalline (3*S*,5*R*)-3-aminomethyl-5-methyl-octanoic acid Form A, which is characterized by a differential scanning calorimetry (DSC) thermogram showing a single sharp endotherm peak maximum at 194°C $\pm$ 2°C.

As Embodiment E2, the invention provides essentially pure, crystalline (3*S*,5*R*)-3-aminomethyl-5-methyl-octanoic acid Form A, which is characterized by a Fourier Transform Infrared (FT-IR) spectrum which includes absorption bands at 1006 and 894 $cm^{-1}$.

As Embodiment E3, the invention provides essentially pure, crystalline (3*S*,5*R*)-3-aminomethyl-5-methyl-octanoic acid Form A, which is characterized by a Fourier Transform-Raman (FT-Ramon) spectrum which includes absorption bands at 1550, 595 and 386 $cm^{-1}$.

The expression 'essentially pure' when used herein means at least 95% by weight purity. More preferably, 'essentially pure' means at least 98% by weight purity and most preferably means at least 99% by weight purity.

As Embodiment E4, the invention provides (3*S*,5*R*)-3-aminomethyl-5-methyl-octanoic acid Form A for use as a medicament.

As Embodiment E5, the invention provides (3*S*,5*R*)-3-aminomethyl-5-methyl-octanoic acid Form A for use in the treatment of a disease or disorder for which an alpha-2-delta receptor ligand is indicated, particularly for the treatment of a disease or disorder selected from epilepsy; pain (e.g., acute and chronic pain, neuropathic pain, and psychogenic pain); neurodegenerative disorders (e.g., acute brain injury arising from stroke, head trauma, and asphyxia); psychiatric disorders (e.g., anxiety and depression); sleep disorders (e.g., insomnia, drug-associated sleeplessness, hypersomnia, narcolepsy, sleep apnea, and parasomnias); obsessive compulsive disorder (OCD); phobias; post traumatic stress disorder (PTSD); restless legs syndrome; premenstrual dysphoric disorder; vasomotor symptoms (hot flashes and night sweats); and fibromyalgia.

As Embodiment E6, the invention provides the use of (3*S*,5*R*)-3-aminomethyl-5-methyl-octanoic acid Form A in the manufacture of a medicament for the treatment of a disease or disorder for which an alpha-2-delta receptor ligand is indicated, particularly for the treatment of a disease or disorder selected from epilepsy; pain (e.g., acute and chronic pain, neuropathic pain, and psychogenic pain); neurodegenerative disorders (e.g., acute brain injury arising from stroke, head trauma, and asphyxia); psychiatric disorders (e.g., anxiety and depression); sleep disorders (e.g., insomnia, drug-associated sleeplessness, hypersomnia, narcolepsy, sleep apnea, and parasomnias); obsessive compulsive disorder (OCD); phobias; post traumatic stress disorder (PTSD); restless legs syndrome; premenstrual dysphoric disorder; vasomotor symptoms (hot flashes and night sweats); and fibromyalgia.

As Embodiment E7, the invention provides a method of treating a disease or disorder for which an alpha-2-delta receptor ligand is indicated in a mammal, particularly a disease or disorder selected from epilepsy; pain (e.g., acute and chronic pain, neuropathic pain, and psychogenic pain); neurodegenerative disorders (e.g., acute brain injury arising from stroke, head trauma, and asphyxia); psychiatric disorders (e.g., anxiety and depression); sleep disorders (e.g., insomnia, drug-associated sleeplessness, hypersomnia, narcolepsy, sleep apnea, and parasomnias); obsessive compulsive disorder (OCD); phobias; post traumatic stress disorder (PTSD); restless legs syndrome; premenstrual dysphoric disorder; vasomotor symptoms (hot flashes and night sweats); and fibromyalgia, comprising administering comprising administering to a mammal in need of such treatment (3*S*,5*R*)-3-aminomethyl-5-methyl-octanoic acid Form A.

The treatment of vasomotor symptoms (hot flashes and night sweats) is a preferred use.

As Embodiment E8, the invention provides a pharmaceutical composition including (3S,5R)-3-aminomethyl-5-methyl-octanoic acid Form A and one or more pharmaceutically acceptable excipients. As Embodiment E9, the invention provides a process for preparing (3S,5R)-3-aminomethyl-5-methyl-octanoic acid Form A by recrystallisation from a solution of crude (3S,5R)-3-aminomethyl-5-methyl-octanoic acid in a mixture of ethanol and water or isopropyl alcohol (IPA) and water; more preferably from a solution of crude (3*S*,5*R*)-3-aminomethyl-5-methyl-octanoic acid in a 1:1 mixture by volume of ethanol:water or a 1:1 mixture by volume of IPA:water; most preferably from a solution of crude (3*S*,5*R*)-3-aminomethyl-5-methyl-octanoic acid in a 1:1 mixture by volume of ethanol:water.

The present invention includes all complexes and salts, whether pharmaceutically acceptable or not, solvates, hydrates, and polymorphic forms of the disclosed compounds. Certain compounds may contain an alkenyl or cyclic group, so that *cis*/*trans* (or Z/E) stereoisomers are possible, or may contain a keto or oxime group, so that tautomerism may occur. In such cases, the present invention generally includes all Z/E isomers and tautomeric forms, whether they are pure, substantially pure, or mixtures.

Unless otherwise indicated, this disclosure uses definitions provided below. Some of the definitions and formulae may include a dash ("-") to indicate a bond between atoms or a point of attachment to a named or unnamed atom or group of atoms. Other definitions and formulae may include an equal sign ("=") or an identity symbol ("≡") to indicate a double bond or a triple bond, respectively. Certain formulae may also include one or more asterisks ("*") to indicate stereogenic (asymmetric or chiral) centers, although the absence of an asterisk does not indicate that the compound lacks a stereocenter. Such formulae may refer to the racemate or to individual enantiomers or to individual diastereomers, which may or may not be pure or substantially pure. Other formulae may include one or more wavy bonds ("〰"). When attached to a stereogenic center, the wavy bonds refer to both stereoisomers, either individually or as mixtures. Likewise, when attached to a double bond, the wavy bonds indicate a Z-isomer, an E-isomer, or a mixture of ***Z*** and *E* isomers. Some formulae may include a dashed bond "$\overline{-----}$" to indicate a single or a double bond.

"Substituted" groups are those in which one or more hydrogen atoms have been replaced with one or more non-hydrogen atoms or groups, provided that valence requirements are met and that a chemically stable compound results from the substitution.

"About" or "approximately," when used in connection with a measurable numerical variable, refers to the indicated value of the variable and to all values of the variable that are within the experimental error of the indicated value (e.g., within the 95% confidence interval for the mean) or within $\pm 10$ percent of the indicated value, whichever is greater.

"Alkyl" refers to straight chain and branched saturated hydrocarbon groups, generally having a specified number of carbon atoms (i.e., $C_{1-3}$ alkyl refers to an alkyl group having 1, 2 or 3 carbon atoms and $C_{1-6}$ alkyl refers to an alkyl group having 1, 2, 3, 4, 5 or 6 carbon atoms). Examples of alkyl groups include methyl, ethyl, *n*-propyl, *i*-propyl, *n*-butyl, s-butyl, *i*-butyl, t-butyl, pent-1-yl, pent-2-yl, pent-3-yl, 3-methylbut-1-yl, 3-methylbut-2-yl, 2-methylbut-2-yl, 2,2,2-trimethyleth-1-yl, *n*-hexyl, and the like.

"Alkenyl" refers to straight chain and branched hydrocarbon groups having one or more unsaturated carbon-carbon bonds, and generally having a specified number of carbon atoms. Examples of alkenyl groups include ethenyl, 1-propen-1-yl, 1-propen-2-yl, 2-propen-1-yl, 1-buten-1-yl, 1-buten-2-yl, 3-buten-1-yl, 3-buten-2-yl, 2-buten-1-yl, 2-buten-2-yl, 2-methyl-1-propen-1-yl, 2-methyl-2-propen-1-yl, 1,3-butadien-1-yl, 1,3-butadien-2-yl, and the like.

"Alkynyl" refers to straight chain or branched hydrocarbon groups having one or more triple carbon-carbon bonds, and generally having a specified number of carbon atoms. Examples of alkynyl groups include ethynyl, 1-propyn-1-yl, 2-propyn-1-yl, 1-butyn-1-yl, 3-butyn-1-yl, 3-butyn-2-yl, 2-butyn-1-yl, and the like.

"Alkoxy" refers to alkyl-O-, alkenyl-O, and alkynyl-O, where alkyl, alkenyl, and alkynyl are defined above. Examples of alkoxy groups include methoxy, ethoxy, *n*-propoxy, *i*-propoxy, *n*-butoxy, *s*-butoxy, t-butoxy, *n*-pentoxy, s-pentoxy, and the like.

"Halo," "halogen" and "halogeno" may be used interchangeably, and refer to fluoro, chloro, bromo, and iodo.

"Haloalkyl," "haloalkenyl," "haloalkynyl," and "haloalkoxy," refer, respectively, to alkyl, alkenyl, alkynyl, and alkoxy, groups substituted with one or more halogen atoms, where alkyl, alkenyl, alkynyl, and alkoxy are defined above. Examples of haloalkyl groups include trifluoromethyl, trichloromethyl, pentafluoroethyl, pentachloroethyl, and the like.

"Cycloalkyl" refers to saturated monocyclic and bicyclic hydrocarbon rings, generally having a specified number of carbon atoms that comprise the ring (i.e., $C_{3-7}$ cycloalkyl refers to a cycloalkyl group having 3, 4, 5, 6 or 7 carbon atoms as ring members). The cycloalkyl may be attached to a parent group or to a substrate at any ring atom, unless such attachment would violate valence requirements. Likewise, the cycloalkyl groups may include one or more non-hydrogen substituents unless such substitution would violate valence requirements. Useful substituents include alkyl, alkenyl, alkynyl, haloalkyl, haloalkenyl, haloalkynyl, alkoxy, alkoxycarbonyl, alkanoyl, and halo, as defined above, and hydroxy, mercapto, nitro, and amino.

Examples of monocyclic cycloalkyl groups include cyclopropyl, cyclobutyl, cyclopentyl, cyclohexyl, and the like. Examples of bicyclic cycloalkyl groups include bicyclo[1.1.0]butyl, bicyclo[1.1.1]pentyl, bicyclo[2.1.0]pentyl, bicyclo[2.1.1]hexyl, bicyclo[3.1.0]hexyl, bicyclo[2.2.1]heptyl, bicyclo[3.2.0]heptyl, bicyclo[3.1.1]heptyl, bicyclo[4.1.0]heptyl, bicyclo[2.2.2]octyl, bicyclo[3.2.1]octyl, bicyclo[4.1.1]octyl, bicyclo[3.3.0]octyl, bicyclo[4.2.0]octyl, bicyclo[3.3.1]nonyl, bicyclo[4.2.1]nonyl, bicyclo[4.3.0]nonyl, bicyclo[3.3.2]decyl, bicyclo[4.2.2]decyl, bicyclo[4.3.1]decyl, bicyclo[4.4.0]decyl, bicyclo[3.3.3]undecyl, bicyclo[4.3.2]undecyl, bicyclo[4.3.3]dodecyl, and the like.

"Cycloalkenyl" refers monocyclic and bicyclic hydrocarbon rings having one or more unsaturated carbon-carbon bonds and generally having a specified number of carbon atoms that comprise the ring (i.e., $C_{3-7}$ cycloalkenyl refers to a cycloalkenyl group having 3, 4, 5, 6 or 7 carbon atoms as ring members). The cycloalkenyl may be attached to a parent group or to a substrate at any ring atom, unless such attachment would violate valence requirements. Likewise, the cycloalkenyl groups may include one or more non-hydrogen substituents unless such substitution would violate valence requirements. Useful substituents include alkyl, alkenyl, alkynyl, haloalkyl, haloalkenyl, haloalkynyl, alkoxy, alkoxycarbonyl, alkanoyl, and halo, as defined above, and hydroxy, mercapto, nitro, and amino.

"Aryl" and "arylene" refer to monovalent and divalent aromatic groups, respectively, including 5- and 6-membered monocyclic aromatic groups that contain 0 to 4 heteroatoms independently selected from nitrogen, oxygen, and

sulfur. Examples of monocyclic aryl groups include phenyl, pyrrolyl, furanyl, thiopheneyl, thiazolyl, isothiazolyl, imidazolyl, triazolyl, tetrazolyl, pyrazolyl, oxazolyl, isooxazolyl, pyridinyl, pyrazinyl, pyridazinyl, pyrimidinyl, and the like. Aryl and arylene groups also include bicyclic groups, tricyclic groups, etc., including fused 5- and 6-membered rings described above. Examples of multicyclic aryl groups include naphthyl, biphenyl, anthracenyl, pyrenyl, carbazolyl, benzoxazolyl, benzodioxazolyl, benzothiazolyl, benzoimidazolyl, benzothiopheneyl, quinolinyl, isoquinolinyl, indolyl, benzofuranyl, purinyl, indolizinyl, and the like. They aryl and arylene groups may be attached to a parent group or to a substrate at any ring atom, unless such attachment would violate valence requirements. Likewise, aryl and arylene groups may include one or more non-hydrogen substituents unless such substitution would violate valence requirements. Useful substituents include alkyl, alkenyl, alkynyl, haloalkyl, haloalkenyl, haloalkynyl, cycloalkyl, cycloalkenyl, alkoxy, cycloalkoxy, alkanoyl, cycloalkanoyl, cycloalkenoyl, alkoxycarbonyl, cycloalkoxycarbonyl, and halo, as defined above, and hydroxy, mercapto, nitro, amino, and alkylamino.

"Arylalkyl" refers to aryl-alkyl where aryl and alkyl are defined above. Examples include benzyl, fluorenylmethyl, and the like.

"Leaving group" refers to any group that leaves a molecule during a fragmentation process, including substitution reactions, elimination reactions, and addition-elimination reactions. Leaving groups may be nucleofugal, in which the group leaves with a pair of electrons that formerly served as the bond between the leaving group and the molecule, or may be electrofugal, in which the group leaves without the pair of electrons. The ability of a nucleofugal leaving group to leave depends on its base strength, with the strongest bases being the poorest leaving groups. Common nucleofugal leaving groups include nitrogen (e.g., from diazonium salts); sulfonates, including alkylsulfonates (e.g., mesylate), fluoroalkylsulfonates (e.g., triflate, hexaflate, nonaflate, and tresylate), and arylsulfonates (e.g., tosylate, brosylate, closylate, and nosylate). Others include carbonates, halide ions, carboxylate anions, phenolate ions, and alkoxides. Some stronger bases, such as $NH_2^-$ and $OH^-$ can be made better leaving groups by treatment with an acid. Common electrofugal leaving groups include the proton, $CO_2$, and metals.

"Enantiomeric excess" or "ee" is a measure, for a given sample, of the excess of one enantiomer over a racemic sample of a chiral compound and is expressed as a percentage. Enantiomeric excess is defined as 100 x (er - 1) / (er + 1), where "er" is the ratio of the more abundant enantiomer to the less abundant enantiomer.

"Diastereomeric excess" or "de" is a measure, for a given sample, of the excess of one diastereomer over a sample having equal amounts of diastereomers and is expressed as a percentage. Diastereomeric excess is defined as 100 x (dr - 1) / (dr + 1), where "dr" is the ratio of a more abundant diastereomer to a less abundant diastereomer.

"Stereoselective," "enantioselective," "diastereoselective," and variants thereof, refer to a given process (e.g., hydrogenation) that yields more of one stereoisomer, enantiomer, or diastereoisomer than of another, respectively.

"High level of stereoselectivity," "high level of enantioselectivity," "high level of diastereoselectivity," and variants thereof, refer to a given process that yields products having an excess of one stereoisomer, enantiomer, or diastereoisomer, which comprises at least about 90% of the products. For a pair of enantiomers or diastereomers, a high level of enantioselectivity or diastereoselectivity would correspond to an ee or de of at least about 80%.

"Stereoisomerically enriched," "enantiomerically enriched," "diastereomerically enriched," and variants thereof, refer, respectively, to a sample of a compound that has more of one stereoisomer, enantiomer or diastereomer than another. The degree of enrichment may be measured by % of total product, or for a pair of enantiomers or diastereomers, by ee or de.

"Stereoisomers" of a specified compound refer to the opposite enantiomer of the compound and to any diastereoisomers or geometric isomers (Z/E) of the compound. For example, if the specified compound has S,R,Z stereochemical configuration, its stereoisomers would include its opposite enantiomer having R,S,Z configuration, its diastereomers having S,S,Z configuration and R,R,Z configuration, and its geometric isomers having *S,R,E* configuration, *R,S,E* configuration, *S,S,E* configuration, and *R,R,E* configuration. "Substantially pure stereoisomer," "substantially pure enantiomer," "substantially pure diastereomer," and variants thereof, refer, respectively, to a sample containing a stereoisomer, enantiomer, or diastereomer, which comprises at least about 95% of the sample. For pairs of enantiomers and diastereomers, a substantially pure enantiomer or diastereomer would correspond to samples having an ee or de of about 90% or greater. A "pure stereoisomer," "pure enantiomer," "pure diastereomer," and variants thereof, refer, respectively, to a sample containing a stereoisomer, enantiomer, or diastereomer, which comprises at least about 99.5% of the sample. For pairs of enantiomers and diastereomers, a pure enantiomer or pure diastereomer" would correspond to samples having an ee or de of about 99% or greater.

"Opposite enantiomer" refers to a molecule that is a non-superimposable mirror image of a reference molecule, which may be obtained by inverting all of the stereogenic centers of the reference molecule. For example, if the reference molecule has S absolute stereochemical configuration, then the opposite enantiomer has R absolute stereochemical configuration. Likewise, if the reference molecule has S,S absolute stereochemical configuration, then the opposite enantiomer has R,R stereochemical configuration, and so on.

"Enantioselectivity value" or "E" refers to the ratio of specificity constants for each enantiomer (or for each stereoisomer of a pair of diastereomers) of a compound undergoing chemical reaction or conversion and may be calculated (for the S-enantiomer) from the expression,

$$E = \frac{K_S / K_{SM}}{K_R / K_{RM}} = \frac{\ln\left[1 - \chi\left(1 + ee_p\right)\right]}{\ln\left[1 - \chi\left(1 - ee_p\right)\right]} = \frac{\ln\left[1 - \chi\left(1 - ee_S\right)\right]}{\ln\left[1 - \chi\left(1 + ee_S\right)\right]},$$

where $K_S$ and $K_R$ are the 1 st order rate constants for the conversion of the *S*- and *R*-enantiomers, respectively; $K_{SM}$ and $K_{RM}$ are the Michaelis constants for the S- and *R*-enantiomers, respectively; $\chi$ is the fractional conversion of the substrate; $ee_p$ and $ee_s$ are the enantiomeric excess of the product and substrate (reactant), respectively.

"Lipase Unit" or "LU" refers to the amount of enzyme (in g) that liberates 1 μmol of titratable butyric acid/min when contacted with tributyrin and an emulsifier (gum arabic) at 30°C and pH 7.

"Solvate" refers to a molecular complex comprising a disclosed or claimed compound and a stoichiometric or non-stoichiometric amount of one or more solvent molecules (e.g., ethanol).

"Hydrate" refers to a solvate comprising a disclosed or claimed compound and a stoichiometric or non-stoichiometric amount of water.

"Pharmaceutically acceptable complexes, salts, solvates, or hydrates" refers to complexes, acid or base addition salts, solvates or hydrates of claimed and disclosed compounds, which are within the scope of sound medical judgment, suitable for use in contact with the tissues of patients without undue toxicity, irritation, allergic response, and the like, commensurate with a reasonable benefit/risk ratio, and effective for their intended use.

"Pre-catalyst" or "catalyst precursor" refers to a compound or set of compounds that are converted into a catalyst prior to use.

"Treating" refers to reversing, alleviating, inhibiting the progress of, or preventing a disorder or condition to which such term applies, or to preventing one or more symptoms of such disorder or condition. "Treatment" refers to the act of "treating," as defined immediately above.

Table 1 lists abbreviations used throughout the specification.

TABLE 1. List of Abbreviations

| Abbreviation | Description |
|---|---|
| Ac | acetyl |
| ACN | acetonitrile |
| $Ac_2O$ | acetic anhydride |
| aq | aqueous |
| (R,R)-BDPP | (2*R*,4*R*)-(+)-2,4-bis(diphenylphosphino)pentane |
| BES | N,N-bis(2-hydroxyethyl)-2-aminoethanesulfonic acid |
| (*R*)-BICHEP | (*R*)-(-)-2,2'-bis(dicyclohexylphosphino)-6,6'-dimethyl-1,1'-biphenyl |
| BICINE | *N,N*-bis(2-hydroxyethyl)glycine |
| (*S,S*)-BICP | (2*S*,2'*S*)-bis(diphenylphosphino)-(1*S*,1'*S*)-bicyclopentane |
| BIFUP | 2,2'-bis(diphenylphosphino)-4,4',6,6'-tetrakis(trifluoromethyl)-1,1'-biphenyl |
| (*R*)-Tol-BINAP | (*R*)-(+)-2,2'-bis(di-*p*-tolylphosphino)-1,1'-binaphthyl |
| (*S*)-Tol-BINAP | (*S*)-(+)-2,2'-bis(di-*p*-tolylphosphino)-1,1'-binaphthyl |
| (*R*)-BINAP | (*R*)-2,2'-bis(diphenylphosphino)-1'1-binaphthyl |
| (*S*)-BINAP | (*S*)-2,2'-bis(diphenylphosphino)-1'1-binaphthyl |
| BIPHEP | 2,2'-bis(diphenylphosphino)-1,1'-biphenyl |
| (*R*)-MeO-BIPHEP | (*R*)-(6,6'-dimethoxybiphenyl-2,2'-diyl)-bis(diphenylphosphine) |
| (*R*)-Cl-MeO-BIPHEP | (*R*)-(+)-5,5'-dichloro-6,6'-dimethoxy-2,2'-bis(diphenylphosphino)-1,1'-biphenyl |
| (*S*)-Cl-MeO-BIPHEP | (*S*)-(+)-5,5'-dichloro-6,6'-dimethoxy-2,2'-bis(diphenylphosphino)-1,1'-biphenyl |
| BisP* | (*S,S*)-1,2-bis(*t*-butylmethylphosphino)ethane |
| (+)-tetraMeBITIANP | (*S*)-(+)-2,2'-bis(diphenylphosphino)-4,4',6,6'-tetramethyl-3,3'-bibenzo[*b*]thiophene |
| Bn | benzyl |
| BnBr, BnCl | benzylbromide, benzylchloride |
| Boc | *t*-butoxycarbonyl |
| BOP | benzotriazol-1-yloxy-tris-(dimethylamino)-phosphonium hexafluorophosphate |
| (R)-(S)-BPPFA | (-)-(*R*)--*N,N*-dimethyl-1-((*S*)-1',2-bis(diphenylphosphino)ferrocenyl)ethylamine |

(continued)

| Abbreviation | Description |
|---|---|
| (R,R)-Et-BPE | (+)-1,2-bis((2R,5i)-2,5-diethylphospholano)ethane |
| (R,R)-Me-BPE | (+)-1,2-bis((2R,5R)-2,5-dimethylphospholano)ethane |
| (S,S)-BPPM | (-)-(2*S*,4*S*)-2-diphenylphosphinomethyl-4-diphenylphosphino-1-*t*-butoxycarbonylpyrrolidine |
| Bs | brosyl or *p*-bromo-benzenesulfonyl |
| Bu | butyl |
| *n*-BuLi | *n*-butyl lithium |
| t-Bu | tertiary butyl |
| $Bu_4N^+Br^-$ | tetrabutyl-ammonium bromide |
| *t*-BuOK | potassium tertiary-butoxide |
| *t*-BuOLi | lithium tertiary-butoxide |
| *t*-BuOMe | tertiary butyl methyl ether |
| *t*-BuONa | sodium tertiary butyl oxide |
| (+)-CAMP | (*R*)-(+)-cyclohexyl(2-anisyl)methylphosphine; a monophosphine |
| CARBOPHOS | methyl-α-D-glucopyranoside-2,6-dibenzoate-3,4-di(bis(3,5-dimethylphenyl) phosphinite) |
| Cbz | benzyloxycarbonyl |
| CDI | *N,N*-carbonyldiimidazole |
| $\chi$ | fractional conversion |
| CnTunaPHOS | 2,2'-bis-diphenylphosphanyl-biphenyl having an -O- $(CH_2)_n$-O-group linking the 6,6' carbon atoms of the biphenyl (e.g., (R)-1,14-bis-diphenylphosphanyl-6,7,8,9-tetrahydro-5,10-dioxa-dibenzo[a,c]cyclodecene for n=4). |
| COD | 1,5-cyclooctadiene |
| (*R*)-CYCPHOS | (*R*)-1,2-bis(diphenylphosphino)-1-cyclohexylethane |
| DABCO | 1,4-diazabicyclo[2.2.2]octane |
| DBAD | di-t-butyl azodicarboxylate |
| DBN | 1,5-diazabicyclo[4.3.0]non-5-ene |
| DBU | 1,8-diazabicyclo[5.4.0]undec-7-ene |
| DCC | dicycohexylcarbodiimide |
| de | diastereomeric excess |
| DEAD | diethyl azodicarboxylate |
| (*R,R*)-DEGUPHOS | *N*-benzyl-(3*R*,4*R*)-3,4-bis(diphenylphosphino)pyrrolidine |
| DIAD | diisopropyl azodicarboxylate |
| (*R,R*)-DIOP | (4*R*,5*R*)-(-)-O-isopropylidene-2,3-dihydroxy-1,4-bis(diphenylphosphino)butane |
| (*R,R*)-DIPAMP | (*R,R*)-(-)-1,2-bis[(*O*-methoxyphenyl)(phenyl)phosphino]ethane |
| DIPEA | diisopropylethylamine (Hünig's Base) |
| DMAP | 4-(dimethylamino) pyridine |
| DMF | dimethylformamide |
| DMSO | dimethylsulfoxide |
| DMT-MM | 4-(4,6-dimethoxy-1,3,5-triazin-2-yl)-4-methylmorpholinium chloride |
| (*R,R*)-Et-DUPHOS | (-)-1,2-bis((2*R*,5*R*)-2,5-diethylphospholano)benzene |
| (*S,S*)-Et-DUPHOS | (-)-1,2-bis((2*S*,5*S*)-2,5-diethylphospholano)benzene |
| (*R,R*)-*i*-Pr-DUPHOS | (+)-1,2-bis((2*R*,5*R*)-2,5-di-*i*-propylphospholano)benzene |
| (*R,R*)-Me-DUPHOS | (-)-1,2-bis((2*R*,5*R*)-2,5-dimethylphospholano)benzene |
| (*S,S*)-Me-DUPHOS | (-)-1,2-bis((2*S*,5*S*)-2,5-dimethylphospholano)benzene |
| E | Enantioselectivity value or ratio of specificity constants for each enantiomer of a compound undergoing chemical reaction or conversion |
| EDCI | 1-(3-dimethylaminopropyl)-3-ethylcarbodiimide |
| ee ($ee_p$ or $ee_s$) | enantiomeric excess (of product or reactant) |
| eq | equivalents |

(continued)

| Abbreviation | Description |
|---|---|
| er | enantiomeric ratio |
| Et | ethyl |
| $Et_3N$ | triethyl-amine |
| EtOAc | ethyl acetate |
| $Et_2O$ | diethyl ether |
| EtOH | ethyl alcohol |
| FDPP | pentafluorophenyl diphenylphosphinate |
| (*R*,*R*)-Et-FerroTANE | 1,1'-bis((2*R*,4*R*)-2,4-diethylphosphotano)ferrocene |
| Fmoc | 9-fluoroenylmethoxycarbonyl |
| GC | gas chromatography |
| h, min, s | hour(s), minute(s), second(s) |
| HEPES | 4-(2-hydroxyethyl)piperazine-1-ethanesulfonic acid |
| HOAc | acetic acid |
| HOAt | 1-hyd roxy-7-azabenzotriazole |
| HOBt | *N*-hydroxybenzotriazole |
| HODhbt | 3-hydroxy-3,4-dihydro-4-oxo-1,2,3-benzotriazine |
| HPLC | high performance liquid chromatography |
| IAcOEt | ethyl iodoacetate |
| IPA | isopropanol |
| *i*-PrOAc | isopropyl acetate |
| (*R*)-(*R*)-JOSIPHOS | (*R*)-(-)-1-[(*R*)-2-(diphenylphosphino)ferrocenyl]ethyldicyclohexylphosphine |
| (*S*)-(*S*)-JOSIPHOS | (*S*)-(-)-1-[(*S*)-2-(diphenylphosphino)ferrocenyl]ethyldicyclohexylphosphine |
| (*R*)-(*S*)-JOSIPHOS | (*R*)-(-)-1-[(*S*)-2-(diphenylphosphino)ferrocenyl]ethyldicyclohexylphosphine |
| KHMDS | potassium hexamethyldisilazane |
| KF | Karl Fischer |
| $K_S$, $K_S$ | 1st order rate constant for *S*- or *R*-enantiomer |
| $K_{SM}$, $K_{RM}$ | Michaelis constant for *S*- or *R*-enantiomer |
| LAH | lithium aluminum hydride |
| LC/MS | liquid chromatography mass spectrometry |
| LDA | lithium diisopropylamide |
| LHMDS | lithium hexamethyldisilazane |
| LICA | lithium isopropylcyclohexylamide |
| LTMP | 2,2,6,6-tetramethylpiperidine |
| LU | lipase unit |
| Me | methyl |
| $MeCl_2$ | methylene chloride |
| Mel | methyl iodide |
| MEK | methylethylketone or butan-2-one |
| MeOH | methyl alcohol |
| MeONa | sodium methoxide |
| MES | 2-morpholinoethanesulfonic acid |
| (*R*,*R*)-*t*-butyl-miniPHOS | (R,R)-1,2-bis(di-t-butylmethylphosphino)methane |
| (S,S) MandyPhos | (*S*,*S*)-(-)-2,2'-bis[(*R*)-(*N*,*N*-dimethylamino) (phenyl)methyl]-1,1'-bis (diphenylphosphino)ferrocene |
| (*R*)-MonoPhos | (*R*)-(-)-[4,*N*,*N*-dimethylamino]dinaphtho[2,1-d:1',2'-f][1,3,2]dioxaphosphepin |
| (R)-MOP | (*R*)-(+)-2-(diphenylphosphino)-2'-methoxy-1,1'-binaphthyl |
| MOPS | 3-(*N*-morpholino)propanesulfonic acid |
| MPa | mega Pascals |
| mp | melting point |
| Ms | mesyl or methanesulfonyl |

(continued)

| Abbreviation | Description |
|---|---|
| MTBE | methyl tertiary butyl ether |
| NMP | *N*-methylpyrrolidone |
| Ns | nosyl or nitrobenzene sulfonyl |
| (*R,R*)-NORPHOS | (2*R*,3*R*)-(-)-2,3-bis(diphenylphosphino)bicyclo[2.2.1]hept-5-ene |
| OTf | triflate (trifluoro-methanesulfonic acid anion) |
| $PdCl_2(dppf)_2$ | dichloro[1,1'-bis(diphenylphosphino)ferrocene]palladium (II) dichloromethane adduct |
| (*R,S,R,S*)-Me-PENNPHOS | (1*R*,2*S*,4*R*,5*S*)-2,5-dimethyl-7-phosphadicyclo[2.2.1]heptane |
| Ph | phenyl |
| $Ph_3P$ | triphenylphosphine |
| $Ph_3As$ | triphenylarsine |
| (*R*)-PHANEPHOS | (*R*)-(-)-4,12-bis(diphenylphosphino)-[2.2]-paracyclophane |
| (*S*)-PHANEPHOS | (*S*)-(-)-4,12-bis(diphenylphosphino)-[2.2]-paracyclophane |
| (*R*)-PNNP | *N,N'*-bis[(*R*)-(+)-a-methylbenzyl]-*N,N'*-bis(diphenylphosphino)ethylene diamine |
| $PPh_2$-PhOx-Ph | (*R*)-(-)-2-[2-(diphenylphosphino)phenyl]-4-phenyl-2-oxazoline |
| PIPES | piperazine-1,4-bis(2-ethanesulfonic acid) |
| Pr | propyl |
| *i*-Pr | isopropyl |
| (*R*)-PROPHOS | (*R*)-(+)-1,2-bis(diphenylphosphino)propane |
| PyBOP | benzotriazole-1-yl-oxy-tris-pyrrolidino-phosphonium hexafluorophosphate |
| (*R*)-QUINAP | (*R*)-(+)-1-(2-diphenylphosphino-1-naphthyl)isoquinoline |
| RaNi | Raney nickel |
| RI | refractive index |
| RT | room temperature (approximately 20°C to 25°C) |
| s/c | substrate-to-catalyst molar ratio |
| sp | species |
| (*R*)-SpirOP | (1*R*,5*R*,6*R*)-spiro[4.4]nonane-1,6-diyl-diphenylphosphinous acid ester; a spirocyclic phosphinite ligand |
| (*R,R,S,S*) TangPhos | (*R,R,S,S*) 1,1'-di-*t*-butyl-[2,2']biphospholanyl |
| TAPS | *N*-[tris(hydroxymethyl)methyl]-3-aminopropanesulfonic acid |
| TATU | *O*-(7-azabenzotriazol-1-yl)-1,1,3,3-tetramethyluronium tetrafluoroborate |
| (*R*)-eTCFP | (*R*)-2-{[(di-*t*-butyl-phosphanyl)-ethyl]-methyl-phosphanyl}-2-methyl-propane |
| (*S*)-eTCFP | (*S*)-2-{[(di-*t*-butyl-phosphanyl)-ethyl]-methyl-phosphanyl}-2-methyl-propane |
| (*R*)-mTCFP | (*R*)-2-{[(di-*t*-butyl-phosphanyl)-methyl]-methyl-phosphanyl}-2-methyl-propane |
| (*S*)-mTCFP | (*S*)-2-{[(di-*t*-butyl-phosphanyl)-methyl]-methyl-phosphanyl}-2-methyl-propane |
| TEA | triethanolamine |
| TES | *N*-[tris(hydroxymethyl)methyl]-2-aminoethanesulfonic acid |
| Tf | triflyl or trifluoromethylsulfonyl |
| TFA | trifluoroacetic acid |
| THF | tetrahydrofuran |
| TLC | thin-layer chromatography |
| TMEDA | *N,N,N',N'*-tetramethyl-1,2-ethylenediamine |
| TMS | trimethylsilyl |
| Tr | trityl or triphenylmethyl |
| TRICINE | *N*-[tris(hydroxymethyl)methyl]glycine |
| Tris buffer | tris(hydroxymethyl)aminomethane buffer |
| TRITON B | benzyltrimethylammonium hydroxide |
| TRIZMA® | 2-amino-2-(hydroxymethyl)-1,3-propanediol |
| Ts | tosyl or *p*-toluenesulfonyl |
| *p*-TSA | para-toluene sulfonic acid |
| v/v | volume percent |

(continued)

| Abbreviation | Description |
|---|---|
| w/w | weight (mass) percent |

Some of the schemes and examples below may omit details of common reactions, including oxidations, reductions, and so on, separation techniques, and analytical procedures, which are known to persons of ordinary skill in the art of organic chemistry. The details of such reactions and techniques can be found in a number of treatises, including Richard Larock, *Comprehensive Organic Transformations* (1999), and the multi-volume series edited by Michael B. Smith and others, *Compendium of Organic Synthetic Methods* (1974-2005). In many cases, starting materials and reagents may be obtained from commercial sources or may be prepared using literature methods. Some of the reaction schemes may omit minor products resulting from chemical transformations (e.g., an alcohol from the hydrolysis of an ester, $CO_2$ from the decarboxylation of a diacid, etc.). In addition, in some instances, reaction intermediates may be used in subsequent steps without isolation or purification (i.e., in situ).

In some of the reaction schemes and examples below, certain compounds can be prepared using protecting groups, which prevent undesirable chemical reaction at otherwise reactive sites. Protecting groups may also be used to enhance solubility or otherwise modify physical properties of a compound. For a discussion of protecting group strategies, a description of materials and methods for installing and removing protecting groups, and a compilation of useful protecting groups for common functional groups, including amines, carboxylic acids, alcohols, ketones, aldehydes, and the like, see T. W. Greene and P. G. Wuts, Protecting Groups in Organic Chemistry (1999) and P. Kocienski, Protective Groups (2000), which are herein incorporated by reference in their entirety for all purposes.

Generally, the chemical transformations described throughout the specification may be carried out using substantially stoichiometric amounts of reactants, though certain reactions may benefit from using an excess of one or more of the reactants. Additionally, many of the reactions disclosed throughout the specification may be carried out at about room temperature and ambient pressure, but depending on reaction kinetics, yields, and the like, some reactions may be run at elevated pressures or employ higher (e.g., reflux conditions) or lower (e.g., -70°C to 0°C) temperatures. Many of the chemical transformations may also employ one or more compatible solvents, which may influence the reaction rate and yield. Depending on the nature of the reactants, the one or more solvents may be polar protic solvents (including water), polar aprotic solvents, non-polar solvents, or some combination. Any reference in the disclosure to a stoichiometric range, a temperature range, a pH range, etc., whether or not expressly using the word "range," also includes the indicated endpoints. Generally, and unless stated otherwise, when a particular substituent identifier ($R^1$, $R^2$, $R^3$, etc.) is defined for the first time in connection with a formula, the same substituent identifier, when used in a subsequent formula, will have the same definition as in the earlier formula. Thus, for example, if $R^{30}$ in a first Formula 1 is hydrogen, halogeno, or $C_{1-6}$ alkyl, then unless stated differently or otherwise clear from the context of the text, $R^{30}$ in a second Formula 1 is also hydrogen, halogeno, or $C_{1-6}$ alkyl. This disclosure concerns materials and methods for preparing optically active γ-amino acids of Formula 1, above, as well as their stereoisomers (e.g., diastereomers and opposite enantiomers) and their pharmaceutically acceptable complexes, salts, solvates and hydrates. The claimed and disclosed methods provide compounds of Formula 1 (or their stereoisomers) that are stereoisomerically enriched, and which in many cases, are pure or substantially pure stereoisomers. For clarity, the specification describes methods and materials for preparing intermediates and final products having specific stereochemical configurations. However, by using starting materials, resolving agents, chiral catalysts, enzymes, and the like, having different stereochemical configurations, the methods may be used to prepare the corresponding diastereomers and opposite enantiomers of the disclosed products and intermediates.

The compounds of Formula 1 have at least two stereogenic centers, as denoted by wedged bonds, and include substituents $R^1$, $R^2$, and $R^3$, which are defined above. Compounds of Formula 1 include those in which $R^1$ and $R^2$ are each independently hydrogen or methyl, provided that $R^1$ and $R^2$ are not both hydrogen, and those in which $R^3$ is $C_{1-6}$ alkyl, including methyl, ethyl, *n*-propyl or *i*-propyl. Representative compounds of Formula 1 also include those in which $R^1$ is hydrogen, $R^2$ is methyl, and $R^3$ is methyl, ethyl, *n*-propyl, or *i*-propyl, i.e., (3*S*,5*R*)-3-aminomethyl-5-methyl-heptanoic acid, (3*S*,5*R*)-3-aminomethyl-5-methyl-octanoic acid, (3*S*,5*R*)-3-aminomethyl-5-methyl-nonanoic acid, or (3*S*,5*R*)-3-aminomethyl-5,7-dimethyl-octanoic acid. Representative diastereomers of the latter compounds are (3R,5R)- or (3*S*,5*S*)-3-aminomethyl-5-methyl-heptanoic acid, (3R,5R) or (3S,5S)-3-aminomethyl-5-methyl-octanoic acid, (3R,5R) or (3*S*,5*S*)-3-aminomethyl-5-methyl-nonanoic acid, and (3*R*,5*R*) or (3*S*,5*S*)-3-aminomethyl-5,7-dimethyl-octanoic acid; representative opposite enantiomers are (3*R*,5*S*)-3-aminomethyl-5-methyl-heptanoic acid, (3*R*,5*S*)-3-aminomethyl-5-methyl-octanoic acid, (3*R*,5*S*)-3-aminomethyl-5-methyl-nonanoic acid, and (3*R*,5*S*)-3-aminomethyl-5,7-dimethyl-octanoic acid.

Scheme I shows two methods for preparing compounds of Formula 1. The methods include reacting a chiral alcohol (Formula 2) with an activating agent (Formula 3). The resulting activated alcohol (Formula 4) is reacted with a 2-cyano succinic acid diester (Formula 5) to provide a 2-alkyl-2-cyano succinic acid diester (Formula 6) having a second

stereogenic center, which is represented by wavy bonds. The ester moiety that is directly attached to the second asymmetric carbon atom (see Formula 6) is subsequently cleaved to give a 3-cyano carboxylic acid ester (Formula 7), which is converted to the desired final product (Formula 1) through contact with either a resolving agent or an enzyme. In the former method, the ester (Formula 7) is hydrolyzed to give a 3-cyano carboxylic acid (Formula 8) or salt. Reduction of the cyano moiety (see Formula 8) gives, upon acidification (if necessary), a γ-amino acid (Formula 9) which is resolved via contact with a resolving agent (e.g., a chiral acid), followed by separation of the desired diastereomeric salt or free amino acid (Formula 1). Alternatively, one diastereomer of the monoester (Formula 7) is diastereoselectively hydrolyzed through contact with an enzyme, which results in a mixture enriched in a 3-cyano carboxylic acid or ester having the requisite stereochemical configuration at C-3 (Formula 10). The ester or acid (Formula 10) is separated from the undesirable diastereomer (Formula 11) and is hydrolyzed (if necessary) to give a pure, or substantially pure, diastereomer of 3-cyano carboxylic acid (Formula 10a), or is alternatively converted to a salt. Reduction of the cyano moiety gives, upon acid workup (if necessary), the compound of Formula 1.

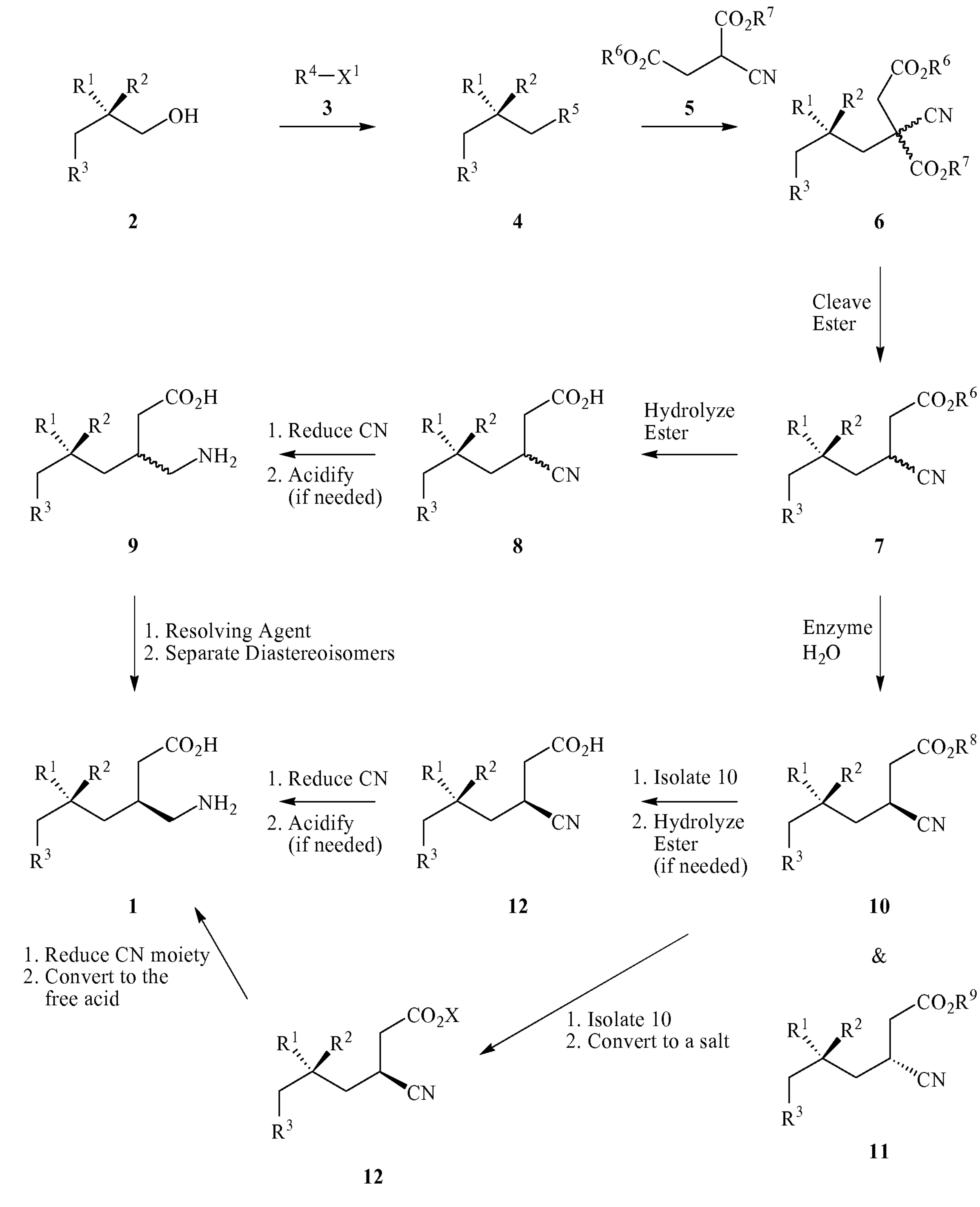


Scheme I

Substituents $R^1$, $R^2$, and $R^3$ in Formula 2, 4, and 6-12 are as defined for Formula 1, above; substituent $R^4$ in Formula 3 is selected from tosyl, mesyl, brosyl, closyl (*p*-chloro-benzenesulfonyl), nosyl, and triflyl; substituent $R^5$ in Formula 4 is a leaving group (e.g., $R^4O$-); and substituent $X^1$ in Formula 3 is halogeno (e.g., Cl) or $R^4O$-. Substituents $R^6$ and $R^7$ in Formula 5-7 are each independently selected from $C_{1-6}$ alkyl, $C_{2-6}$ alkenyl, $C_{2-6}$ alkynyl, $C_{3-7}$ cycloalkyl, $C_{3-7}$ cycloalkenyl, halo-$C_{1-6}$ alkyl, halo-$C_{2-6}$ alkenyl, halo-$C_{2-6}$ alkynyl, aryl-$C_{1-6}$ alkyl, aryl-$C_{2-6}$ alkenyl, and aryl-$C_{2-6}$ alkynyl. Substituents $R^8$ and $R^9$ in Formula 10 and 11 are each independently selected from hydrogen, $C_{1-6}$ alkyl, $C_{2-6}$ alkenyl, $C_{2-6}$ alkynyl, $C_{3-7}$ cycloalkyl, $C_{3-7}$ cycloalkenyl, halo-$C_{1-6}$ alkyl, halo-$C_{2-6}$ alkenyl, halo-$C_{2-6}$ alkynyl, aryl-$C_{1-6}$

alkyl, aryl-$C_{2-6}$ alkenyl, and aryl-$C_{2-6}$ alkynyl. Each of the aforementioned aryl moieties may be optionally substituted with from one to three substituents independently selected from $C_{1-3}$ alkyl, $C_{1-3}$ alkoxy, amino, $C_{1-3}$ alkylamino, and halogeno.

X is a suitable counterion; preferably an alkali metal; more preferably sodium.

The chiral alcohol (Formula 2) shown in Scheme I has a stereogenic center at C-2, as denoted by wedge bonds, and includes substituents $R^1$, $R^2$, and $R^3$, which are as defined above. Compounds of Formula 2 include those in which $R^1$ and $R^2$ are each independently hydrogen or methyl, provided that $R^1$ and $R^2$ are not both hydrogen, and those in which $R^3$ is $C_{1-6}$ alkyl, including methyl, ethyl, *n*-propyl or *i*-propyl. Representative compounds of Formula 2 also include those in which $R^1$ is hydrogen, $R^2$ is methyl, and $R^3$ is methyl, ethyl, *n*-propyl, or *i*-propyl, i.e., (*R*)-2-methyl-butan-1-ol, (R)-2-methyl-pentan-1-ol, (*R*)-2-methyl-hexan-1-ol, or (*R*)-2,4-dimethyl-pentan-1-ol. Representative opposite enantiomers of the latter compounds are (*S*)-2-methyl-butan-1-ol, (*S*)-2-methyl-pentan-1-ol, (S)-2-methyl-hexan-1-ol, and (*S*)-2,4-dimethyl-pentan-1-ol.

As shown in Scheme I, the hydroxy moiety of the chiral alcohol (Formula 2) is activated via reaction with a compound of Formula 3. The reaction is typically carried out with excess (e.g., about 1.05 eq to about 1.1 eq) activating agent (Formula 3) at a temperature of about -25°C to about room temperature. Useful activating agents include sulfonylating agents, such as TsCl, MsCl, BsCl, NsCl, TfCl, and the like, and their corresponding anhydrides (e.g., *p*-toluenesulfonic acid anhydride). Thus, for example, compounds of Formula 2 may be reacted with TsCl in the presence of pyridine and an aprotic solvent, such as EtOAc, $MeCl_2$, ACN, THF, and the like, to give (*R*)-toluene-4-sulfonic acid 2-methyl-butyl ester, (*R*)-toluene-4-sulfonic acid 2-methyl-pentyl ester, (*R*)-toluene-4-sulfonic acid 2-methyl-hexyl ester, and (*R*)-toluene-4-sulfonic acid 2,4-dimethyl-pentyl ester. Likewise, compounds of Formula 2 may be reacted with MsCl in the presence of an aprotic solvent, such as MTBE, toluene, or $MeCl_2$, and a weak base, such as $Et_3N$, to give (R)-methanesulfonic acid 2-methyl-butyl ester, (R)-methanesulfonic acid 2-methyl-pentyl ester, (R)-methanesulfonic acid 2-methyl-hexyl ester, and (R)-methanesulfonic acid 2,4-dimethyl-pentyl ester.

Upon activation of the hydroxy moiety, the resulting intermediate (Formula 4) is reacted with a 2-cyano succinic acid diester (Formula 5) in the presence of a base and one or more solvents to give a 2-alkyl-2-cyano succinic acid diester (Formula 6). Representative compounds of Formula 5 include 2-cyano-succinic acid diethyl ester. Likewise, representative compounds of Formula 6 include (2'R)-2-cyano-2-(2'-methyl-butyl)-succinic acid diethyl ester, (2'*R*)-2-cyano-2-(2'-methyl-pentyl)-succinic acid diethyl ester, (2'*R*)-2-cyano-2-(2'-methyl-hexyl)-succinic acid diethyl ester, and (2'*R*)-2-cyano-2-(2',4'-dimethyl-pentyl)-succinic acid diethyl ester.

The alkylation may be carried out at temperatures that range from about room temperature to reflux, from about 70°C to 110°C, or from about 90°C to about 100°C, using stoichiometric or excess amounts (e.g., about 1 eq to about 1.5 eq) of the base and the diester (Formula 5). Representative bases include Group 1 metal carbonates (e.g., $Cs_2CO_3$ and $K_2CO_3$), phosphates (e.g., $K_3PO_4$), and alkoxides (e.g., 21 % NaOEt in EtOH), as well as hindered, non-nucleophilic bases, such as $Et_3N$, t-BuOK, DBN, DBU, and the like. The reaction mixture may comprise a single organic phase or may comprise an aqueous phase, an organic phase, and a phase-transfer catalyst (e.g., a tetraalkylammonium salt such as $Bu_4N^+Br^-$). Representative organic solvents include polar protic solvents, such as MeOH, EtOH, *i*-PrOH, and other alcohols; polar aprotic solvents, such as EtOAc, i-PrOAc, THF, $MeCl_2$, and ACN; and non-polar aromatic and aliphatic solvents, such as toluene, heptane, and the like.

Following alkylation, the ester moiety that is directly attached to the second asymmetric carbon atom (see Formula 6) is cleaved to give a 3-cyano carboxylic acid ester (Formula 7), such as (5*R*)-3-cyano-5-methyl-heptanoic acid ethyl ester, (5*R*)-3-cyano-5-methyl-octanoic acid ethyl ester, (5*R*)-3-cyano-5-methyl-nonanoic acid ethyl ester, and (5*R*)-3-cyano-5,7-dimethyl-octanoic acid ethyl ester. The ester may be removed by reacting the diester (Formula 6) with a chloride salt (e.g., LiCl, NaCl, etc.) in a polar aprotic solvent, such as aqueous DMSO, NMP, and the like, at a temperature of about 135°C or greater (i.e., Krapcho conditions). Higher temperatures (e.g., 150°C, 160°C, or higher) or the use of a phase transfer catalyst (e.g., $Bu_4N^+Br^-$) may be used to reduce the reaction times to 24 hours or less. Typically, the reaction employs excess chloride salt (e.g., from about 1.1 eq to about 4 eq or from about 1.5 eq to about 3.5 eq).

As shown in Scheme I and as noted above, the 3-cyano carboxylic acid ester (Formula 7) may be converted to the desired product (Formula 1) through contact with a resolving agent. In this method, the ester (Formula 7) is hydrolyzed via contact with an aqueous acid or base to give a 3-cyano carboxylic acid (Formula 8) or salt. For example, the compound of Formula 7 may be treated with HCl, $H_2SO_4$, and the like, and with excess $H_2O$ to give the carboxylic acid of Formula 8. Alternatively, the compound of Formula 7 may be treated with an aqueous inorganic base, such as LiOH, KOH, NaOH, CsOH, $Na_2CO_3$, $K_2CO_3$, $Cs_2CO_3$, and the like, in an optional polar solvent (e.g., THF, MeOH, EtOH, acetone, ACN, etc.) to give a base addition salt, which may be treated with an acid to generate the 3-cyano carboxylic acid (Formula 8). Representative compounds of Formula 8 include (5R)-3-cyano-5-methyl-heptanoic acid, (5*R*)-3-cyano-5-methyl-octanoic acid, (5*R*)-3-cyano-5-methyl-nonanoic acid, and (5*R*)-3-cyano-5,7-dimethyl-octanoic acid, and their salts.

The cyano moiety of the carboxylic acid (Formula 8), or of its corresponding salt, is subsequently reduced to give, upon acid workup if necessary, a γ-amino acid (Formula 9). The penultimate free acid may be obtained by treating

a salt of the γ-amino acid with a weak acid, such as aq HOAc. Representative compounds of Formula 9 include (5*R*)-3-aminomethyl-5-methyl-heptanoic acid, (5R)-3-aminomethyl-5-methyl-octanoic acid, (5*R*)-3-aminomethyl-5-methyl-nonanoic acid, and (5R)-3-aminomethyl-5,7-dimethyl-octanoic acid, and their salts.

The cyano moiety may be reduced via reaction with $H_2$ in the presence of a catalyst or through reaction with a reducing agent, such as $LiAlH_4$, $BH_3$-$Me_2S$, and the like. In addition to Raney nickel and other sponge metal catalysts, potentially useful catalysts include heterogeneous catalysts containing from about 0.1 % to about 20%, or from about 1% to about 5%, by weight, of transition metals such as Ni, Pd, Pt, Rh, Re, Ru, and Ir, including oxides and combinations thereof, which are typically supported on various materials, including $Al_2O_3$, C, $CaCO_3$ $SrCO_3$ $BaSO_4$, MgO, $SiO_2$ $TiO_2$ $ZrO_2$ and the like. Many of these metals, including Pd, may be doped with an amine, sulfide, or a second metal, such as Pb, Cu, or Zn. Exemplary catalysts thus include palladium catalysts such as Pd/C, Pd/$SrCO_3$ Pd/$Al_2O_3$, Pd/MgO, Pd/$CaCO_3$ Pd/$BaSO_4$, PdO, Pd black, $PdCl_2$, and the like, containing from about 1% to about 5% Pd, based on weight. Other catalysts include Rh/C, Ru/C, Re/C, $PtO_2$ Rh/C, $RuO_2$ and the like.

The catalytic reduction of the cyano moiety is typically carried out in the presence of one or more polar solvents, including without limitation, water, alcohols, ethers, esters and acids, such as MeOH, EtOH, IPA, THF, EtOAc, and HOAc. The reaction may be carried out at temperatures ranging from about 5°C to about 100°C, though reactions at room temperature are common. Generally, the substrate-to-catalyst ratio may range from about 1:1 to about 1000:1, based on weight, and $H_2$ pressure may range from about atmospheric pressure, 0 psig, to about 1500 psig. More typically, the substrate-to-catalyst ratios range from about 4:1 to about 20:1, and $H_2$ pressures range from about 25 psig to about 150 psig.

As shown in Scheme I, the penultimate γ-amino acid (Formula 9) is resolved to give the desired stereoisomer (Formula 1). The amino acid (Formula 9) may be resolved through contact with a resolving agent, such as an enantiomerically pure or substantially pure acid or base (e.g., *S*-mandelic acid, S-tartaric acid, and the like) to yield a pair of diastereoisomers (e.g., salts having different solubilities), which are separated via, e.g., recrystallization or chromatography. The γ-amino acid having the desired stereochemical configuration (Formula 1) is subsequently regenerated from the appropriate diastereomer via, e.g., contact with a base or acid or through solvent splitting (e.g., contact with EtOH, THF, and the like). The desired stereoisomer may be further enriched through multiple recrystallizations in a suitable solvent.

Besides using a resolving agent as described above, the 3-cyano carboxylic acid ester (Formula 7) may be converted to the desired product (Formula 1) through contact with an enzyme. As shown in Scheme I and as discussed above, one diastereomer of the monoester (Formula 7) is diastereoselectively hydrolyzed through contact with an enzyme, which results in a mixture containing a 3-cyano carboxylic acid (or ester) having the requisite stereochemical configuration at C-3 (Formula 10) and a 3-cyano carboxylic ester (or acid) having the opposite (undesired) stereochemical configuration at C-3 (Formula 11). Representative compounds of Formula 10 include (3S,5R)-3-cyano-5-methyl-heptanoic acid, (3*S*,5*R*)-3-cyano-5-methyl-octanoic acid, (3*S*,5*R*)-3-cyano-5-methyl-nonanoic acid, and (3*S*,5*R*)-3-cyano-5,7-dimethyl-octanoic acid, and salts thereof, as well as $C_{1-6}$ alkyl esters of the aforementioned compounds, including (3*S*,5*R*)-3-cyano-5-methyl-heptanoic acid ethyl ester, (3*S*,5*R*)-3-cyano-5-methyl-octanoic acid ethyl ester, (3*S*,5*R*)-3-cyano-5-methyl-nonanoic acid ethyl ester, and (3*S*,5*R*)-3-cyano-5,7-dimethyl-octanoic acid ethyl ester. Exemplary compounds of Formula 11 include (3R,5R)-3-cyano-5-methyl-heptanoic acid, (3*R*,5*R*)-3-cyano-5-methyl-octanoic acid, (3*R*,5*R*)-3-cyano-5-methyl-nonanoic acid, and (3*R*,5*R*)-3-cyano-5,7-dimethyl-octanoic acid, and salts thereof, as well as $C_{1-6}$ alkyl esters of the aforementioned compounds, including (3R,5R)-3-cyano-5-methyl-heptanoic acid ethyl ester, (3*R*,5*R*)-3-cyano-5-methyl-octanoic acid ethyl ester, (3*R*,5*R*)-3-cyano-5-methyl-nonanoic acid ethyl ester, and (3R,5R)-3-cyano-5,7-dimethyl-octanoic acid ethyl ester.

The choice of enzyme (biocatalyst) used to resolve the desired diastereomer (Formula 10) depends on the structures of the substrate (Formula 7) and the bioconversion product (Formula 10 or Formula 11). The substrate (Formula 7) comprises two diastereoisomers (Formula 13 and Formula 14) having opposite stereochemical configuration at C-3,

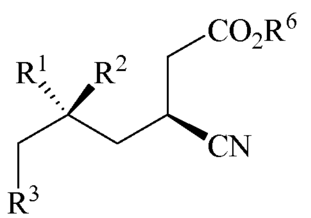

**13**

and

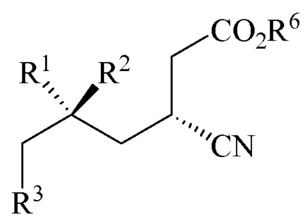

**14**

.

In Formula 13 and Formula 14, substituents $R^1$, $R^2$, and $R^6$ are as defined for Formula 1 and Formula 5, above. The enzyme stereoselectively hydrolyzes one of the two diastereoisomers (Formula 13 or Formula 14). Thus, the enzyme

may be any protein that, while having little or no effect on the compound of Formula 13, catalyzes the hydrolysis of the compound of Formula 14 to give a 3-cyano carboxylic acid (or salt) of Formula 11. Alternatively, the enzyme may be any protein that, while having little or no effect on the compound of Formula 14, catalyzes the hydrolysis of the compound of Formula 13 to give a 3-cyano carboxylic acid (or salt) of Formula 10. Useful enzymes for diastereoselectively hydrolyzing the compounds of Formula 13 or Formula 14 to compounds of Formula 10 or Formula 11, respectively, may thus include hydrolases, including lipases, certain proteases, and other stereoselective esterases. Such enzymes may be obtained from a variety of natural sources, including animal organs and microorganisms. See, e.g., Table 2 for a non-limiting list of commercially available hydrolases.

Table 2. Commercially Available Hydrolases

| Enzyme | Trade name |
|---|---|
| Porcine Pancreatic Lipase | Altus 03 |
| CAL-A, lyophilized | Altus 11 |
| *Candida lipolytica* Lipase | Altus 12 |
| CAL-B, lyophilized | Altus 13 |
| *Geotrichum candidum* Lipase | Altus 28 |
| *Pseudomonas aroginosa* Lipase | Altus 50 |
| *Pseudomonas sp.* Esterase | Amano Cholesterol Esterase 2 |
| *Aspergillus niger* Lipase | Amano Lipase AS |
| *Burkholderia cepacia* Lipase | Amano Lipase AH |
| *Pseudomonas fluorescens* Lipase | Amano Lipase AK 20 |
| *Candida rugosa* Lipase | Amano Lipase AYS |
| *Rhizopus delemar* Lipase | Amano Lipase D |
| *Rhizopus oryzae* Lipase | Amano Lipase F-AP 15 |
| *Penicillium camembertii* Lipase | Amano Lipase G 50 |
| *Mucor javanicus* Lipase | Amano Lipase M 10 |
| *Burkholderia cepacia* Lipase | Amano Lipase PS |
| *Burkholderia cepacia* Lipase | Amano Lipase PS-SD |
| *Burkholderia cepacia* Lipase | Amano Lipase PS-C I |
| *Burkholderia cepacia* Lipase | Amano Lipase PS-C II |
| *Burkholderia cepacia* Lipase | Amano Lipase PS-D I |
| *Penicillium roqueforti* Lipase | Amano Lipase R |
| *Burkholderia cepacia* Lipase | Amano Lipase S |
| *Aspergillus sp.* Protease | BioCatalytics 101 |
| *Pseudomonas sp.* Lipase | BioCatalytics 103 |
| Fungal Lipase | BioCatalytics 105 |
| Microbial, lyophilized Lipase | BioCatalytics 108 |
| CAL-B, lyophilized | BioCatalytics 110 |
| *Candida sp.,* lyophilized | BioCatalytics 111 |
| CAL-A, lyophilized | BioCatalytics 112 |
| *Thermomyces sp.* Lipase | BioCatalytics 115 |
| *Alcaligines sp.,* lyophilized Lipase | BioCatalytics 117 |
| *Chromobacterium viscosum* Lipase | Altus 26 |
| CAL-B, L2 Sol | Chriazyme L2 Sol |
| *Candida cylindracea* Lipase | Fluka 62302 |
| *Candida utilis* Lipase | Fluka 6 |
| *Rhizopus niveus* Lipase | Sigma L8 |
| Porcine Pancreatic Lipase | Sigma L12 |
| *Pseudomonas sp.* Lipoprotein Lipase | Sigma L13 |
| *Thermomuces lanuginosus* Lipase | Sigma L9 Lipolase |
| *Thermomuces lanuginosus* Lipase | Sigma L10 Novo871 |
| *Rhizomucor miehei* Lipase | Sigma L6 Palatase |

(continued)

| Enzyme | Trade name |
|---|---|
| *Pseudomonas* species Lipase | Sigma L14 Type XIII |
| Wheat Germ Lipase | Sigma L11 |
| *Rhizopus arrhizus* Lipase | Sigma L7 Type XI |
| Pancreatic Lipase 250 | Valley Research V1 |
| Trypsin Protease | Altus 33 |
| Chymopapain Protease | Altus 38 |
| Bromelain Protease | Altus 40 |
| *Aspergillus niger* Protease | Altus 41 |
| *Aspergillus oryzae* Protease | Altus 42 |
| *Penicillium sp.* Protease | Altus 43 |
| *Aspergillus sp.* Protease | Altus 45 |
| Renin Calf Stomach Protease | Sigma P24 |
| Subtilisin Carlsberg Protease | Altus 10 |
| *Bacillus lentus* Protease | Altus 53 |
| Fungal protease | Genencor Fungal Protease 500,000 |
| Fungal Protease | Genencor Fungal Protease Concentrate |
| Bacterial Protease | Genencor Protex 6L |
| Protease | Genencor Protease 899 |
| Bacterial protease | Genencor Multifect P3000 |
| Bacterial protease | Genencor Primatan |
| Bacterial protease | Genencor Purafect (4000L) |
| Bacterial protease | Genencor Multifect Neutral |
| *Aspergillus niger* Protease | Amano Acid Protease A |
| *Rhizopus niveus* Protease | Amano Acid Protease II |
| *Rhizopus niveus* Protease | Amano Newlase F |
| *Rhizopus oryzae* Protease | Amano Peptidase R |
| *Bacillus subtilis* Protease | Amano Proleather FGF |
| *Aspergillus oryzae* Protease | Amano Protease A |
| *Aspergillus oryzae* Protease | Amano Protease M |
| *Bacillus subtilis* Protease | Amano Protease N |
| *Aspergillus melleus* Protease | Amano Protease P 10 |
| *Bacillus stearothermophilus* Protease | Amano Protease SG |
| Pig Liver Esterase, lyophilized | BioCat Chirazyme E1 |
| Pig Liver Esterase, lyophilized | BioCat Chirazyme E2 |
| *Streptomyces sp.* Proteases | BioCatalytics 118 |
| Tritirachium album Protease | Fluka P6 Proteinase K |
| Bovine Pancreas Protease | Sigma P18 alpha chymotrypsin I |
| *Streptomyces griseus* Protease | Sigma P16 Bacterial |
| Bovine Pancreas Protease | Sigma P21 Beta chymotrypsin |
| *Clostridium histolyticum* Protease | Sigma P13 Clostripain |
| Bovine Intestine Protease | Sigma P17 Enteropeptidase |
| Porcine Intestine Protease | Sigma P25 Enteropeptidase |
| *Bacillus sp.* Protease | Sigma P8 Esperase |
| *Aspergillus oryzae* Protease | Sigma P1 Flavourzyme |
| *Bacillus amyloliquefaciens* Protease | Sigma P5 Neutrase |
| *Carica papaya* Protease | Sigma P12 Papain |
| *Bacillus thermoproteolyticus* rokko | Sigma P10 Protease |
| *Pyrococcus furiosis* Protease | Sigma P14 Protease S |
| *Bacillus sp.* Protease | Sigma P9 Savinase |
| Bovine Pancreas Protease | Sigma P19 Type 1 (crude) |

(continued)

| Enzyme | Trade name |
|---|---|
| *Bacillus polymyxa* Protease | Sigma P7 Type IX |
| *Bacillus licheniformis* Protease | Sigma P6 Type VIII |
| *Aspergillus saitoi* Protease | Sigma P3 Type XIII |
| *Aspergillus sojae* Protease | Sigma P4 Type XIX |
| *Aspergillus oryzae* Protease | Sigma P2 Type XXIII |
| Bacterial Protease | Sigma P11 Type XXIV |
| *Rhizopus sp.* Newlase | Sigma15 Newlase |
| *Aspergillus oryzae* Protease | Validase FP Concentrate |
| Pineapple [Ananas comosus & Ananas bracteatus (L)] | Bromelian Concentrate |
| *Aspergillus sp.* Acylase | Amano Am 1 |
| Porcine kidney Acylase | Sigma A-S2 Acylase I |
| Penicillin G Acylase | Altus 06 |
| Esterase from *Mucor meihei* | Fluka E5 |
| *Candida rugosa* Esterase | Altus 31 |
| Porcine Pancreatic Elastase | Altus 35 |
| Cholinesterase, acetyl | Sigma ES8 |
| Cholesterol Esterase | BioCatalytics E3 |
| PLE - Ammonium Sulfate | BioCatalytics 123 |
| Rabbit Liver Esterase | Sigma ES2 |
| Cholesterol Esterase *Pseudomonas sp.* | Sigma ES4 |

As shown in the Example section, useful enzymes for the diastereoselective conversion of the cyano-substituted ester (Formula 13 or Formula 14) to the carboxylic acid (or salt) of Formula 10 or Formula 11 include lipases. Particularly useful lipases for conversion of the cyano-substituted ester of Formula 14 to a carboxylic acid (or salt) of Formula 11 include enzymes derived from the microorganism *Burkholderia cepacia* (formerly *Pseudomonas cepacia),* such as those available from Amano Enzyme Inc. under the trade names PS, PS-SD, PS-C I, PS-C II, PS-D I, and S. These enzymes are available as free-flowing powder (PS) or as lyophilized powder (S) or may be immobilized on ceramic particles (PS-C I and PS-C II) or diatomaceous earth (PS-D I). They have lypolytic activity that may range from about 30 KLu/g (PS) to about 2,200 KLu/g (S). Lipase PS-SD from Amano Enzyme Inc. is a preferred enzyme for use in the process of the invention.

Particularly useful lipases for the conversion of the cyano-substituted ester of Formula 13 to a carboxylic acid (or salt) of Formula 10 include enzymes derived from the microorganism Thermomyces *lanuginosus,* such as those available from Novo-Nordisk A/S under the trade name LIPOLASE®. LIPOLASE® enzymes are obtained by submerged fermentation of an *Aspergillus oryzae* microorganism genetically modified with DNA from *Thermomyces lanuginosus* DSM 4109 that encodes the amino acid sequence of the lipase. LIPOLASE® 100L and LIPOLASE® 100T are available as a liquid solution and a granular solid, respectively, each having a nominal activity of 100 kLU/g. Other forms of LIPOLASE® include LIPOLASE® 50L, which has half the activity of LIPOLASE® 100L, and LIPOZYME® 100L, which has the same activity of LIPOLASE® 100L, but is food grade.

Various screening techniques may be used to identify suitable enzymes. For example, large numbers of commercially available enzymes may be screened using high throughput screening techniques described in the Example section below. Other enzymes (or microbial sources of enzymes) may be screened using enrichment isolation techniques. Such techniques typically involve the use of carbon-limited or nitrogen-limited media supplemented with an enrichment substrate, which may be the substrate (Formula 7) or a structurally similar compound. Potentially useful microorganisms are selected for further investigation based on their ability to grow in media containing the enrichment substrate. These microorganisms are subsequently evaluated for their ability to stereoselectively catalyze ester hydrolysis by contacting suspensions of the microbial cells with the unresolved substrate and testing for the presence of the desired diastereoisomer (Formula 10) using analytical methods such as chiral HPLC, gas-liquid chromatography, LC/MS, and the like.

Once a microorganism having the requisite hydrolytic activity has been isolated, enzyme engineering may be employed to improve the properties of the enzyme it produces. For example, and without limitation, enzyme engineering may be used to increase the yield and the diastereoselectivity of the ester hydrolysis, to broaden the temperature and pH operating ranges of the enzyme, and to improve the enzyme’s tolerance to organic solvents. Useful enzyme engineering techniques include rational design methods, such as site-directed mutagenesis, and in vitro-directed evolution techniques that utilize successive rounds of random mutagenesis, gene expression, and high throughput screening to

optimize desired properties. See, e.g., K. M. Koeller & C.-H. Wong, "Enzymes for chemical synthesis," Nature 409: 232-240 (11 Jan. 2001), and references cited therein, the complete disclosures of which are herein incorporated by reference.

The enzyme may be in the form of whole microbial cells, permeabilized microbial cells, extracts of microbial cells, partially purified enzymes, purified enzymes, and the like. The enzyme may comprise a dispersion of particles having an average particle size, based on volume, of less than about 0.1 mm (fine dispersion) or of about 0.1 mm or greater (coarse dispersion). Coarse enzyme dispersions offer potential processing advantages over fine dispersions. For example, coarse enzyme particles may be used repeatedly in batch processes, or in semi-continuous or continuous processes, and may usually be separated (e.g., by filtration) from other components of the bioconversion more easily than fine dispersions of enzymes.

Useful coarse enzyme dispersions include cross-linked enzyme crystals (CLECs) and cross-linked enzyme aggregates (CLEAs), which are comprised primarily of the enzyme. Other coarse dispersions may include enzymes immobilized on or within an insoluble support. Useful solid supports include polymer matrices comprised of calcium alginate, polyacrylamide, EUPERGIT®, and other polymeric materials, as well as inorganic matrices, such as CELITE®. For a general description of CLECs and other enzyme immobilization techniques, see U.S. Patent No. 5,618,710 to M. A. Navia & N. L. St. Clair. For a general discussion of CLEAs, including their preparation and use, see U.S. Patent Application No. 2003/0149172 to L. Cao & J. Elzinga et al. See also A. M. Anderson, Biocat. Biotransform, 16:181 (1998) and P. López-Serrano et al., Biotechnol. Lett. 24:1379-83 (2002) for a discussion of the application of CLEC and CLEA technology to a lipase. The complete disclosures of the abovementioned references are herein incorporated by reference for all purposes.

The reaction mixture may comprise a single phase or may comprise multiple phases (e.g., a two- or a three-phase system). Thus, for example, the diastereoselective hydrolysis shown in Scheme I may take place in a single aqueous phase, which contains the enzyme, the substrate (Formula 7), the desired diastereomer (Formula 10), and the undesired diastereomer (Formula 11). Alternatively, the reaction mixture may comprise a multi-phase system that includes an aqueous phase in contact with a solid phase (e.g., enzyme or product), an aqueous phase in contact with an organic phase, or an aqueous phase in contact with an organic phase and a solid phase. For example, the diastereoselective hydrolysis may be carried out in a two-phase system comprised of a solid phase, which contains the enzyme, and an aqueous phase, which contains the substrate (Formula 7), the desired diastereomer (Formula 10), and the undesired diastereomer (Formula 11).

Alternatively, the diastereoselective hydrolysis may be carried out in a three-phase system comprised of a solid phase, which contains the enzyme, an organic phase that contains the substrate (Formula 7), and an aqueous phase that initially contains a small fraction of the substrate. In some cases the desired diastereomer (Formula 10) is a carboxylic acid which has a lower pKa than the unreacted ester (Formula 14). Because the carboxylic acid exhibits greater aqueous solubility, the organic phase becomes enriched in the unreacted ester (Formula 14) while the aqueous phase becomes enriched in the desired carboxylic acid (or salt). In other cases the undesired diastereomer (Formula 11) is a carboxylic acid, so the organic phase becomes enriched in the desired unreacted ester (Formula 13) while the aqueous phase becomes enriched in the undesired carboxylic acid (or salt). Preferably, the undesired diastereomer (Formula 11) is selectively hydrolysed to the carboxylic acid, which is soluble in the aqueous phase, while the desired diastereomer (ester of Formula 10) is unreacted and remains in the organic phase.

The amounts of the substrate (Formula 7) and the biocatalyst used in the stereoselective hydrolysis will depend on, among other things, the properties of the particular cyano-substituted ester and the enzyme. Generally, however, the reaction may employ a substrate having an initial concentration of about 0.1 M to about 5.0 M, and in many cases, having an initial concentration of about 0.1 M to about 1.0 M. Additionally, the reaction may generally employ an enzyme loading of about 1 % to about 20%, and in many cases, may employ an enzyme loading of about 5% to about 15% (w/w).

The stereoselective hydrolysis may be carried out over a range of temperature and pH. For example, the reaction may be carried out at temperatures of about 10°C to about 60°C, but is typically carried out at temperatures of about RT to about 45°C. Such temperatures generally permit substantially full conversion (e.g., about 42 % to about 50 %) of the substrate (Formula 7) with a de (3*S*,5*R* diastereomer) of about 80% or greater (e.g., 98%) in a reasonable amount of time (e.g., about 1 h to about 48 h or about 1 h to about 24 h) without deactivating the enzyme. Additionally, the stereoselective hydrolysis may be carried out at a pH of about 5 to a pH of about 11, more typically at a pH of about 6 to a pH of about 9, and often at a pH of about 6.5 to a pH of about 7.5. In the absence of pH control, the reaction mixture pH will decrease as the hydrolysis of the substrate (Formula 7) proceeds because of the formation of a carboxylic acid (Formula 10 or Formula 11). To compensate for this change, the hydrolysis reaction may be run with internal pH control (i.e., in the presence of a suitable buffer) or may be run with external pH control through the addition of a base. Suitable buffers include sodium hydrogen carbonate, potassium phosphate, sodium phosphate, sodium acetate, ammonium acetate, calcium acetate, BES, BICINE, HEPES, MES, MOPS, PIPES, TAPS, TES, TRICINE, Tris, TRIZMA®, or other buffers having a pKa of about 6 to a pKa of about 9. The buffer concentration generally ranges from about 5 mM to about 1 mM, and typically ranges from about 50 mM to about 200 mM. Suitable bases include aqueous solutions

comprised of KOH, NaOH, $NH_4OH$, etc., having concentrations ranging from about 0.5 M to about 15 M, or more typically, ranging from about 5 M to about 10 M. Other inorganic additives such as calcium acetate may also be used.

Following or during the enzymatic conversion of the substrate (Formula 7), the desired diastereomer (Formula 10) is isolated from the product mixture using standard techniques. For example, in the case of a single (aqueous) phase batch reaction, the product mixture may be extracted one or more times with an organic solvent, such as hexane, heptane, $MeCl_2$, toluene, MTBE, THF, etc., which separates the acid (ester) having the requisite stereochemical configuration at C-3 (Formula 10) from the undesirable ester (acid) (Formula 11) in the aqueous (organic) and organic (aqueous) phases, respectively. Alternatively, in the case of a multi-phase reaction employing aqueous and organic phases enriched in the acid or ester, the two diastereomers (Formula 10 and Formula 11) may be separated batch-wise following reaction, or may be separated semi-continuously or continuously during the stereoselective hydrolysis.

As shown in Scheme I, once the desired diastereomer (Formula 10) is isolated from the product mixture, it is optionally hydrolyzed using conditions and reagents associated with the ester hydrolysis of the compound of Formula 7, above. The cyano moiety of the resulting carboxylic acid (Formula 10a), or its corresponding salt, is subsequently reduced to give, upon acid workup if necessary, the desired γ-amino acid (Formula 1). The reduction may employ the same conditions and reagents described above for reduction of the cyano moiety of the compound of Formula 8 and may be undertaken without isolating the cyano acid of Formula 10a. Representative compounds of Formula 10a include (3*S*,5*R*)-3-cyano-5-methyl-heptanoic acid, (3*S*,5*R*)-3-cyano-5-methyl-octanoic acid, (3*S*,5*R*)-3-cyano-5-methyl-nonanoic acid, and (3*S*,5*R*)-3-cyano-5,7-dimethyl-octanoic acid, and their salts.

As shown in Scheme 1, the desired diastereomer (Formula 10) may be converted to a suitable salt, preferably an alkali metal salt. The cyano moiety of the resulting salt is subsequently reduced to give the salt of the desired γ-amino acid (Formula 1). The reduction may employ the same conditions and reagents described above for reduction of the cyano moiety of the compound of Formula 8. The resulting salt of the compound of Formula 1 may then be further converted to the free acid, or to a pharmaceutically acceptable salt, solvate or hydrate thereof. A preferred salt of a compound of Formula 10a is the sodium salt of (3*S*,5*R*)-3-cyano-5-methyl-octanoic acid.

The chiral alcohol (Formula 2) shown in Scheme I may be prepared using various methods. For example, the chiral alcohol may be prepared by stereoselective enzyme-mediated hydrolysis of a racemic ester using conditions and reagents described above in connection with the enzymatic resolution of the compound of Formula 7. For example, *n*-decanoic acid 2-methyl-pentyl ester may be hydrolyzed in the presence of a hydrolase (e.g., lipase) and water to give a pure (or substantially pure) chiral alcohol, (*R*)-2-methyl-pentan-1-ol, which may be separated from the non-chiral acid and the unreacted chiral ester (*n*-decanoic acid and (*S*)-pentanoic acid 2-methyl-pentyl ester) by fractional distillation. The ester substrate may be prepared from the corresponding racemic alcohol (e.g., 2-methyl-pentan-1-ol) and acid chloride (e.g., *n*-decanoic acid chloride) or anhydride using methods known in the art.

Alternatively, the chiral alcohol (Formula 2) may be prepared by asymmetric synthesis of an appropriately substituted 2-alkenoic acid. For example, 2-methyl-pent-2-enoic acid (or its salt) may be hydrogenated in the presence of a chiral catalyst to give (*R*)-2-methyl-pentaonic acid or a salt thereof, which may be reduced directly with LAH to give (*R*)-2-methyl-pentan-1-ol or converted to the mixed anhydride or acid chloride and then reduced with $NaBH_4$ to give the chiral alcohol. Potentially useful chiral catalysts include cyclic or acyclic, chiral phosphine ligands (e.g., monophosphines, bisphosphines, bisphospholanes, etc.) or phosphinite ligands bound to transition metals, such as ruthenium, rhodium, iridium or palladium. Ru-, Rh-, Ir-or Pd-phosphine, phosphinite or phosphino oxazoline complexes are optically active because they possess a chiral phosphorus atom or a chiral group connected to a phosphorus atom, or because in the case of BINAP and similar atropisomeric ligands, they possess axial chirality.

Exemplary chiral ligands include BisP*; (*R*)-BINAPINE; (S)-Me-ferrocene-Ketalphos, (*R*,*R*)-DIOP; (*R*,*R*)-DIPAMP; (R)-(S)-BPPFA; (S,S)-BPPM; (+)-CAMP; (S,S)-CHIRAPHOS; (R)-PROPHOS; (R,R)-NORPHOS; (*R*)-BINAP; (R)-CYCPHOS; (R,R)-BDPP; (R,R)-DEGUPHOS; (R,R)-Me-DUPHOS; (R,R)-Et-DUPHOS; (*R*,*R*)-*i*-Pr-DUPHOS; (R,R)-Me-BPE; (R,R)-Et-BPE (R)-PNNP; (*R*)-BICHEP; (R,S,R,S)-Me-PENNPHOS; (S,S)-BICP; (*R*,*R*)-Et-FerroTANE; (*R*,*R*)-*t*-butyl-miniPHOS; (*R*)-Tol-BINAP; (R)-MOP; (*R*)-QUINAP; CARBOPHOS; (*R*)-(*S*)-JOSIPHOS; (R)-PHANEPHOS; BIPHEP; (*R*)-Cl-MeO-BIPHEP; (*R*)-MeO-BIPHEP; (*R*)-MonoPhos; BIFUP; (R)-SpirOP; (+)-TMBTP; (+)-tetraMeBITIANP; (R,R,S,S) TANGPhos; (*R*)-$PPh_2$-PhOx-Ph; (S,S) MandyPhos; (R)-eTCFP; (R)-mTCFP; and (R)-CnTunaPHOS, where n is an integer of 1 to 6.

Other chiral ligands include (R)-(-)-1-[(S)-2-(di(3,5-bistrifluoromethylphenyl)phosphino)ferrocenyl]ethyldicyclohexyl-phosphine; (*R*)-(-)-1-[(*S*)-2-(di(3,5-bistrifluoromethylphenyl)phosphino)ferrocen-yl]ethyldi(3,5-dimethylphenyl) phosphine; (*R*)-(-)-1-[(*S*)-2-(dit-butylphosphino)ferro-cenyl]ethyldi(3,5-dimethylphenyl)phosphine; (*R*)-(-)-1-[(*S*)-2-(dicyclohexylphosphi-no)ferrocenyl]ethyldi-*t*-butylphosphine; (*R*)-(-)-1-[(*S*)-2-(dicyclohexylphosphino)fer-rocenyl]ethyldicyclohexylphosphine; (*R*)-(-)-1-[(*S*)-2-(dicyclohexylphosphino)ferro-cenyl]ethyldiphenylphosphine; (*R*)-(-)-1-[(*S*)-2-(di(3,5-dimethyl-4-methoxyphen-yl)phosphino)ferrocenyl]ethyldicyclohexylphosphine; (*R*)-(-)-1-[(*S*)-2-(diphenylphos-phino) ferrocenyl]ethyldi-*t*-butylphosphine; (*R*)-*N*-[2-(*N*,*N*-dimethylamino)ethyl]-*N*-methyl-1-[(*S*)-1',2-bis(diphenylphosphino) ferrocenyl]ethylamine; (*R*)-(+)-2-[2-(diphenylphosphino)phenyl]-4-(1-methylethyl)-4,5-dihydrooxazole; (1-[((*R*,*R*)-2-benzyl-phospholanyl)-phen-2-yl]-(*R**,*R**)-phospholan-2-yl}-phenyl-methane; and {1-[((*R*,*R*)-2-benzyl-phospholа-

nyl)-ethyl]-(*R**,*R**)-phospholan-2-yl}-phenylmethane.

Useful ligands may also include stereoisomers (enantiomers and diastereoisomers) of the chiral ligands described in the preceding paragraphs, which may be obtained by inverting all or some of the stereogenic centers of a given ligand or by inverting the stereogenic axis of an atropoisomeric ligand. Thus, for example, useful chiral ligands may also include (*S*)-Cl-MeO-BIPHEP; (S)-PHANEPHOS; (S,S)-Me-DUPHOS; (S,S)-Et-DUPHOS; (*S*)-BINAP; (S)-Tol-BINAP; (*R*)-(*R*)-JOSIPHOS; (S)-(S)-JOSIPHOS; (S)-eTCFP; (S)-mTCFP and so on.

Many of the chiral catalysts, catalyst precursors, or chiral ligands may be obtained from commercial sources or may be prepared using known methods. A catalyst precursor or pre-catalyst is a compound or set of compounds, which are converted into the chiral catalyst prior to use. Catalyst precursors typically comprise Ru, Rh, Ir or Pd complexed with the phosphine ligand and either a diene (e.g., norboradiene, COD, (2-methylallyl)$_2$, etc.) or a halide (Cl or Br) or a diene and a halide, in the presence of a counterion, X-, such as $OTf^-$, $PF_6^-$, $BF_4^-$, $SbF_6^-$, $ClO_4^-$, etc. Thus, for example, a catalyst precursor comprised of the complex, [(bisphosphine ligand)Rh(COD)]$^+X^-$ may be converted to a chiral catalyst by hydrogenating the diene (COD) in MeOH to yield [(bisphosphine ligand)Rh(MeOH)$_2$]$^+X^-$. MeOH is subsequently displaced by the enamide (Formula 2) or enamine (Formula 4), which undergoes enantioselective hydrogenation to the desired chiral compound (Formula 3). Examples of chiral catalysts or catalyst precursors include (+)-TMBTP-ruthenium (II) chloride acetone complex; (S)-Cl-MeO-BIPHEP-ruthenium(II) chloride $Et_3N$ complex; (S)-BINAP-ruthenium(II) $Br_2$ complex; (S)-tol-BINAP-ruthenium(II) $Br_2$ complex; [((3*R*,4*R*)-3,4-bis(diphenylphosphino)-1-methylpyrrolidine)-rhodium-(1,5-cyclooctadiene)]-tetrafluoroborate complex; [((*R*,*R*,*S*,*S*)-TANGPhos)-rhodium(I)-bis(1,5-cyclooctadiene)]-trifluoromethane sulfonate complex; [(*R*)-BINAPINE-rhodium-(1,5-cyclooctaidene)]-tetrafluoroborate complex; [(*S*)-eTCFP-(1,5-cyclooctadiene)-rhodium(I)]-tetrafluoroborate complex; and [(*S*)-mTCFP-(1,5-cyclooctadiene)-rhodium(I)]-tetrafluroborate complex.

For a given chiral catalyst and hydrogenation substrate, the molar ratio of the substrate and catalyst (s/c) may depend on, among other things, $H_2$ pressure, reaction temperature, and solvent (if any). Usually, the substrate-to-catalyst ratio exceeds about 100:1 or 200:1, and substrate-to-catalyst ratios of about 1000:1 or 2000:1 are common. Although the chiral catalyst may be recycled, higher substrate-to-catalyst ratios are more useful. For example, substrate-to-catalyst ratios of about 1000:1, 10,000:1, and 20,000:1, or greater, would be useful. The asymmetric hydrogenation is typically carried out at about room temperature or above, and under about 10 kPa (0.1 atm) or more of $H_2$. The temperature of the reaction mixture may range from about 20°C to about 80°C, and the $H_2$ pressure may range from about 10 kPa to about 5000 kPa or higher, but more typically, ranges from about 10 kPa to about 100 kPa. The combination of temperature, $H_2$ pressure, and substrate-to-catalyst ratio is generally selected to provide substantially complete conversion (i.e., about 95 wt %) of the substrate (Formula 2 or 4) within about 24 hours. With many of the chiral catalysts, decreasing the $H_2$ pressure increases the enantioselectivity.

A variety of solvents may be used in the asymmetric hydrogenation, including protic solvents, such as water, MeOH, EtOH, and *i*-PrOH. Other useful solvents include aprotic polar solvents, such as THF, ethyl acetate, and acetone. The stereoselective hydrogenation may employ a single solvent or may employ a mixture of solvents, such as THF and MeOH, THF and water, EtOH and water, MeOH and water, and the like.

The compound of Formula 1, or its diastereoisomers, may be further enriched through, e.g., fractional recrystallization or chromatography or by recrystallization in a suitable solvent.

Alternatively, a compound of Formula 10 or 11 may be prepared as illustrated by Scheme 2.

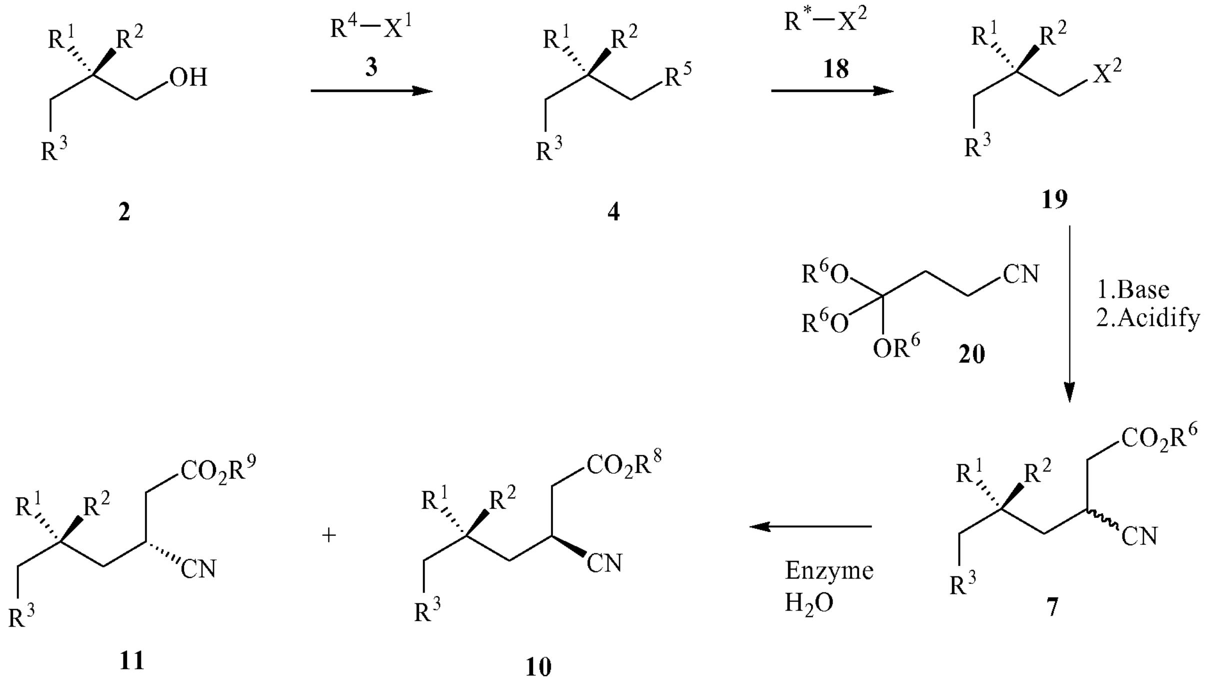


Scheme 2

wherein $R^1$, $R^2$, $R^3$, $R^8$ and $R^9$ are as defined above;
$R^4$ is selected from tosyl, mesyl, brosyl, closyl (*p*-chloro-benzenesulfonyl), nosyl and triflyl, preferably mesyl;
$R^5$ is a suitable leaving group such as $R^4O$-, preferably mesyl-O-;
$R^6$ is $C_{1-6}$ alkyl, preferably methyl;
$X^1$ is halageno, preferably chloro or bromo;
$R^*$-$X^2$ is an alkali metal halide, preferably sodium bromide; and
$X^2$ is halogeno, preferably bromo.

A compound of Formula 4 may be prepared from a compound of Formula 3 by the process described above for Scheme 1.

A compound of Formula 19 may be prepared from a compound of Formula 4 and a compound of Formula 18, by reacting a stoichiometric or excess amount of a compound of Formula 18 with a compound of Formula 4 over from 2 to 6 hours, over a temperature range of from 45°C to 90°C. The reaction mixture may comprises of an aqueous phase, an organic phase and a phase transfer catalyst (eg a tetraalkylammonium salt such as Bu4N+Br-) Representative organic solvents include polar aprotic solvents such as TMBE, THF, EtOAc, iPrOAc and non-polar aromatic solvents such as toluene.

A compound of Formula 7 may be prepared from a compound of Formula 19 and an orthoester compound of Formula 20. The alkylation maybe carried out at temperatures that range from -5°C to 5°C, using a stoichiometric or excess amount (e.g. 1 to 1.5 equivalents) of the compound of Formula 20 over from 2 to 12 hours. Representative bases include KOtBu, LDA , nBuLi and LiHMDS using an excess of the base (e.g. 1.2-3 equivalents). The reaction mixture comprises a single organic solvent (e.g. THF, TBME or Toluene). The intermediate orthoester product is hydrolysed during the work-up under acidic conditions, for example HCl, $H_2SO_4$ and the like with excess water to give the carboxylic ester of the Formula 7.

A compound of Formula 10 or 11 may be prepared from a compound of Formula 7 through diastereoselective hydrolyzation with a suitable enzyme, as described above for Scheme 1. Preferably the enzyme is a lipase from the microorganism *Burkholderia cepacia* or the microorganism Thermomyces *lanuginosus.* Most preferably the enzyme is a commercially available *Burkholderia* cepacia Lipase from Amano Enzyme Inc; most preferably Lipase PS-SD.

As described throughout the specification, many of the disclosed compounds have stereoisomers. Some of these compounds may exist as single enantiomers (enantiopure compounds) or mixtures of enantiomers (enriched and racemic samples), which depending on the relative excess of one enantiomer over another in a sample, may exhibit optical activity. Such stereoisomers, which are non-superimposable mirror images, possess a stereogenic axis or one or more stereogenic centers (i.e., chirality). Other disclosed compounds may be stereoisomers that are not mirror images.

Such stereoisomers, which are known as diastereoisomers, may be chiral or achiral (contain no stereogenic centers). They include molecules containing an alkenyl or cyclic group, so that *cis*/*trans* (or *Z*/*E*) stereoisomers are possible, or molecules containing two or more stereogenic centers, in which inversion of a single stereogenic center generates a corresponding diastereoisomer. Unless stated or otherwise clear (e.g., through use of stereobonds, stereocenter descriptors, etc.) the scope of the present invention generally includes the reference compound and its stereoisomers, whether they are each pure (e.g., enantiopure) or mixtures (e.g., enantiomerically enriched or racemic).
Some of the compounds may also contain a keto or oxime group, so that tautomerism may occur. In such cases, the present invention generally includes tautomeric forms, whether they are each pure or mixtures.

Pharmaceutically acceptable salts of the compounds of Formula 1 include the acid addition and base salts thereof.

Suitable acid addition salts are formed from acids which form non-toxic salts. Examples include the acetate, adipate, aspartate, benzoate, besylate, bicarbonate/carbonate, bisulphate/sulphate, borate, camsylate, citrate, cyclamate, edisylate, esylate, formate, fumarate, gluceptate, gluconate, glucuronate, hexafluorophosphate, hibenzate, hydrochloride/chloride, hydrobromide/bromide, hydroiodide/iodide, isethionate, lactate, malate, maleate, malonate, mesylate, methylsulphate, naphthylate, 2-napsylate, nicotinate, nitrate, orotate, oxalate, palmitate, pamoate, phosphate/hydrogen phosphate/dihydrogen phosphate, pyroglutamate, saccharate, stearate, succinate, tannate, tartrate, tosylate, trifluoroacetate and xinofoate salts.

Suitable base salts are formed from bases which form non-toxic salts. Examples include the aluminium, arginine, benzathine, calcium, choline, diethylamine, diolamine, glycine, lysine, magnesium, meglumine, olamine, potassium, sodium, tromethamine and zinc salts.

Hemisalts of acids and bases may also be formed, for example, hemisulphate and hemicalcium salts.

For a review on suitable salts, see Handbook of Pharmaceutical Salts: Properties, Selection, and Use by Stahl and Wermuth (Wiley-VCH, 2002).

Pharmaceutically acceptable salts of compounds of Formula 1 may be prepared by one or more of three methods:

(i) by reacting the compound of Formula 1 with the desired acid or base;
(ii) by removing an acid- or base-labile protecting group from a suitable precursor of the compound of Formula 1 or by ring-opening a suitable cyclic precursor, for example, a lactone or lactam, using the desired acid or base; or
(iii) by converting one salt of the compound of Formula 1 to another by reaction with an appropriate acid or base or by means of a suitable ion exchange column.

All three reactions are typically carried out in solution. The resulting salt may precipitate out and be collected by filtration or may be recovered by evaporation of the solvent. The degree of ionisation in the resulting salt may vary from completely ionised to almost non-ionised.

The compounds of the invention may exist in a continuum of solid states ranging from fully amorphous to fully crystalline. The term 'amorphous' refers to a state in which the material lacks long range order at the molecular level and, depending upon temperature, may exhibit the physical properties of a solid or a liquid. Typically such materials do not give distinctive X-ray diffraction patterns and, while exhibiting the properties of a solid, are more formally described as a liquid. Upon heating, a change from solid to liquid properties occurs which is characterised by a change of state, typically second order ('glass transition'). The term 'crystalline' refers to a solid phase in which the material has a regular ordered internal structure at the molecular level and gives a distinctive X-ray diffraction pattern with defined peaks. Such materials when heated sufficiently will also exhibit the properties of a liquid, but the change from solid to liquid is characterised by a phase change, typically first order ('melting point').

The compounds of the invention may also exist in unsolvated and solvated forms. The term 'solvate' is used herein to describe a molecular complex comprising the compound of the invention and one or more pharmaceutically acceptable solvent molecules, for example, ethanol. The term 'hydrate' is employed when said solvent is water.

A currently accepted classification system for organic hydrates is one that defines isolated site, channel, or metal-ion coordinated hydrates - see Polymorphism in Pharmaceutical Solids by K. R. Morris (Ed. H. G. Brittain, Marcel Dekker, 1995). Isolated site hydrates are ones in which the water molecules are isolated from direct contact with each other by intervening organic molecules. In channel hydrates, the water molecules lie in lattice channels where they are next to other water molecules. In metal-ion coordinated hydrates, the water molecules are bonded to the metal ion.

When the solvent or water is tightly bound, the complex will have a well-defined stoichiometry independent of humidity. When, however, the solvent or water is weakly bound, as in channel solvates and hygroscopic compounds, the water/solvent content will be dependent on humidity and drying conditions. In such cases, non-stoichiometry will be the norm.

Also included within the scope of the invention are multi-component complexes (other than salts and solvates) wherein the drug and at least one other component are present in stoichiometric or non-stoichiometric amounts. Complexes of this type include clathrates (drug-host inclusion complexes) and co-crystals. The latter are typically defined as crystalline complexes of neutral molecular constituents which are bound together through non-covalent interactions, but could also be a complex of a neutral molecule with a salt. Co-crystals may be prepared by melt crystallisation, by recrystallisation from solvents, or by physically grinding the components together - see Chem Commun, 17, 1889-1896, by O. Almarsson and M. J. Zaworotko (2004). For a general review of multi-component complexes, see J Pharm Sci, 64 (8), 1269-1288, by Haleblian (August 1975).

The compounds of the invention may also exist in a mesomorphic state (mesophase or liquid crystal) when subjected to suitable conditions. The mesomorphic state is intermediate between the true crystalline state and the true liquid state (either melt or solution). Mesomorphism arising as the result of a change in temperature is described as 'thermotropic' and that resulting from the addition of a second component, such as water or another solvent, is described as 'lyotropic'. Compounds that have the potential to form lyotropic mesophases are described as 'amphiphilic' and consist of molecules which possess an ionic (such as $-COO^-Na^+$, $-COO^-K^+$, or $-SO_3^-Na^+$) or non-ionic (such as - $N^-N^+(CH_3)_3$) polar head group. For more information, see Crystals and the Polarizing Microscope by N. H. Hartshorne and A. Stuart, 4th Edition (Edward Arnold, 1970).

Hereinafter all references to compounds of Formula 1 include references to salts, solvates, multi-component complexes and liquid crystals thereof and to solvates, multi-component complexes and liquid crystals of salts thereof; and include all polymorphs and crystal habits thereof, prodrugs and isomers thereof (including optical, geometric and tautomeric isomers) as hereinafter defined and isotopically-labeled compounds of Formula 1.

The compounds of Formula 1 may be assessed for their biopharmaceutical properties, such as solubility and solution stability (across pH), permeability, etc., in order to select the most appropriate dosage form and route of administration for treatment of the proposed indication.

Compounds of Formula 1 intended for pharmaceutical use may be administered as crystalline or amorphous products. They may be obtained, for example, as solid plugs, powders, or films by methods such as precipitation, crystallization, freeze drying, spray drying, or evaporative drying. Microwave or radio frequency drying may be used for this purpose.

They may be administered alone or in combination with one or more other compounds of the invention or in combination with one or more other drugs (or as any combination thereof). Generally, they will be administered as a formulation in association with one or more pharmaceutically acceptable excipients. The term 'excipient' is used herein to describe any ingredient other than the compound(s) of the invention. The choice of excipient will to a large extent depend on factors such as the particular mode of administration, the effect of the excipient on solubility and stability, and the nature of the dosage form.

Pharmaceutical compositions suitable for the delivery of compounds of Formula 1 and methods for their preparation will be readily apparent to those skilled in the art. Such compositions and methods for their preparation may be found, for example, in Remington's Pharmaceutical Sciences, 19th Edition (Mack Publishing Company, 1995).

ORAL ADMINISTRATION

The compounds of Formula 1, in particular (3*S*,5*R*)-3-aminomethyl-5-methyl-octanoic acid Form A, may be administered orally. Oral administration may involve swallowing, so that the compound enters the gastrointestinal tract, and/or buccal, lingual, or sublingual administration by which the compound enters the blood stream directly from the mouth.

Formulations suitable for oral administration include solid, semi-solid and liquid systems such as tablets; soft or hard capsules containing multi- or nano-particulates, liquids, or powders; lozenges (including liquid-filled); chews; gels; fast dispersing dosage forms; films; ovules; sprays; and buccal/mucoadhesive patches.

Liquid formulations include suspensions, solutions, syrups and elixirs. Such formulations may be employed as fillers in soft or hard capsules (made, for example, from gelatin or hydroxypropylmethylcellulose) and typically comprise a carrier, for example, water, ethanol, polyethylene glycol, propylene glycol, methylcellulose, or a suitable oil, and one or more emulsifying agents and/or suspending agents. Liquid formulations may also be prepared by the reconstitution of a solid, for example, from a sachet.

The compounds of Formula 1, in particular (3*S*,5*R*)-3-aminomethyl-5-methyl-octanoic acid Form A, may also be used in fast-dissolving, fast-disintegrating dosage forms such as those described in Expert Opinion in Therapeutic Patents, 11 (6), 981-986, by Liang and Chen (2001).

For tablet dosage forms, depending on dose, the drug may make up from 1 weight % to 80 weight % of the dosage form, more typically from 5 weight % to 60 weight % of the dosage form. In addition to the drug, tablets generally contain a disintegrant. Examples of disintegrants include sodium starch glycolate, sodium carboxymethyl cellulose, calcium carboxymethyl cellulose, croscarmellose sodium, crospovidone, polyvinylpyrrolidone, methyl cellulose, microcrystalline cellulose, lower alkyl-substituted hydroxypropyl cellulose, starch, pregelatinised starch and sodium alginate. Generally, the disintegrant will comprise from 1 weight % to 25 weight %, preferably from 5 weight % to 20 weight % of

the dosage form.

Binders are generally used to impart cohesive qualities to a tablet formulation. Suitable binders include microcrystalline cellulose, gelatin, sugars, polyethylene glycol, natural and synthetic gums, polyvinylpyrrolidone, pregelatinised starch, hydroxypropyl cellulose and hydroxypropyl methylcellulose. Tablets may also contain diluents, such as lactose (monohydrate, spray-dried monohydrate, anhydrous and the like), mannitol, xylitol, dextrose, sucrose, sorbitol, microcrystalline cellulose, starch and dibasic calcium phosphate dihydrate.

Tablets may also optionally comprise surface active agents, such as sodium lauryl sulfate and polysorbate 80, and glidants such as silicon dioxide and talc. When present, surface active agents may comprise from 0.2 weight % to 5 weight % of the tablet, and glidants may comprise from 0.2 weight % to 1 weight % of the tablet.

Tablets also generally contain lubricants such as magnesium stearate, calcium stearate, zinc stearate, sodium stearyl fumarate, and mixtures of magnesium stearate with sodium lauryl sulphate. Lubricants generally comprise from 0.25 weight % to 10 weight %, preferably from 0.5 weight % to 3 weight % of the tablet.

Other possible ingredients include anti-oxidants, colourants, flavouring agents, preservatives and taste-masking agents.

Exemplary tablets contain up to about 80% drug, from about 10 weight % to about 90 weight % binder, from about 0 weight % to about 85 weight % diluent, from about 2 weight % to about 10 weight % disintegrant, and from about 0.25 weight % to about 10 weight % lubricant.

Tablet blends may be compressed directly or by roller to form tablets. Tablet blends or portions of blends may alternatively be wet-, dry-, or melt-granulated, melt congealed, or extruded before tabletting. The final formulation may comprise one or more layers and may be coated or uncoated; it may even be encapsulated.

The formulation of tablets is discussed in Pharmaceutical Dosage Forms: Tablets, Vol. 1, by H. Lieberman and L. Lachman (Marcel Dekker, New York, 1980).

Consumable oral films for human or veterinary use are typically pliable water-soluble or water-swellable thin film dosage forms which may be rapidly dissolving or mucoadhesive and typically comprise a compound of Formula 1, a film-forming polymer, a binder, a solvent, a humectant, a plasticiser, a stabiliser or emulsifier, a viscosity-modifying agent and a solvent. Some components of the formulation may perform more than one function.

The compound of Formula 1 may be water-soluble or insoluble. A water-soluble compound typically comprises from 1 weight % to 80 weight %, more typically from 20 weight % to 50 weight %, of the solutes. Less soluble compounds may comprise a greater proportion of the composition, typically up to 88 weight % of the solutes. Alternatively, the compound of Formula 1 may be in the form of multiparticulate beads.

The film-forming polymer may be selected from natural polysaccharides, proteins, or synthetic hydrocolloids and is typically present in the range 0.01 to 99 weight %, more typically in the range 30 to 80 weight %.

Other possible ingredients include anti-oxidants, colorants, flavourings and flavour enhancers, preservatives, salivary stimulating agents, cooling agents, co-solvents (including oils), emollients, bulking agents, anti-foaming agents, surfactants and taste-masking agents.

Films in accordance with the invention are typically prepared by evaporative drying of thin aqueous films coated onto a peelable backing support or paper. This may be done in a drying oven or tunnel, typically a combined coater dryer, or by freeze-drying or vacuuming.

Solid formulations for oral administration may be formulated to be immediate and/or modified release. Modified release formulations include delayed-, sustained-, pulsed-, controlled-, targeted and programmed release.

Suitable modified release formulations for the purposes of the invention are described in US Patent No. 6,106,864. Details of other suitable release technologies such as high energy dispersions and osmotic and coated particles are to be found in Pharmaceutical Technology On-line, 25(2), 1-14, by Verma et al (2001). The use of chewing gum to achieve controlled release is described in WO 00/35298.

## PARENTERAL ADMINISTRATION

The compounds of Formula 1, in particular (3*S*,5*R*)-3-aminomethyl-5-methyl-octanoic acid Form A, may also be administered directly into the blood stream, into muscle, or into an internal organ. Suitable means for parenteral administration include intravenous, intraarterial, intraperitoneal, intrathecal, intraventricular, intraurethral, intrasternal, intracranial, intramuscular, intrasynovial and subcutaneous. Suitable devices for parenteral administration include needle (including microneedle) injectors, needle-free injectors and infusion techniques.

## TOPICAL ADMINISTRATION

The compounds of Formula 1, in particular (3*S*,5*R*)-3-aminomethyl-5-methyl-octanoic acid Form A, may also be administered topically, (intra)dermally, or transdermally to the skin or mucosa. Typical formulations for this purpose include gels, hydrogels, lotions, solutions, creams, ointments, dusting powders, dressings, foams, films, skin patches,

wafers, implants, sponges, fibres, bandages and microemulsions. Liposomes may also be used. Typical carriers include alcohol, water, mineral oil, liquid petrolatum, white petrolatum, glycerin, polyethylene glycol and propylene glycol. Penetration enhancers may be incorporated - see, for example, J Pharm Sci, 88 (10), 955-958, by Finnin and Morgan (October 1999). Other means of topical administration include delivery by electroporation, iontophoresis, phonophoresis, sonophoresis and microneedle or needle-free (e.g. Powderject™, Bioject™, etc.) injection.

INHALED/INTRANASAL ADMINISTRATION

The compounds of Formula 1, in particular (3*S*,5*R*)-3-aminomethyl-5-methyl-octanoic acid Form A, can also be administered intranasally or by inhalation, typically in the form of a dry powder (either alone, as a mixture, for example, in a dry blend with lactose, or as a mixed component particle, for example, mixed with phospholipids, such as phosphatidylcholine) from a dry powder inhaler, as an aerosol spray from a pressurised container, pump, spray, atomiser (preferably an atomiser using electrohydrodynamics to produce a fine mist), or nebuliser, with or without the use of a suitable propellant, such as 1,1,1,2-tetrafluoroethane or 1,1,1,2,3,3,3-heptafluoropropane, or as nasal drops. For intranasal use, the powder may comprise a bioadhesive agent, for example, chitosan or cyclodextrin.

RECTAL/INTRAVAGINAL ADMINISTRATION

The compounds of Formula 1, in particular (3*S*,5*R*)-3-aminomethyl-5-methyl-octanoic acid Form A, may be administered rectally or vaginally, for example, in the form of a suppository, pessary, or enema. Cocoa butter is a traditional suppository base, but various alternatives may be used as appropriate.

OCULAR/AURAL ADMINISTRATION

The compounds of Formula 1, in particular (3*S*,5*R*)-3-aminomethyl-5-methyl-octanoic acid Form A, may also be administered directly to the eye or ear, typically in the form of drops of a micronised suspension or solution in isotonic, pH-adjusted, sterile saline. Other formulations suitable for ocular and aural administration include ointments, gels, biodegradable (e.g. absorbable gel sponges, collagen) and non-biodegradable (e.g. silicone) implants, wafers, lenses and particulate or vesicular systems, such as niosomes or liposomes.

OTHER TECHNOLOGIES

The compounds of Formula 1, in particular (3*S*,5*R*)-3-aminomethyl-5-methyl-octanoic acid Form A, may be combined with soluble macromolecular entities, such as cyclodextrin and suitable derivatives thereof or polyethylene glycol-containing polymers, in order to improve their solubility, dissolution rate, taste-masking, bioavailability and/or stability for use in any of the aforementioned modes of administration.
Drug-cyclodextrin complexes, for example, are found to be generally useful for most dosage forms and administration routes. Both inclusion and non-inclusion complexes may be used. As an alternative to direct complexation with the drug, the cyclodextrin may be used as an auxiliary additive, i.e. as a carrier, diluent, or solubiliser. Most commonly used for these purposes are alpha-, beta- and gamma-cyclodextrins, examples of which may be found in International Patent Applications Nos. WO 91/11172, WO 94/02518 and WO 98/55148.

KIT-OF-PARTS

Inasmuch as it may desirable to administer a combination of active compounds, for example, for the purpose of treating a particular disease or condition, it is within the scope of the present invention that two or more pharmaceutical compositions, at least one of which contains a compound of Formula 1, may conveniently be combined in the form of a kit suitable for coadministration of the compositions.
Thus the kit of the invention comprises two or more separate pharmaceutical compositions, at least one of which contains a compound of Formula 1, in particular (3S,5R)-3-aminomethyl-5-methyl-octanoic acid Form A, and means for separately retaining said compositions, such as a container, divided bottle, or divided foil packet. An example of such a kit is the familiar blister pack used for the packaging of tablets, capsules and the like.
The kit of the invention is particularly suitable for administering different dosage forms, for example, oral and parenteral, for administering the separate compositions at different dosage intervals, or for titrating the separate compositions against one another. To assist compliance, the kit typically comprises directions for administration and may be provided with a so-called memory aid.

DOSAGE

For administration to human patients, the total daily dose of the compounds of Formula 1 is typically in the range 0.1 mg to 1000 mg depending, of course, on the mode of administration. The total daily dose may be administered in single or divided doses and may, at the physician's discretion, fall outside of the typical range given herein. The preferred daily dose range for (3S,5R)-3-aminomethyl-5-methyl-octanoic acid Form A is in the range 1 mg to 250 mg; more preferably the daily dose range is in the range 1 mg to 125 mg.

These dosages are based on an average human subject having a weight of about 60kg to 70kg. The physician will readily be able to determine doses for subjects whose weight falls outside this range, such as infants and the elderly.

For the avoidance of doubt, references herein to "treatment" include references to curative, palliative and prophylactic treatment.

EXAMPLES

The following examples are intended to be illustrative and non-limiting, and represent specific embodiments of the present invention.

Enzyme screening was carried out using a 96-well plate, which is described in D. Yazbeck et al., Synth. Catal. 345:524-32 (2003), the complete disclosure of which is herein incorporated by reference for all purposes. All enzymes used in the screening plate (see Table 2) were obtained from commercial enzyme suppliers including Amano Enzyme Inc. (Nagoya, Japan), Roche (Basel, Switzerland), Novo Nordisk (Bagsvaerd, Denmark), Altus Biologics Inc. (Cambridge, MA), Biocatalytics (Pasadena, CA), Toyobo (Osaka, Japan), Sigma-Aldrich (St. Louis, MO), Fluka (Buchs, Switzerland), Genencor International, Inc. (Rochester, NY), and Valley Research (South Bend, IN). The screening reactions were performed in an Eppendorf Thermomixer-R (VWR). Subsequent larger scale enzymatic resolutions employed LIPOLASE® 100L EX, which is available form Novo-Nordisk A/S (CAS no. 9001-62-1), as well as Lipase PS, PS-C I, PS-C II, and PS-D I, which are available from Amano Enzyme Inc.

EXAMPLE 1. Preparation of (*R*)-methanesulfonic acid 2-methyl-pentyl ester A 4000 L reactor was charged with (*R*)-2-methyl-pentan-1-ol (260 kg, 2500 mol), methyl tertiary butyl ether (2000 L), and cooled to -10°C to 0°C. Methanesulfonyl chloride (310 kg, 2600 mol) was charged, and then $Et_3N$ (310 kg, 3100 mol) was added while maintaining the internal temperature at 0°C to 10°C. After the addition was complete, the reaction mixture was warmed to 15°C to 25°C and stirred at this temperature for at least 1 hour until complete by gas chromatography analysis. A solution of aqueous HCl (88 kg of HCl in 700 L of water) was then added to the reaction mixture. The resulting mixture stirred for at least 15 minutes, settled for at least 15 minutes, and then the lower aqueous phase was removed. The upper organic phase was washed with water (790 L) and aqueous sodium bicarbonate (67 kg of sodium bicarbonate in 840 L of water). The solution was then concentrated under vacuum to remove the methyl tertiary butyl ether to afford the titled compound as an oil (472 kg, 95% yield). $^1H$ NMR (400MHz, $CDCl_3$) 4.07-3.93 ppm (m, 2H), 2.97 (s, 3H), 1.91-1.80 (m, 1 H), 1.42-1.09 (m, 4H), 0.94 (d, J=6.57Hz, 3H), 0.87 (t, J=6.56Hz, 3H); $^{13}C$ NMR ($CDCl_3$) 74.73, 37.01, 34.81, 32.65, 19.71, 16.29, 14.04.

EXAMPLE 2. Preparation of (2'*R*)-2-cyano-2-(2'-methyl-pentyl)-succinic acid diethyl ester

A 4000 L reactor was charged with (R)-methanesulfonic acid 2-methyl-pentyl ester (245 kg, 1359 mol), 2-cyano-succinic acid diethyl ester (298 kg, 1495 mol), and anhydrous ethanol (1300 kg). Sodium ethoxide (506 kg, 21 wt% in ethanol) was added. The resulting solution was heated to 70°C to 75°C, and the mixture stirred at this temperature for at least 18 hour until complete by gas chromatography analysis. After the reaction was complete, a solution of aqueous HCl (32 kg of HCl in 280 L of water) was then added to the reaction mixture until the pH was < 2. Additional water (400 L) was added, and the reaction mixture was then concentrated under vacuum to remove the ethanol. Methyl tertiary butyl ether (1000 kg) was added, and the mixture was stirred for at least 15 minutes, settled for at least 15 minutes, and then the lower aqueous layer was back extracted with methyl tertiary butyl ether (900 kg). The combined organic phases were concentrated under vacuum to afford the titled compound as a dark oil (294 kg, 79% yield corrected for purity). $^1H$ NMR (400MHz, $CDCl_3$) 4.29 ppm (q, J=7.07Hz, 2H), 4.18 (q, J=7.07Hz, 2H), 3.03 (dd, J=6.6, 7.1Hz, 2H), 1.93-1.61 (m, 3H), 1.40-1.20 (m, 10H), 0.95-0.82 (m, 6H); $^{13}C$ NMR ($CDCl_3$) 168.91, 168.67, 168.59, 168.57, 119.08, 118.82, 62.95, 62.90, 44.32, 44.19, 42.21, 42.02, 39.77, 39.64, 30.05, 29.91, 20.37, 19.91, 19.66, 13.99.

EXAMPLE 3. Preparation of (5*R*)-3-cyano-5-methyl-octanoic acid ethyl ester (Method A)

A 4000 L reactor was charged with NaCl (175 kg, 3003 mol), tetrabutylammonium bromide (33.1 kg, 103 mol), water (87 L), and dimethylsulfoxide (1000 kg). (2'*R*)-2-Cyano-2-(2'-methyl-pentyl)-succinic acid diethyl ester (243 kg, 858 mol) was charged and the mixture was heated to 135°C to 138°C and stirred at this temperature for at least 48

hours, until complete by gas chromatography analysis. After the reaction was cooled to 25°C to 35°C, heptane (590 kg) was added, and the mixture stirred for at least 15 minutes, settled for at least 15 minutes, and then the lower aqueous phase was removed. The upper organic phase was washed with water (800 L). The heptane solution containing the product was decolorized with carbon, and concentrated under vacuum to afford the titled compound as an orange oil (133.9 kg, 74% yield corrected for purity). $^1$H NMR (400MHz, $CDCl_3$) 4.20 ppm (q, J=7.07Hz, 2H), 3.13-3.01 (m, 1 H), 2.75-2.49 (m, 2H), 1.80-1.06 (m, 10H), 0.98-086 (m, 6H); $^{13}$C NMR (CDCl3) 169.69, 169.65, 121.28, 120.99, 61.14, 39.38, 39.15, 38.98, 37.67, 37.23, 36.95, 30.54, 30.47, 25.67, 25.45, 19.78, 19.61, 19.53, 18.56, 14.13, 14.05.

EXAMPLE 4. Preparation of (5*R*)-3-cyano-5-methyl-octanoic acid ethyl ester (Method B)

A 250 mL flask was charged with LiCl (3.89 g, 0.0918 mol), water (7 mL), and dimethylsuloxide (72 mL). (2'*R*)-2-Cyano-2-(2'-methyl-pentyl)-succinic acid diethyl ester (25.4 g, 0.0706 mol, 78.74% by gas chromatography) was charged and the mixture was heated to 135°C to 138°C and stirred at this temperature for at least 24 hours, until complete by gas chromatography analysis. After the reaction was cooled to 25°C to 35°C, heptane (72 mL), saturated NaCl (72 mL), and water (72 mL) was added and the mixture stirred for at least 15 minutes, settled for at least 15 minutes, and then the lower aqueous phase was washed with heptane (100 mL). The combined organic phases were concentrated under vacuum to afford the titled compound as an orange oil (13.0 g, 84% yield corrected for purity).

EXAMPLE 5. Preparation of (5*R*)-3-cyano-5-methyl-octanoic acid sodium salt

A 4000 L reactor was charged with (5*R*)-3-cyano-5-methyl-octanoic acid ethyl ester (250 kg, 1183 mol) and tetrahydrofuran (450 kg). An aqueous solution of NaOH was prepared (190 kg of 50% NaOH in 350 L of water) and then added to the tetrahydrofuran solution. The resulting solution was stirred at 20°C to 30°C for at least 2 hours, until the reaction was complete by gas chromatography analysis. After this time, tetrahydrofuran was removed by vacuum distillation to afford an aqueous solution of the titled compound, which was used immediately in the next step.

EXAMPLE 6. Preparation of (5*R*)-3-aminomethyl-5-methyl-octanoic acid sodium salt

A 120 L autoclave was charged with sponge nickel catalyst (3.2 kg, Johnson & Mathey A7000) followed by an aqueous solution of (5*R*)-3-cyano-5-methyl-octanoic acid sodium salt (15 kg in 60 L of water) and the resulting mixture was hydrogenated under 50 psig of hydrogen at 30°C to 35°C for at least 18 hours, or until hydrogen uptake ceased. The reaction was then cooled to 20°C to 30°C, and the spent catalyst was removed by filtration through a 0.2 $\mu$ filter. The filter cake was washed with water (2 x 22 L), and the resulting aqueous solution of the titled compound was used directly in the next step.

EXAMPLE 7. Preparation of (5*R*)-3-aminomethyl-5-methyl-octanoic acid

A 4000 L reactor was charged with an aqueous solution of (5*R*)-3-aminomethyl-5-methyl-octanoic acid (~150 kg in ~1000 L of water) and cooled to 0°C to 5°C. Glacial acetic acid was added until the pH was 6.3 to 6.8. To the mixture was added anhydrous ethanol (40 kg). The resulting slurry was heated to 65°C to 70°C for less than 20 minutes and was cooled to 0°C to 5°C over 3 hours. The product was collected by filtration to afford the titled compound as a water-wet cake (76 kg, 97% yield corrected for purity, 10% water by Karl Fischer analysis), which was used in the next step. $^1$H NMR (400MHz, $D_3COD$) 4.97 ppm (BS, 3H), 3.00-2.74 (m, 2H), 2.48-2.02 (m, 3H), 1.61-1.03 (m, 7H), 0.94-086 (m, 6H); $^{13}$C NMR ($D_3COD$) 181.10, 181.07, 46.65, 45.86, 44.25, 43.15, 42.16, 41.64, 41.35, 33.45, 31.25, 31.20, 21.45, 21.41, 20.52, 20.12, 15.15, 15.12.

EXAMPLE 8. Preparation of (3*S*,5*R*)-3-aminomethyl-5-methyl-octanoic acid via contact with a resolving agent

A 4000 L reactor was charged with water wet (10%) (5*R*)-3-aminomethyl-5-methyl-octanoic acid (76 kg, 365 mol), (S)-mandelic acid (34.8 kg, 229 mol), anhydrous ethanol (1780 kg), and water (115 L). The resulting mixture was heated to 65°C to 70°C and stirred until the solids dissolved. The solution was then cooled to 0°C to 5°C over 2 hours and stirred at this temperature for an additional 1 hour. The product was collected by filtration, and the cake was washed with -20°C ethanol (3 x 60 kg). The crude product (18 kg in 48% yield) and ethanol (167 kg) were charged to a reactor. The mixture was cooled to 0°C to 5°C and stirred at this temperature for 1.5 hours. The product was then collected by filtration, and the cake was washed with -20°C ethanol (3 x 183 kg) to afford the titled compound (17 kg, 94% yield). The quasimolecular ion (MH+) of the titled compound was observed at 188.1653 amu and is in agreement with the theoretical value of 188.1650; the measured value establishes the molecular formula as $C_{10}H_{21}NO_2$ as no reasonable alternate chemical entity containing only C, H, N, and O can exist with a molecular ion within the 5-ppm (0.9 mDa)

experimental error of the measured value; IR (KBr) 2955.8 $cm^{-1}$, 22.12.1, 1643.8, 1551.7, 1389.9; $^1H$ NMR (400MHz, $D_3COD$) 4.91 ppm (bs, 2H), 3.01-2.73 (m, 2H), 2.45-2.22 (m, 2H), 1.60-1.48 (m, 1 H), 1.45-1.04 (m, 6H), 0.98-086 (m, 6H); $^{13}C$ NMR ($D_3COD$) 181.04, 45.91, 44.30, 42.13, 40.65, 33.42, 31.24, 21.39, 20.49, 15.11.

EXAMPLE 9. Enzyme screening via enzymatic hydrolysis of (5*R*)-3-cyano-5-methyl-octanoic acid ethyl ester (Formula 15) to yield (3*S*,5*R*)-3-cyano-5-methyl-octanoic acid sodium salt (Formula 16, $R^{10}=Na^+$) and (3*R*,5*R*)-3-cyano-5-methyl-octanoic acid ethyl ester (Formula 17, $R^{11}$=Et) or (3S,5R)-3-cyano-5-methyl-octanoic acid ethyl ester (Formula 16, $R^{10}$=Et) and (3*R*,5*R*)-3-cyano-5-methyl-octanoic acid sodium salt (Formula 17, $R^{11}=Na^+$).

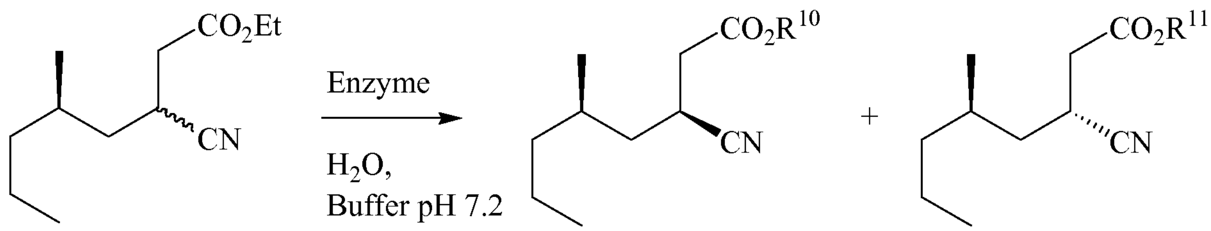


15 16 17

Enzyme screening was carried out using a screening kit comprised of individual enzymes deposited in separate wells of a 96-well plate, which was prepared in advance in accordance with a method described in D. Yazbeck et al., Synth. Catal. 345:524-32 (2003). Each of the wells has an empty volume of 0.3 mL (shallow well plate). One well of the 96-well plate contains only phosphate buffer (10 μL, 0.1 M, pH 7.2). With few exceptions, each of the remaining wells contain one aliquot of enzyme (10 μL, 83 mg/mL), most of which are listed in Table 2, above. Prior to use, the screening kit is removed from storage at -80°C and the enzymes are allowed to thaw at room temperature for about 5 min. Potassium phosphate buffer (85 μL, 0.1 M, pH 7.2) is dispensed into each of the wells using a multi-channel pipette. Concentrated substrate (Formula 15, 5 μL) is subsequently added to each well via a multi-channel pipette and the 96 reaction mixtures are incubated at 30°C and 750 rpm. The reactions are quenched and sampled after 24 h by transferring each of the reaction mixtures into separate wells of a second 96-well plate. Each of the wells has an empty volume of 2 mL (deep well plate) and contains ethyl acetate (1 mL) and HCl (1N, 100 μL). The components of each well are mixed by aspirating the well contents with a pipette. The second plate is centrifuged and 100 μL of the organic supernatant is transferred from each well into separate wells of a third 96-well plate (shallow plate). The wells of the third plate are subsequently sealed using a penetrable mat cover.

Once the wells are sealed, the third plate is transferred to a GC system for determination of diastereoselectivity (de).

Table 3 lists enzyme, trade name, E value, $\chi$, and selectivity for some of the enzymes that were screened. For a given enzyme, the E value may be interpreted as the relative reactivity of a pair of diastereomers (substrates). The E values listed in Table 3 were calculated from GC/derivatization data (fractional conversion, $\chi$, and de) using a computer program called Ee2, which is available from the University of Graz. In Table 3, selectivity corresponds to the diastereomer-(3*R*,5*R*)-3-cyano-5-methyl-octanoic acid ethyl ester or (3*S*,5*R*)-3-cyano-5-methyl-octanoic acid ethyl ester-that underwent the greatest hydrolysis for a given enzyme.

Table 3. Results from Screening Reactions of Example 1

| **Enzyme** | Trade Name | E | $\chi$ | Selectivity |
|---|---|---|---|---|
| Porcine Pancreatic Lipase | Altus 03 | 1.5 | 15 | (3R,5R) |
| *Candida cylindracea* Lipase | Fluka 62302 | 1.4 | 3 | (3R,5R) |
| *Burkholderia cepacia* Lipase | Amano Lipase AH | 200 | 15 | (3R,5R) |
| *Pseudomonas fluorescens* Lipase | Amano Lipase AK 20 | 200 | 25 | (3R,5R) |
| *Candida rugosa* Lipase | Amano Lipase AYS | 1.4 | 2 | (3R,5R) |
| *Rhizopus delemar* Lipase | Amano Lipase D | 6 | 44 | (3S,5R) |
| *Rhizopus oryzae* Lipase | Amano Lipase F-AP 15 | 20 | 1 | (3S,5R) |
| *Penicillium camembertii* Lipase | Amano Lipase G 50 | 1.1 | 6 | (3S,5R) |
| *Mucor javanicus* Lipase | Amano Lipase M 10 | 8 | 3 | (3S,5R) |
| *Burkholderia cepacia* Lipase | Amano Lipase PS | 200 | 45 | (3R,5R) |
| *Pseudomonas sp.* Lipase | BioCatalytics 103 | 4 | 7 | (3S,5R) |
| Microbial, lyophilized Lipase | BioCatalytics 108 | 17 | 45 | (3R,5R) |

(continued)

| Enzyme | Trade Name | E | $\chi$ | Selectivity |
|---|---|---|---|---|
| CAL-B, lyophilized | BioCatalytics 110 | 1.2 | 96 | (3S,5R) |
| *Candida sp.,* lyophilized | BioCatalytics 111 | 1.2 | 8 | (3R,5R) |
| CAL-A, lyophilized | BioCatalytics 112 | 1.6 | 5 | (3R,5R) |
| *Thermomyces sp.* Lipase | BioCatalytics 115 | 7 | 50 | (3S,5R) |
| *Alcaligines sp.,* lyophilized Lipase | BioCatalytics 117 | 15 | 31 | (3R,5R) |
| CAL-B, L2 Sol | Chriazyme L2 Sol | 1.3 | 31 | (3R,5R) |
| *Thermomuces lanuginosus* Lipase | Sigma L9 Lipolase | 15 | 50 | (3S,5R) |
| *Thermomuces lanuginosus* Lipase | Sigma L10 Novo871 | 10 | 68 | (3S,5R) |
| *Rhizomucor miehei* Lipase | Sigma L6 Palatase | 5.3 | 90 | (3S,5R) |
| Fungal protease concentrate | Genencor | 10 | 10 | (3R,5R) |
| Bovine Pancreas Protease | Sigma P18 $\alpha$-chymotrypsin I | 10 | 10 | (3R,5R) |
| Pineapple [Ananas comosus & Ananas Bromelian | Concentrate bracteatus (L)] | 10 | 10 | (3*R*,5*R*) |
| Porcine kidney Acylase | Sigma A-S2 Acylase I | 2 | 60 | (3S,5R) |
| Esterase from *Mucor meihei* | Fluka E5 | 5 | 79 | (3S,5R) |
| Cholinesterase, acetyl | Sigma ES8 | 1.1 | 54 | (3S,5R) |
| Cholesterol Esterase | BioCatalytics E3 | 1.1 | 54 | (3S,5R) |
| PLE - Ammonium Sulfate | BioCatalytics 123 | 1.3 | 71 | (3S,5R) |

EXAMPLE 10. Preparation of (3*S*,5*R*)-3-cyano-5-methyl-octanoic acid *tert*-butyl-ammonium salt via enzymatic resolution

To a 50 mL reactor equipped with a pH electrode, an overhead stirrer and a base addition line, was added (5*R*)-3-cyano-5-methyl-octanoic acid ethyl ester (8 g, 37.85 mmol), followed by calcium acetate solution (8 mL), deionized water (3.8 mL), and LIPOLASE® 100L EX (0.2 mL). The resulting suspension was stirred at room temperature for 24 hours. The pH of the solution was maintained at 7.0 by adding 4M NaOH. The course of the reaction was tracked by gas chromatography (conversion and % de of the product and starting material), and was stopped after 45% of the starting material had been consumed (-4.3 mL of NaOH had added). After reaction completion, toluene (20 mL) was added, and the mixture stirred for 1 minute. The pH was lowered to 3.0 by adding concentrated aqueous HCl and the solution was stirred for 5 minutes and then transferred to a separatory funnel/extractor. The organic layer was separated and the aqueous layer extracted once with 10 mL of toluene. The organic layers were pooled and toluene evaporated to dryness. The crude product (sodium salt of (3*S*,5*R*)-3-cyano-5-methyl-octanoic acid, 75% diastereomeric excess by gas chromatography) was re-suspended in methyl tertiary butyl ether (40 mL). Tert-butylamine (1.52 g, 1.1 equivalents) was added dropwise to the mixture with stirring over a 5 minute period. Crystals precipitated shortly after the addition was finished and they were collected in a buchner funnel. The solid was washed with methyl tertiary-butyl ether (2 x 20 mL). The residue was then dried under vacuum to afford the titled compound (2.58 g, 96% diastereomeric excess by gas chromatography).

EXAMPLE 11. Resolution of (3*S*,5*R*)-3-cyano-5-methyl-octanoic acid ethyl ester via enzymatic hydrolysis of (3*R*,5*R*)-3-cyano-5-methyl-octanoic acid ethyl ester to (3*R*,5*R*)-3-cyano-5-methyl-octanoic acid sodium salt

To a vessel containing sodium phosphate (monobasic) monohydrate (4.7 kg) and water (1650 L) at a temperature of 20°C to 25°C is added 50% NaOH aq (2.0 kg). After stirring for 15 minutes, the pH of the mixture is checked to ensure that it is in the range of 6.0 to 8.0. Amano PS lipase (17 kg) is added and the mixture is stirred for 30 to 60 minutes at 20°C to 25°C. The mixture is filtered to remove solids and the filtrate is combined with sodium bicarbonate (51 kg), (5*R*)-3-cyano-5-methyl-octanoic acid ethyl ester (154 kg), and water (10 L). The mixture is allowed to react at about 50°C for 24 to 48 hours. The course of the enzymatic hydrolysis is monitored by gas chromatography and is considered to be complete when the ratio of (3*S*,5*R*)-3-cyano-5-methyl-octanoic acid ethyl ester to (3*R*,5*R*)-3-cyano-5-methyl-octanoic acid sodium salt is greater than 99:1 based on gas chromatography analaysis. Following completion of the reaction, the mixture is added to a vessel charged with NaCl (510 kg), and the contents of the vessel are stirred at 20°C to 25°C. The mixture is extracted with methyl tertiary-butyl ether (680 L) and the aqueous and organic phases are separated. The aqueous phase is discarded and the organic phase is washed with NaCl (26 kg), sodium bicarbonate (2 kg), and water (85 L). After the solids are dissolved, the mixture is again extracted with MTBE (680 L), the aqueous and organic phases separated, and the organic phase is again washed with NaCl (26 kg), sodium bicarbonate (2 kg), and water (85 L). Following separation of the aqueous and organic phases, the organic phase is distilled at 70°C and

atmospheric pressure to give (3*S*,5*R*)-3-cyano-5-methyl-octanoic acid ethyl ester as an oil (48.9 kg, 88% yield). $^1$H NMR (400MHz, $CDCl_3$) 4.17 ppm (q, J=7.83Hz, 2H), 3.13-3.06 (m, 1 H), 2.71-2.58 (m, 2H), 1.75-1.64 (m, 10H), 0.95 (d, J=6.34 3H), 0.92 (t, J=6.83, 3H, $^{13}$C NMR ($CDCl_3$) 170.4, 121.8, 61.1, 39.6, 38.6, 37.0, 31.0, 25.9, 20.0, 18.5, 13.9.

EXAMPLE 12. Preparation of (3*S*,5*R*)-3-aminomethyl-5-methyl-octanoic acid from (3*S*,5*R*)-3-cyano-5-methyl-octanoic acid ethyl ester

A solution (700 kg) containing (3*S*,5*R*)-3-cyano-5-methyl-octanoic acid ethyl ester (30%) in methyl tertiary-butyl ether is treated with aqueous sodium hypochlorite solution (35 kg, 12%) and water (35 L). After stirring for 2 hours at room temperature, the mixture is allowed to settle for 3 hours, and the aqueous and organic phases are separated. The organic phase is washed with water (150 L) at room temperature and the mixture is allowed to separate into aqueous and organic phases. The organic phase is separated and subsequently reacted with NaOH aq (134 kg, 50%) and water (560 L). The reaction mixture is stirred for 2.5 to 3.5 hours at room temperature and the mixture is allowed to settle for 2 hours. The resulting aqueous phase, which contains (3*S*,5*R*)-3-cyano-5-methyl-octanoic acid sodium salt, is fed to an autoclave which has been charged with sponge nickel A-7063 (43 kg) and purged with nitrogen. The autoclave is heated to 28°C to 32°C and is pressurized with hydrogen to 50 psig. The pressure is maintained at 50 psig for 18 to 24 hours. The autoclave is subsequently cooled to 20°C to 30°C and the pressure is reduced to 20 to 30 psig for sampling. The reaction is complete when the fractional conversion of (3*S*,5*R*)-3-cyano-5-methyl-octanoic acid sodium salt is 99% or greater. The reaction mixture is filtered and the filtrate is combined with an aqueous citric acid solution (64 kg in 136 kg of water) at a temperature of 20°C to 30°C. Ethanol (310 L) is added and the mixture is heated to 55°C to 60°C. The mixture is held for 1 hour and then cooled at a rate of about -15°C/hour until the mixture reaches at temperature of about 2°C to 8°C. The mixture is stirred at that temperature for about 1.5 hours and filtered. The resulting filter cake is rinsed with water (150 L) at 2°C to 8°C and then dried at room temperature with a nitrogen sweep until the water content is less than 1% by Karl Fischer analysis, thus giving crude (3S,5R)-3-aminomethyl-5-methyl-octanoic acid.

The crude product (129 kg) is charged to a vessel. Water (774 kg) and anhydrous ethanol (774 kg) are added to the vessel and the resulting mixture is heated at reflux (about 80°C) until the solution clears. The solution is passed through a polish filter (1μ) and is again heated at reflux until the solution clears. The solution is allowed to cool at a rate of about -20°C/hour until it reaches a temperature of about 5°C, during which a precipitate forms. The resulting slurry is held at 0°C to 5°C for about 90 minutes to complete the crystallization process. The slurry is filtered to isolate the titled compound, which is rinsed with anhydrous ethanol (305 kg) and dried at under a nitrogen sweep at a temperature of 40°C to about 45°C until the water content (by Karl Fischer analysis) and the ethanol content (by gas chromatography analysis) are each less than 0.5% by weight. Representative yield of the titled compound from (3*S*,5*R*)-3-cyano-5-methyl-octanoic acid ethyl ester is about 76%.

EXAMPLE 13. Preparation of (3*S*,5*R*)-3-Cyano-5-methyl-octanoic acid methyl ester

Methane sulfonic acid 2-methyl-pentyl ester

The reaction vessel was charged with toluene (170ml, 8.5 ml/g based in the weight of 2-methyl-pentan-1-ol), 2-methyl-pentan-1-ol (20.00g, 0.20 moles, in one portion) and triethylamine (21.78g, 0.22 moles, in one portion). The reaction mixture was cooled to a temperature of from -10°C to -5°C and methanesulfonyl chloride (22.42g, 0.2 moles) was added dropwise, maintaining the temperature at from -10°C to -5°C. The reaction was stirred for one hour at a temperature of from -10°C to -5°C. The reaction was quenched with 1.0M aqueous HCl (60ml, 3 ml/g based on the weight of 2-methyl-pentan-1-ol) and stirred for 30 minutes. The reaction mixture was allowed to warm to 25°C and the phases separated. The organic phase was washed with 1 M aqueous $NaHCO_3$ (60ml, 3 ml/g based on the weight of 2-methyl-pentan-1-ol) and the phases separated. The resulting toluene solution of methane sulfonic acid 2-methyl-pentyl ester was used directly in the next step.

1-Bromo-2-methyl-pentane

The reaction vessel was charged with methane sulfonic acid 2-methyl-pentyl ester (35.29g, 0.2 moles, toluene solution), $H_2O$ (14ml, 0.4 ml/g based on the weight of methane sulfonic acid 2-methyl-pentyl ester), NaBr (20.14g, 0.2 moles) and tetrabutylammonium bromide (12.61g, 0.04 moles). The reaction mixture was heated to 90°C and stirred at this temperature for 3 hours. $H_2O$ (600ml, 3 ml/g based on the weight of methane sulfonic acid 2-methyl-pentyl ester) was charged and the phases separated. The resulting toluene solution of 1-bromo-2-methyl-pentane was used directly in the following step.

(5R)-3-Cyano-5-methyl octanoic acid methyl ester

The reaction vessel was charged with toluene (152ml, 4.7 ml/g based on the weight of 1-bromo-2-methyl-pentane), followed by potassium *tert*-butoxide (76.87g, 0.69 moles) in one portion with stirring. The reaction mixture was cooled to a temperature of from -10°C to -5°C. 4,4,4-Trimethoxy-butyronitrile (37.39g, 0.23 moles) was charged to the toluene solution of 1-bromo-2-methyl-pentane from the previous step and the resulting solution added dropwise to the reaction, while maintaining the temperature at -5°C. The reaction mixture was stirred for 18 hours at a temperature of from -10°C to - 5°C. The reaction was quenched with $H_2O$ (323ml, 10 ml/g based on the weight of 1-bromo-2-methyl-pentane), concentrated HCl (48.5ml) added dropwise to a pH range of 1-2, and the reaction mixture stirred at 25°C for 1 hour. The phases were separated, the organic phase washed with $H_2O$ (323ml, 10 ml/g based on the weight of 1-bromo-2-methyl-pentane), and the phases separated. Toluene was distilled from the organic phase to leave a volume of 65ml. The resulting toluene solution of (5R)-3-cyano-5-methyl octanoic acid methyl ester was used directly in the following step.

(3*S*,5*R*)-3-Cyano-5-methyl-octanoic acid methyl ester

$NaHCO_3$ (47.4g, 0.75 equivalents), $KH_2PO_4$ (4.4g, 0.042 equivalents) and NaOH (0.84g, 0.031 equivalents) were charged to $H_2O$ (1500mL) and stirred at room temperature until a solution formed. The enzyme Lipase PS-SD (30g, commercially available from Amano Enzyme Inc.) was charged to the reaction and the suspension stirred at room temperature until a solution was formed. The reaction mixture was heated to 45°C while a toluene solution of (5*R*)-3-cyano-5-methyl octanoic acid methyl ester (150g, 0.76 Mol, 1 equivalent) was added in one portion over a period of five minutes. The resulting oil in water suspension was stirred vigorously for 48 hours at 45°C. On completion of the reaction, the product was extracted with *tert*-butyl methyl ether (600mL) and the organic phases were combined and washed with brine (300mL). The title compound was held as a *tert*-butyl methyl ether solution for further use in the preparation of (3*S*,5*R*)-3-aminomethyl-5-methyl-octanoic acid.

EXAMPLE 14. (3*S*,5*R*)-3-Aminomethyl-5-methyl-octanoic acid Form A

Method A

Ethanol (25mL) and water (25mL) were charged to the vessel and stirred vigorously to ensure mixing. Crude (3*S*,5*R*)-3-aminomethyl-5-methyl-octanoic acid (2.5g) was charged and the suspension heated to the reflux temperature (80°C) until a solution was formed. The reaction was stirred at 80°C for 1 hour to ensure full dissolution. The solution was filtered through an in-line filter and transferred to a speck-free vessel. The solution was allowed to cool at a rate of 0.5°C/minute until it reached 67 °C and was seeded with 0.5% micronised seed (2.5-10 μm) in a slurry at 67 °C. The suspension was cooled to 0°C at a rate of 0.5°C/minute and stirred at 0°C for 12 hours. The product was collected by filtration and the cake washed with speck-free water (2.5mL) followed by speck-free ethanol (2.5mL). The product was tray dried under vacuum at 40°C for 24 hours or until water content was <0.5 wt%.

Method B

Crude (3*S*,5*R*)-3-aminomethyl-5-methyl-octanoic acid (25mg/mL) in ethanol and water (50:50 by volume) is charged to the reactor and agitation maintained at a moderately fast speed throughout the process. The reactor is heated to a temperature of 55°C at a heating rate of 0.5°C/minute and the temperature held at 55°C for one hour to ensure complete dissolution. The solution is cooled to 51°C at a rate of 0.5°C/minute and held at this temperature for 15 minutes. The solution is seeded with 5% micronised seed (2-25 μm) in a slurry (75mg/mL in ethanol). The ethanol seed slurry is agitated prior to seeding to break agglomeration. The temperature is held at 51°C for a further 20 minutes post seeding. The resulting slurry is cooled to 0°C at a rate of 0.5°C/minute and held at this temperature for 2 hours. The solids are filtered under vacuum and washed with cold ethanol. The filtered solids are dried in a vacuum oven at 50°C.

Method C

Crude (3*S*,5*R*)-3-aminomethyl-5-methyl-octanoic acid (50mg/mL) in ethanol and water (50:50 by volume) is charged to the reactor and agitation maintained at a moderately fast speed throughout the process. The reactor is heated to 80°C (reflux temperature) at a heating rate of 0.5°C/minute. The temperature is held at 80°C for one hour to ensure complete dissolution. The solution is cooled to 0°C at a rate of 0.5°C/minute and held at 0°C for 2 hours. The solids are filtered under vacuum and washed with cold ethanol. The filtered solids are dried in a vacuum oven at 50°C.

Characterisation of (3*S*,5*R*)-3-aminomethyl-5-methyl-octanoic acid Form A

(3*S*,5*R*)-3-aminomethyl-5-methyl-octanoic acid Form A was characterised using the following techniques:

1. Powder X-ray diffraction (PXRD)
2. Differential scanning calorimetry (DSC)
3. Fourier Transform Infrared Spectroscopy (FT-IR)
4. Fourier Transform Ramon Spectroscopy (FT-Raman)

The following experimental conditions were used.

Powder X-ray diffraction (PXRD)

The powder X-ray diffraction pattern was determined using a Bruker-AXS Ltd. D4 powder X-ray diffractometer fitted with an automatic sample changer, a theta-theta goniometer, automatic beam divergence slit, and a PSD Vantec-1 detector. The sample was prepared for analysis by placing on a low background silicon wafer specimen mount. The specimen was rotated whilst being irradiated with copper K-alpha$_1$ X-rays (wavelength = 1.5406 Angstroms) with the X-ray tube operated at 40kV/30mA. The analyses were performed with the goniometer running in continuous mode set for a 0.2 second count per 0.018° step over a two theta range of 2° to 55°.

As will be appreciated by the skilled person, the relative intensities of the various peaks within Tables 1 and 2 given below may vary due to a number of factors such as for example orientation effects of crystals in the X-ray beam or the purity of the material being analysed or the degree of crystallinity of the sample. The peak positions may also shift for variations in sample height but the peak positions will remain substantially as defined in given Tables.

The skilled person will also appreciate that measurements using a different wavelength will result in different shifts according to the Bragg equation - $n\lambda = 2d \sin \theta$.

Such further PXRD patterns generated by use of alternative wavelengths are considered to be alternative representations of the PXRD patterns of the crystalline material of the present invention and as such are within the scope of the present invention.

The crystal structure of Form A was determined by single crystal X-Ray diffraction analysis. In addition, 2-theta angles, d spacings and relative intensities were calculated from the single crystal structure using the "Reflex Powder Diffraction" module of Accelrys Materials Studio™ [version 2.2]. Pertinent simulation parameters were in each case:

Wavelength = 1.540562 A (Cu K$\alpha$)
Polarisation Factor = 0.5
Pseudo-Voigt Profile (U = 0.01, V = -0.001, W = 0.002)

Differential Scanning Calorimetry (DSC)

DSC was performed using a Perkin Elmer Pyris 1 DSC in 50μl vented aluminium pans with aluminium lids. Approximately 3 mg of the sample was heated at 10°C per minute over a range of 10 to 215°C with a nitrogen gas purge.

FT-IR

The IR spectrum was acquired using a ThermoNicolet Avatar FT-IR spectrometer equipped with a 'Golden Gate™' single bounce ATR accessory (diamond top plate and zinc selenide lenses) and DTGS KBr detector. The spectrum was collected at 2 $cm^{-1}$ resolution and a co-addition of 256 scans. Happ-Genzel apodization was used. Because the FT-IR spectrum was recorded using single reflection ATR, no sample preparation was required. Using ATR FT-IR will cause the relative intensities of infrared bands to differ from those seen in a transmission FT-IR spectrum using KBr disc or nujol mull sample preparations. Due to the nature of ATR FT-IR, the bands at lower wavenumber are more intense than those at higher wavenumber. Experimental error, unless otherwise noted, was $\pm$ 2 $cm^{-1}$.

FT-Raman

The Raman spectrum was collected using a ThermoNicolet 960 FT-Raman spectrometer equipped with a 1064nm NdYAG laser and Germanium detector. The spectrum was collected using 320mW laser power at the sample and 5140 co-added scans at 2 $cm^{-1}$ resolution. Happ-Genzel apodization was used. Each sample (approximately 5 mg) was placed in a glass vial and exposed to the laser radiation. The data are presented as Raman intensity as a function of Raman shift. Experimental error, unless otherwise noted, was $\pm$ 2 $cm^{-1}$

Data

The measured PXRD pattern is shown in Figure 1. The main characteristic peaks, with a relative intensity greater than 5%, are listed in Table 1. The calculated PXRD pattern is shown in Figure 2. The main characteristic peaks, with a relative intensity greater than 5%, are listed in Table 2. The main characteristic peaks for (3*S*,5*R*)-3-aminomethyl-5-methyl-octanoic acid Form A are at 7.7, 15.8, 20.8 and 23.1 degrees of two theta-angle ± 0.2 degree. The DSC thermogram for (3S,5R)-3-aminomethyl-5-methyl-octanoic acid Form A is shown in Figure 3 and this shows a single sharp endotherm peak maximum at 194°C ± 2°C. This event represents the melt of (3S,5R)-3-aminomethyl-5-methyl-octanoic acid Form A. The FT-IR spectrum for (3*S*,5*R*)-3-aminomethyl-5-methyl-octanoic acid Form A is displayed in Figs. 4 and 5. The main characteristic peaks for (3*S*,5*R*)-3-aminomethyl-5-methyl-octanoic acid Form A are listed in Table 3. The FT-Raman spectrum for (3S,5R)-3-aminomethyl-5-methyl-octanoic acid Form A is displayed in Figures 6 and 7. The main characteristic peaks for (3*S*,5*R*)-3-aminomethyl-5-methyl-octanoic acid Form A are listed in Table 4.

Table 1 Characteristic PXRD Peaks from Measured Pattern for (3S,5R)-3-aminomethyl-5-methyl-octanoic acid Form A

| Angle 2-Theta (Degrees) | Relative Intensity % | Angle 2-Theta (Degrees) | Relative Intensity % |
|---|---|---|---|
| 7.7 | 100 | 22.8 | 9.6 |
| 12 | 11.5 | 23.1 | 69.4 |
| 15.5 | 60.9 | 23.3 | 12.9 |
| 15.8 | 51.4 | 25.6 | 11.5 |
| 18.3 | 8.4 | 26 | 5.6 |
| 19.2 | 5.1 | 27.1 | 7.3 |
| 19.5 | 5.4 | 27.7 | 7.4 |
| 20.8 | 40.2 | 39.3 | 6.4 |

Table 2 Characteristic PXRD Peaks from Calculated Pattern for (3S,5R)-3-aminomethyl-5-methyl-octanoic acid Form A

| Angle 2-Theta | Intensity % | Angle 2-Theta | Intensity % | Angle 2-Theta | Intensity % |
|---|---|---|---|---|---|
| 7.7 | 51.2 | 19.5 | 31.8 | 27.1 | 6.4 |
| 12 | 25.1 | 20.8 | 100 | 27.7 | 17.6 |
| 15.5 | 11.4 | 21.3 | 21.6 | 30.1 | 15.8 |
| 15.8 | 35.7 | 22.9 | 10.6 | 31 | 8.8 |
| 16.3 | 11.7 | 23.1 | 50.7 | 33.9 | 5.1 |
| 17.9 | 23.4 | 23.7 | 5.6 | 37.1 | 6.5 |
| 18 | 5 | 25.6 | 5 | 38.2 | 6.1 |
| 18.3 | 48.8 | 26 | 5.7 | 44.4 | 5.2 |
| 19.3 | 6.8 | 26.4 | 12 | | |

Table 3 Peak listing for (3*S*,5*R*)-3-aminomethyl-5-methyl-octanoic acid Form A FT-IR data

| Major absorption band frequencies are listed in the Table below (w:weak, m: medium, s: strong). The intensity assignments are relative to the major band in the spectrum and are not based on absolute values measured from the baseline. | |
|---|---|
| **Wavenumber ($cm^{-1}$)** | **Vibrational band assignment** |
| 2954m, 2919m, 2895w, 2874w | CH stretch (Aliphatic) |
| 2807w, 2758w, 2677w, 2595m | $NH_3^+$ asymmetric and symmetric stretch |

(continued)

| Major absorption band frequencies are listed in the Table below (w:weak, m: medium, s: strong). The intensity assignments are relative to the major band in the spectrum and are not based on absolute values measured from the baseline. | |
|---|---|
| **Wavenumber ($cm^{-1}$)** | **Vibrational band assignment** |
| 2206m | Combination band of $NH_3^+$ asymmetric bending and $NH_3^+$ torsion |
| 1642m | |
| 1545s | $CO_2^-$ asymmetric stretch and $NH_3^+$ asymmetric bend |
| 1464m, 1454m | CH bending (Aliphatic) |
| 1429m | |
| 1416m | |
| 1387s | |
| 1334s | |
| 1277s | |
| 1228w | |
| 1192w | |
| 1163m | |
| 1135w | |
| 1106w | |
| 1020w | |
| 1006m | |
| 970w | |
| 961m | |
| 934w | |
| 914w | |
| 894w | |
| 871w | |
| 858m | |
| 820m | |
| 741w | |
| 701s | |

Table 4 Peak listing for (3*S*,5*R*)-3-aminomethyl-5-methyl-octanoic acid Form A FT-Raman data

| Peak table of relative intense, well defined FT-Raman bands. The intensity assignments are relative to the major band in the spectrum and are not based on absolute values measured from the baseline. (w:weak, m: medium, s: strong, vs: very strong) | | |
|---|---|---|
| **Wavenumber ($cm^{-1}$)** | | |
| 2958vs | 1279w | 595w |
| 2942vs | 1213w | 570w |
| 2932vs | 1190w | 508w |

(continued)

| Peak table of relative intense, well defined FT-Raman bands. The intensity assignments are relative to the major band in the spectrum and are not based on absolute values measured from the baseline. (w:weak, m: medium, s: strong, vs: very strong) | | |
|---|---|---|
| **Wavenumber ($cm^{-1}$)** | | |
| 2919vs | 1158w | 464w |
| 2896vs | 1133w | 440w |
| 2875vs | 1106w | 414w |
| 2849s | 1071w | 386w |
| 1550w | 1038w | 314w |
| 1467m | 969w | |
| 1439m | 933w | |
| 1384w | 912w | |
| 1344m | 869w | |
| 1291w | 820m | |

FIGURES

Figure 1 shows the measured PXRD pattern for (3*S*,5*R*)-3-aminomethyl-5-methyl-octanoic acid Form A
Figure 2 shows the calculated PXRD pattern for (3*S*,5*R*)-3-aminomethyl-5-methyl-octanoic acid Form A
Figure 3 shows the DSC thermogram for (3*S*,5*R*)-3-aminomethyl-5-methyl-octanoic acid Form A
Figure 4 shows the FT-IR spectrum for (3*S*,5*R*)-3-aminomethyl-5-methyl-octanoic acid Form A
Figure 5 shows the fingerprint region of the FT-IR spectrum for (3S,5R)-3-aminomethyl-5-methyl-octanoic acid Form A
Figure 6 shows the FT-Raman spectrum for (3*S*,5*R*)-3-aminomethyl-5-methyl-octanoic acid Form A
Figure 7 shows the fingerprint region of the FT-Raman spectrum for (3S,5R)-3-aminomethyl-5-methyl-octanoic acid Form A

It should be noted that, as used in this specification and the appended claims, singular articles such as "a," "an," and "the," may refer to a single object or to a plurality of objects unless the context clearly indicates otherwise. Thus, for example, reference to a composition containing "a compound" may include a single compound or two or more compounds. It is to be understood that the above description is intended to be illustrative and not restrictive. Many embodiments will be apparent to those of skill in the art upon reading the above description. Therefore, the scope of the invention should be determined with references to the appended claims and includes the full scope of equivalents to which such claims are entitled. The disclosures of all articles and references, including patents, patent applications and publications, are herein incorporated by reference in their entirety and for all purposes.

**Claims**

**1.** A process for preparing a compound of Formula 10, or a salt thereof, and a compound of Formula 11, or a salt thereof:

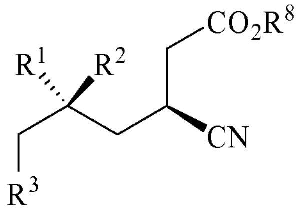

10

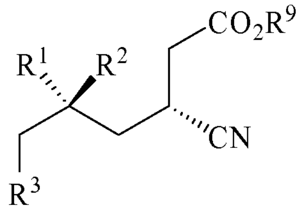

11

comprising

(a) contacting a compound of Formula 7,

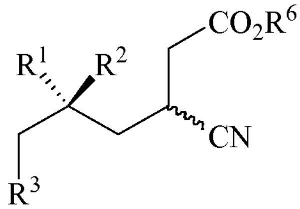

7

with an enzyme, wherein the enzyme diastereoselectively hydrolyzes the compound of Formula 7 to the compound of Formula 10, or a salt thereof, wherein $R^8$ is H, or to a compound of Formula 11, or a salt thereof, wherein $R^9$ is H;
(b) isolating the compound of Formula 10, or a salt thereof; and
(c) optionally hydro lyzing the compound of Formula 10 or 11 wherein $R^8$ or $R^9$, respectively, is other than H to give the free carboxylic acid, or salt thereof, wherein $R^8$ or $R^9$, respectively, is H;
wherein

$R^1$ and $R^2$ are each independently selected from hydrogen and $C_{1-3}$ alkyl, provided that $R^1$ and $R^2$ are not both hydrogen;
$R^3$ is selected from $C_{1-6}$ alkyl and $C_{2-6}$ alkenyl;
$R^6$ in Formula 7 is selected from $C_{1-6}$ alkyl, $C_{2-6}$ alkenyl, $C_{2-6}$ alkynyl, $C_{3-7}$ cycloalkyl, $C_{3-7}$ cycloalkenyl, halo-$C_{1-6}$ alkyl, halo-$C_{2-6}$ alkenyl, halo-$C_{2-6}$ alkynyl, aryl-$C_{1-6}$ alkyl, aryl-$C_{2-6}$ alkenyl, and aryl-$C_{2-6}$ alkynyl; and
$R^8$ and $R^9$ in Formula 10 and 11 are each independently selected from hydrogen, $C_{1-6}$ alkyl, $C_{2-6}$ alkenyl, $C_{2-6}$ alkynyl, $C_{3-7}$ cycloalkyl, $C_{3-7}$ cycloalkenyl, halo-$C_{1-6}$ alkyl, halo-$C_{2-6}$ alkenyl, halo-$C_{2-6}$ alkynyl, aryl-$C_{1-6}$ alkyl, aryl-$C_{2-6}$ alkenyl, and aryl-$C_{2-6}$ alkynyl, provided that one but not both $R^8$ and $R^9$ is hydrogen; and wherein each of the aforementioned aryl moieties may be optionally substituted with from one to three substituents independently selected from $C_{1-3}$ alkyl, $C_{1-3}$ alkoxy, amino, $C_{1-3}$ alkylamino, and halogeno.

**2.** A process as claimed in claim 1 for preparing a compound of Formula 10a, or a salt thereof:

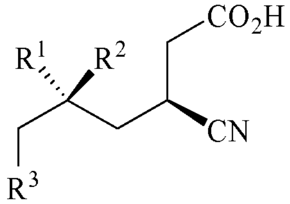

10a

wherein $R^1$ and $R^2$ are each independently selected from hydrogen and $C_{1-3}$ alkyl, provided that $R^1$ and $R^2$ are not both hydrogen;
$R^3$ is selected from $C_{1-6}$ alkyl and $C_{2-6}$ alkenyl; the process comprising the steps of:

(a) contacting a compound of Formula 7,

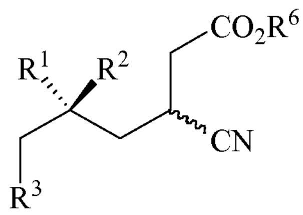

7

with an enzyme to yield the compound of Formula 10, or a salt thereof, and a compound of Formula 11, or a salt thereof,

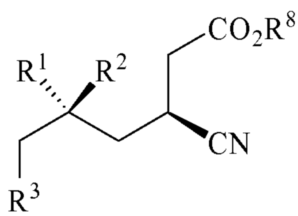

10

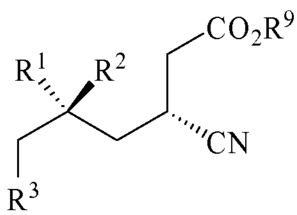

11

wherein the enzyme diastereoselectively hydrolyzes the compound of Formula 7 to the compound of Formula 10, or a salt thereof, wherein $R^8$ is H, or to a compound of Formula 11, or a salt thereof, wherein $R^9$ is H;
(b) isolating the compound of Formula 10, or a salt thereof; and
(c) optionally hydrolyzing the compound of Formula 10 wherein $R^8$ is other than H, to give the compound of Formula 10a, or salt thereof,
wherein

$R^1$, $R^2$, and $R^3$ in Formula 7, Formula 10, and Formula 11 are as defined for Formula 10a;
$R^6$ in Formula 7 is selected from $C_{1-6}$ alkyl, $C_{2-6}$ alkenyl, $C_{2-6}$ alkynyl, $C_{3-7}$ cycloalkyl, $C_{3-7}$ cycloalkenyl, halo-$C_{1-6}$ alkyl, halo-$C_{2-6}$ alkenyl, halo-$C_{2-6}$ alkynyl, aryl-$C_{1-6}$ alkyl, aryl-$C_{2-6}$ alkenyl, and aryl-$C_{2-6}$ alkynyl; and
$R^8$ and $R^9$ in Formula 10 and 11 are each independently selected from hydrogen, $C_{1-6}$ alkyl, $C_{2-6}$ alkenyl, $C_{2-6}$ alkynyl, $C_{3-7}$ cycloalkyl, $C_{3-7}$ cycloalkenyl, halo-$C_{1-6}$ alkyl, halo-$C_{2-6}$ alkenyl, halo-$C_{2-6}$ alkynyl, aryl-$C_{1-6}$ alkyl, aryl-$C_{2-6}$ alkenyl, and aryl-$C_{2-6}$ alkynyl, provided that one but not both $R^8$ and $R^9$ is hydrogen; and wherein each of the aforementioned aryl moieties may be optionally substituted with from one to three substituents independently selected from $C_{1-3}$ alkyl, $C_{1-3}$ alkoxy, amino, $C_{1-3}$ alkylamino, and halogeno.

**3.** A process for the preparation of a compound of Formula 1

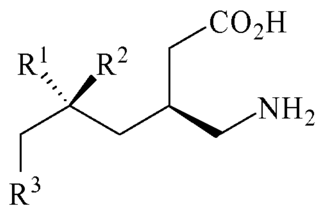

1

,

or a pharmaceutically acceptable salt, solvate or hydrate thereof, wherein:

$R^1$ and $R^2$ are each independently selected from hydrogen and $C_{1-3}$ alkyl, provided that when $R^1$ is hydrogen, $R^2$ is not hydrogen; and
$R^3$ is selected from $C_{1-6}$ alkyl and $C_{2-6}$ alkenyl; comprising steps (a) to (c) of the process as defined in claim 1 or claim 2, and further comprising the steps:

(d) reducing the cyano moiety of a compound of Formula 10 wherein $R^8$ is H, or a salt thereof:

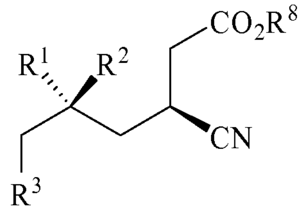

10

wherein $R^1$, $R^2$, and $R^3$ in Formula 10 are as defined for a compound of Formula 1; and
(e) optionally further converting the compound of Formula 1 or a salt thereof into a pharmaceutically acceptable salt, solvate or hydrate thereof.

**4.** A process as claimed in claim 1 wherein $R^9$ is hydrogen.

**5.** A process as claimed in claim 1 or 4 wherein $R^6$ and $R^8$ are independently selected from $C_{1-6}$ alkyl.

**6.** A process as claimed in claim 5 wherein $R^6$ and $R^8$ are independently selected from methyl and ethyl.

**7.** A process as claimed in claim 1, 4, 5 or 6 wherein $R^1$ and $R^2$ are each independently hydrogen or methyl, provided that $R^1$ and $R^2$ are not both hydrogen, and $R^3$ is $C_{1-6}$ alkyl.

**8.** A process as claimed in claim 7 wherein $R^1$ is hydrogen, $R^2$ is methyl, and $R^3$ is methyl, ethyl, *n*-propyl or *i*-propyl.

**9.** A process as claimed in claim 8 wherein $R^1$ is hydrogen, $R^2$ is methyl, and $R^3$ is ethyl.

**10.** A process as claimed in any one of the preceding claims 1 to 9 wherein the enzyme in step (a) is a lipase.

**11.** A process as claimed in claim 10 wherein the enzyme is a lipase from the microorganism *Burkholderia cepacia* or the microorganism Thermomyces *lanuginosus.*

**12.** A process as claimed in claim 1 or any one of claims 4 to 11 wherein the process further comprises the step of optionally converting the compound of Formula 10 wherein $R^8$ is H to a salt thereof; preferably to an alkali metal salt thereof; most preferably to the sodium salt thereof.

**13.** A process as claimed in claim 2 wherein $R^6$ and $R^8$ are independently selected from $C_{1-6}$ alkyl.

**14.** A process as claimed in claim 13 wherein $R^6$ and $R^8$ are independently selected from methyl and ethyl.

**15.** A (3*S*,5*R*)-3-cyano-5-methyl-octanoic acid or a salt or ester thereof of the formula 10b

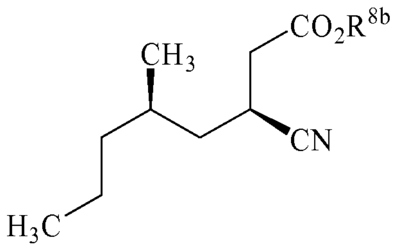

**10b**

wherein $R^{8b}$ is selected from hydrogen, $C_{1-6}$ alkyl, $C_{2-6}$ alkenyl, $C_{2-6}$ alkynyl, $C_{3-7}$ cycloalkyl, $C_{3-7}$ cycloalkenyl, halo-$C_{1-6}$ alkyl, halo-$C_{2-6}$ alkenyl, halo-$C_{2-6}$ alkynyl, aryl-$C_{1-6}$ alkyl, aryl-$C_{2-6}$ alkenyl, and aryl-$C_{2-6}$ alkynyl and wherein each of the aforementioned aryl moieties may be optionally substituted with from one to three substituents independently selected from $C_{1-3}$ alkyl, $C_{1-3}$ alkoxy, amino, $C_{1-3}$ alkylamino, and halogeno.

**16.** A compound as claimed in claim 15 wherein $R^{8b}$ is $C_{1-6}$ alkyl, preferably methyl or ethyl.

**17.** An alkali metal salt of (3*S*,5*R*)-3-cyano-5-methyl-octanoic acid, preferably the sodium salt thereof.

Figure 1

Measured PXRD Pattern for (3*S*,5*R*)-3-aminomethyl-5-methyl-octanoic acid Form A

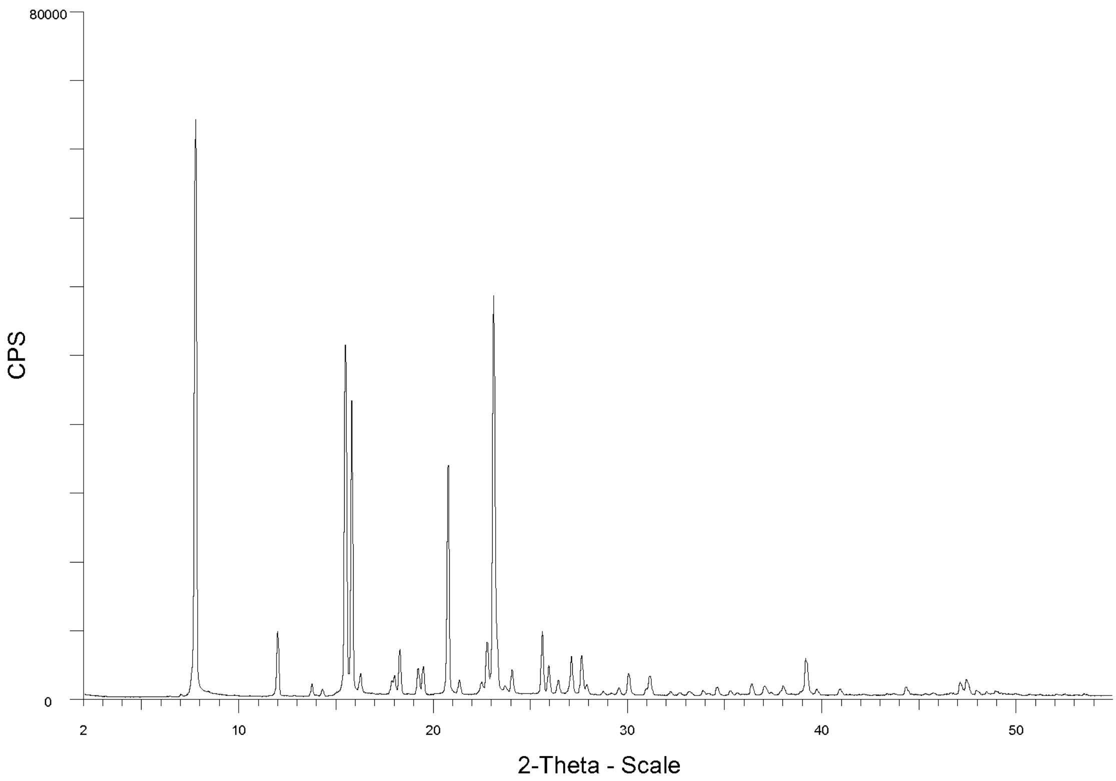

Figure 2

Calculated PXRD Pattern for (3*S*,5*R*)-3-aminomethyl-5-methyl-octanoic acid Form A

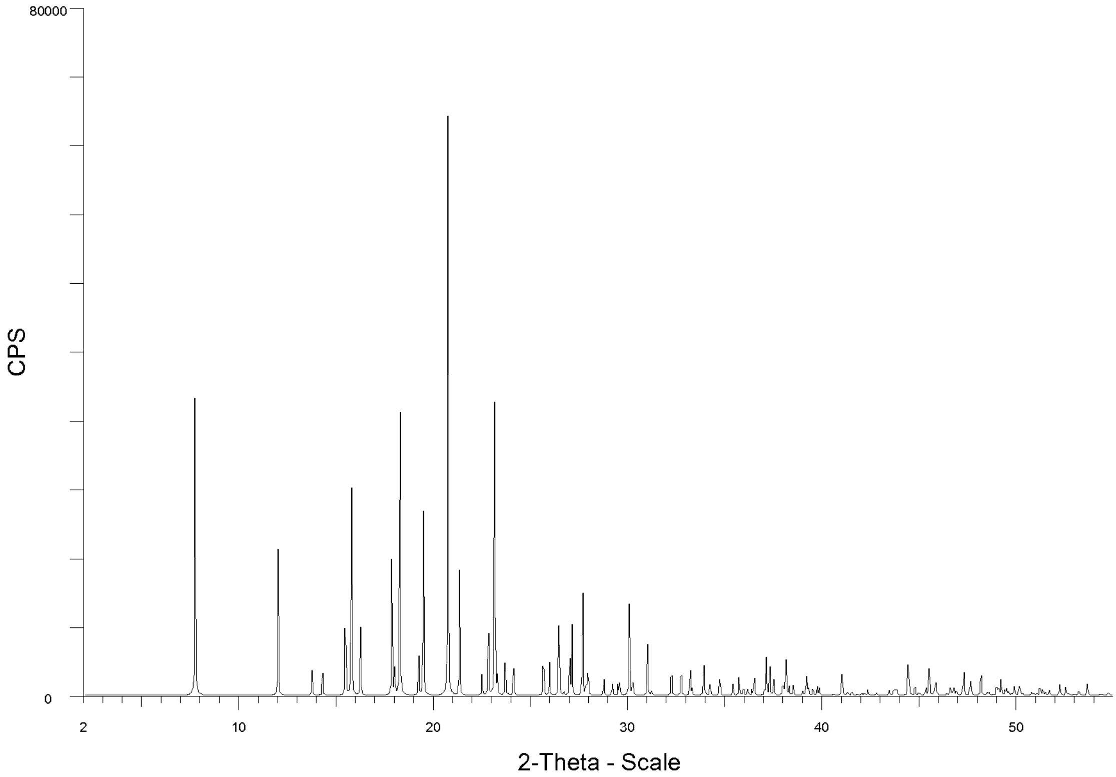

Figure 3

DSC Thermogram for (3*S*,5*R*)-3-aminomethyl-5-methyl-octanoic acid Form A

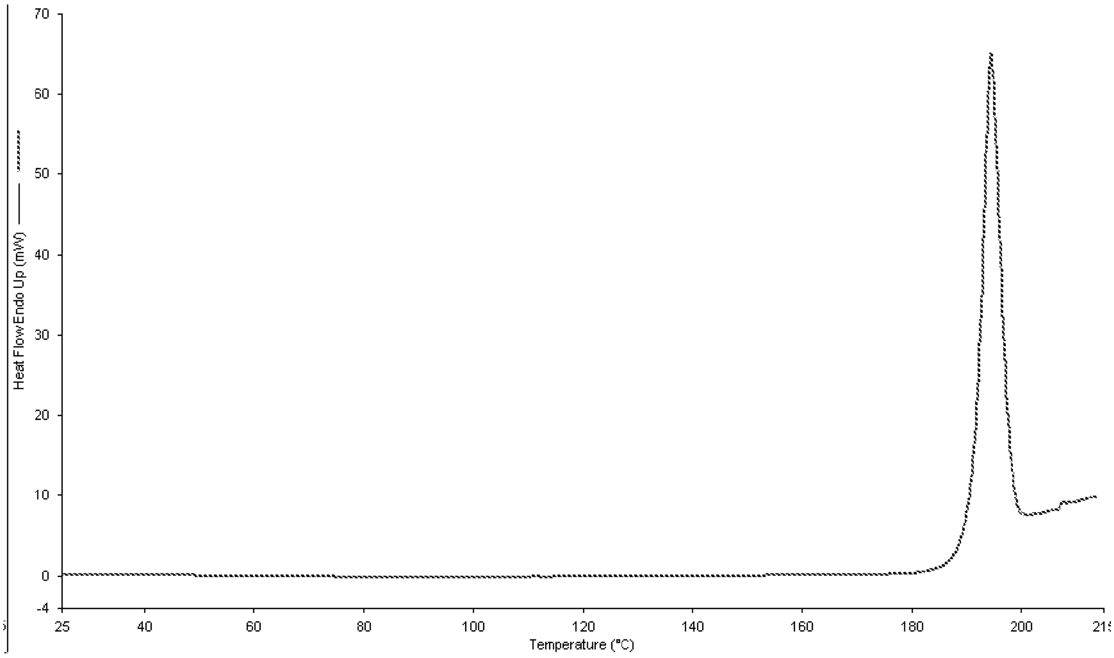

Figure 4

FT-IR spectrum for (3*S*,5*R*)-3-aminomethyl-5-methyl-octanoic acid Form A

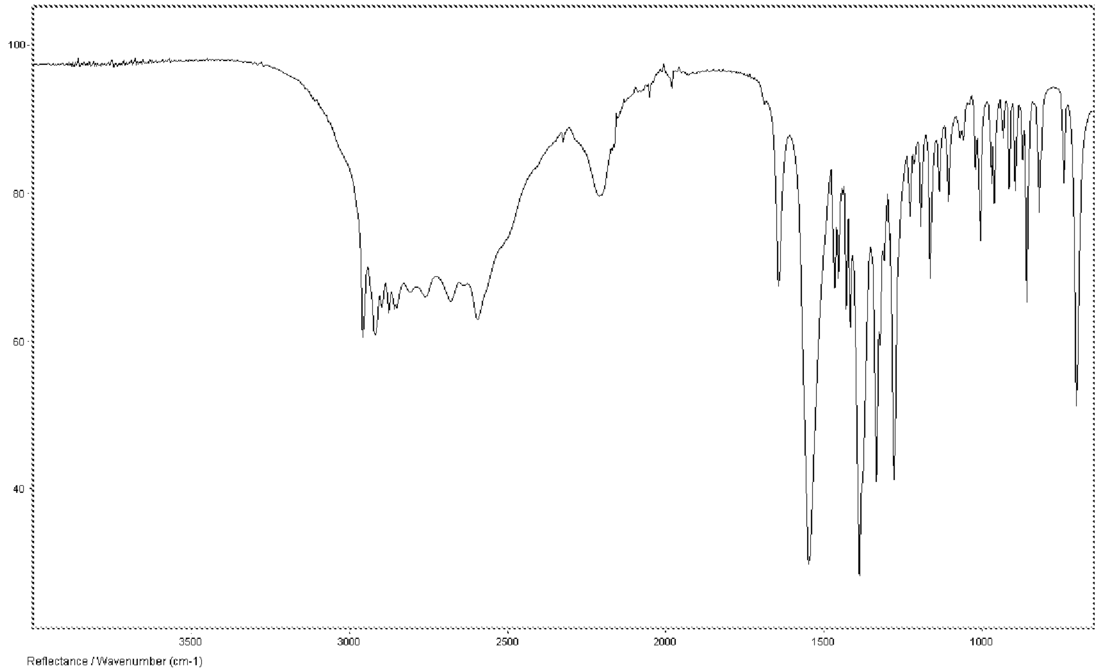

Figure 5

FT-IR spectrum for (3*S*,5*R*)-3-aminomethyl-5-methyl-octanoic acid Form A, fingerprint region

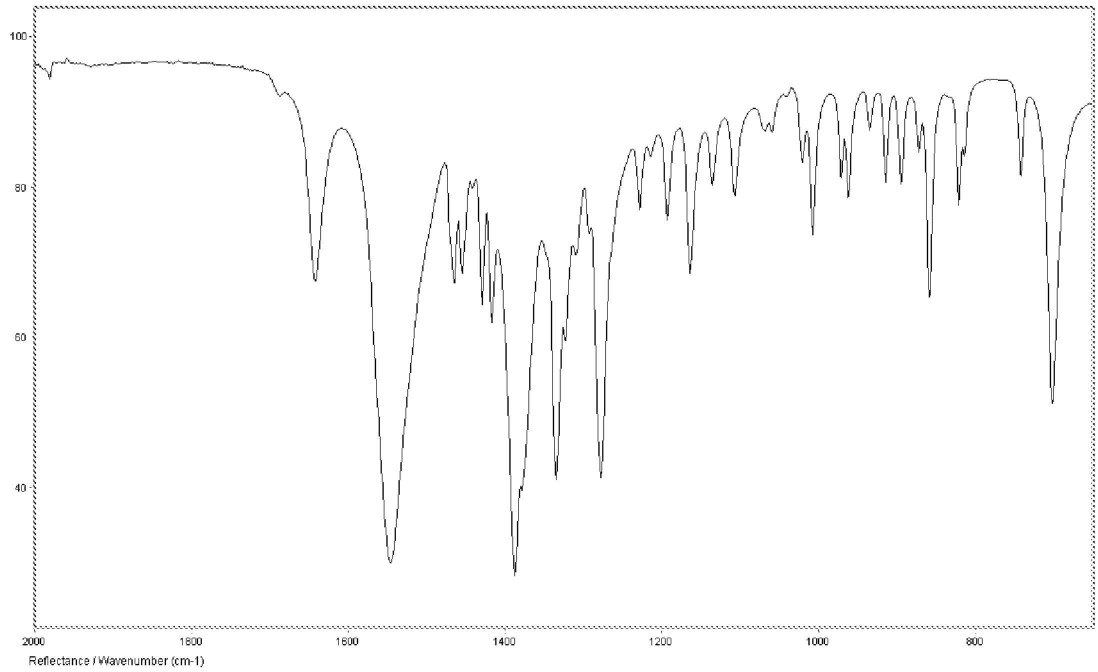

Figure 6

FT-Raman spectrum for (3*S*,5*R*)-3-aminomethyl-5-methyl-octanoic acid Form A

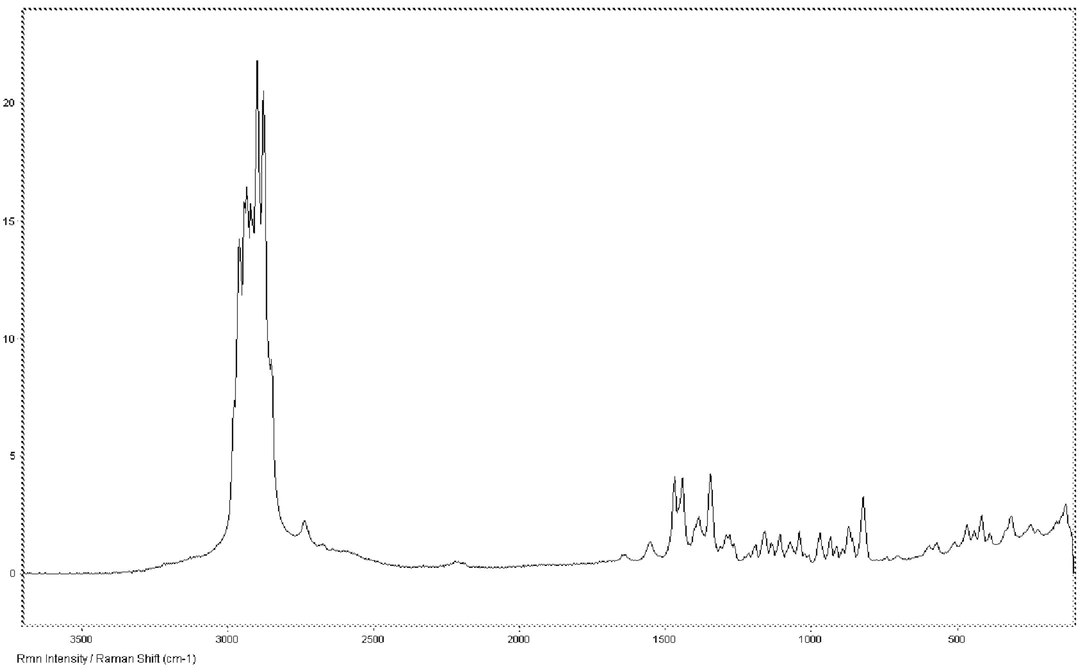

Figure 7

FT-Raman spectrum for (3*S*,5*R*)-3-aminomethyl-5-methyl-octanoic acid Form A, fingerprint region

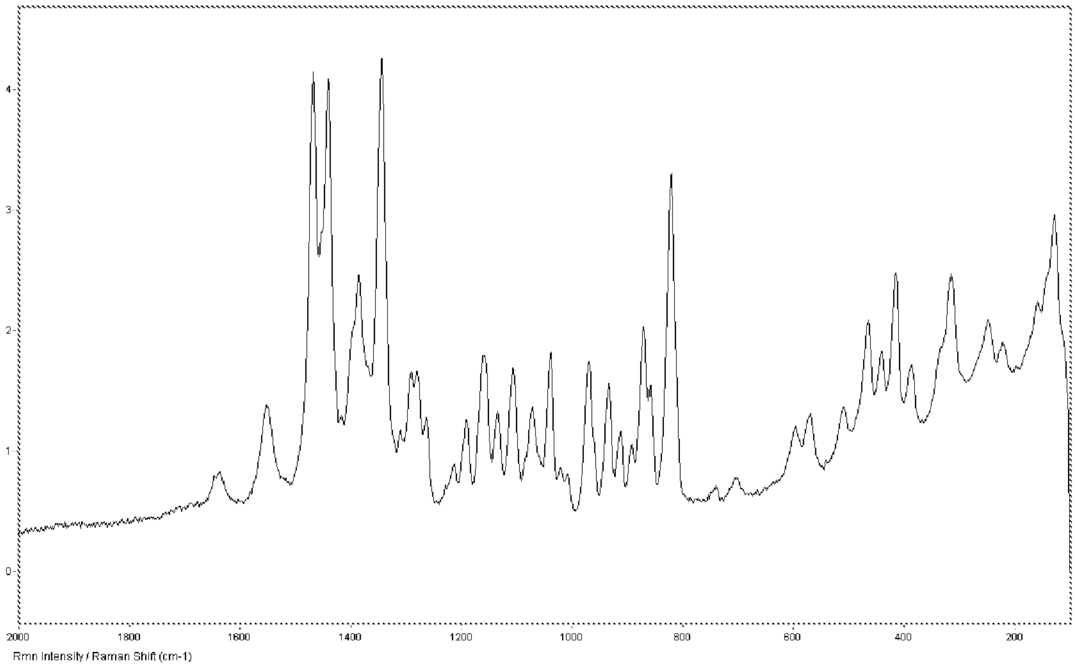

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2000076958 A
- US 6642398 B
- WO 2004054566 A
- US 5618710 A, M. A. Navia & N. L. St. Clair
- US 20030149172 A
- US 6106864 A
- WO 0035298 A
- WO 9111172 A
- WO 9402518 A
- WO 9855148 A


### Non-patent literature cited in the description


- **T. W. GREENE ; P. G. WUTS.** *Protecting Groups in Organic Chemistry,* 1999
- **P. KOCIENSKI.** *Protective Groups,* 2000
- **K. M. KOELLER ; C.-H. WONG.** Enzymes for chemical synthesis. *Nature,* 11 January 2001, vol. 409, 232-240
- **A. M. ANDERSON.** *Biocat. Biotransform,* 1998, vol. 16, 181
- **P. LÓPEZ-SERRANO et al.** *Biotechnol. Lett.,* 2002, vol. 24, 1379-83
- **STAHL ; WERMUTH.** Handbook of Pharmaceutical Salts: Properties, Selection, and Use. Wiley-VCH, 2002
- **K. R. MORRIS.** Polymorphism in Pharmaceutical Solids. Marcel Dekker, 1995
- **O. ALMARSSON ; M. J. ZAWOROTKO.** *Chem Commun,* 2004, vol. 17, 1889-1896
- **HALEBLIAN.** *J Pharm Sci,* August 1975, vol. 64 (8), 1269-1288
- Remington's Pharmaceutical Sciences. Mack Publishing Company, 1995
- **LIANG ; CHEN.** *Expert Opinion in Therapeutic Patents,* 2001, vol. 11 (6), 981-986
- **H. LIEBERMAN ; L. LACHMAN.** Pharmaceutical Dosage Forms: Tablets. Marcel Dekker, 1980, vol. 1
- **VERMA et al.** *Pharmaceutical Technology On-line,* 2001, vol. 25 (2), 1-14
- **FINNIN ; MORGAN.** *J Pharm Sci,* October 1999, vol. 88 (10), 955-958
- **D. YAZBECK et al.** *Synth. Catal.,* 2003, vol. 345, 524-32